# EUROPEAN PATENT SPECIFICATION

(11) **EP 4 048 301 B1**
(45) Date of publication and mention of the grant of the patent: **18.09.2024**
(21) Application number: 20800349.1
(22) Date of filing: 23.10.2020
(51) Int. Cl.: A61K 38/17, C07K 14/705, C12N 15/63, C12N 5/0783, C07K 14/725

(54) **CHIMERIC POLYPEPTIDE FOR REGULATING IMMUNE CELLS**
CHIMÄRES POLYPEPTID ZUM REGULIEREN VON IMMUNZELLEN
POLYPEPTIDE CHIMÈRE POUR LA RÉGULATION DE CELLULES IMMUNITAIRES

(30) Priority: 23.10.2019 NL 2024083
(43) Date of publication of application: 31.08.2022
(73) Proprietor: Stichting Het Nederlands Kanker Instituut- Antoni van Leeuwenhoek Ziekenhuis, 1066 CX Amsterdam (NL)
(72) Inventor: SCHUMACHER, Antonius Nicolaas Maria, 1066 CX AMSTERDAM (NL); SAHILLIOGLU, Ali Can, 1066 CX AMSTERDAM (NL)
(74) Representative: Algemeen Octrooi- en Merkenbureau B.V.
(86) International application number: PCT/NL2020/050655
(87) International publication number: WO 2021/080427

(56) References cited:
- WO-A1-2016/193696
- WO-A1-2019/089592
- WO-A1-97/10253
- WO-A2-2017/181119
- DATABASE MEDLINE [online] US NATIONAL LIBRARY OF MEDICINE (NLM), BETHESDA, MD, US; 15 March 2012 (2012-03-15), WHITING RHIANNON J ET AL: "Targeting Lyn tyrosine kinase through protein fusions encompassing motifs of Cbp (Csk-binding protein) and the SOCS box of SOCS1.", Database accession no. NLM22364282
- WHITING RHIANNON J ET AL: "Targeting Lyn tyrosine kinase through protein fusions encompassing motifs of Cbp (Csk-binding protein) and the SOCS box of SOCS1.", THE BIOCHEMICAL JOURNAL 15 MAR 2012, vol. 442, no. 3, 15 March 2012 (2012-03-15), pages 611 - 620, ISSN: 1470-8728
- IULIA DIACONU ET AL: "Inducible Caspase-9 Selectively Modulates the Toxicities of CD19-Specific Chimeric Antigen Receptor-Modified T Cells", MOLECULAR THERAPY : THE JOURNAL OF THE AMERICAN SOCIETY OF GENE THERAPY, vol. 25, no. 3, 1 March 2017 (2017-03-01), US, pages 580 - 592, XP055692323, ISSN: 1525-0016, DOI: 10.1016/j.ymthe.2017.01.011
- R. SAKEMURA ET AL: "A Tet-On Inducible System for Controlling CD19-Chimeric Antigen Receptor Expression upon Drug Administration", CANCER IMMUNOLOGY RESEARCH, vol. 4, no. 8, 21 June 2016 (2016-06-21), US, pages 658 - 668, XP055513121, ISSN: 2326-6066, DOI: 10.1158/2326-6066.CIR-16-0043

## Description

### Field of the Invention

The present invention relates to chemically regulated chimeric polypeptides which enable reversible and dose-dependent control of T cell receptor and/or NK cell receptor and/or chimeric antigen receptor function.

### Background of the invention

Over the past years, a number of adoptive T cell therapies has been developed for the treatment of both hematological cancers and solid tumors (Rosenberg et al, Science. 2015 Apr 3;348(6230):62-8, June et al, Science. 2018 Mar 23;359(6382):1361-1365). Specifically, following the discovery of the anti-tumor effect of donor-derived T cells upon allogeneic hematopoietic stem cell transplant, donor lymphocyte infusion (DLI) has been developed as an approach to capitalize on this effect (Frey et al, Best Pract Res Clin Haematol. 2008 Jun; 21(2): 205-222).

More recently, T cells modified with chimeric antigen receptors (CARs) and T cell receptors (TCRs) have been utilized to provide patients with tumor reactive T cell populations, and the significant clinical activity of CD19 CAR T cells in patients with B cell malignancies has recently led to the approval of these T cell products for B-ALL and DLBCL (Boyiadzis et al, J Immunother Cancer. 2018 Dec 4;6(1):137).

Importantly, the broader applicability of adoptive T cell therapies in cancer is presently limited by safety concerns. Specifically, on target - off tumor toxicity is often observed when the antigens that are targeted by the adoptively transferred T cell pool are also expressed in healthy tissue (Bonifant et al, Mol Ther Oncolytics. 2016 Apr 20;3:16011).

Over the past years, a substantial effort has been made to discover truly cancer specific markers, in order to avoid such on target - off tumor toxicity. However, strictly cancer specific markers that are expressed by tumors of a large fraction of patients have proven extremely rare. In addition, unexpected cross-reactivity of genetically modified T cells with self-antigens that show low-level expression in vital tissue has been associated with severe toxicity (Morgan et al, J Immunother. 2013 Feb;36(2):133-51).

Finally, even when fully tumor-specific activation of the infused cells can be achieved, the profound cytokine release upon T cell recognition of a large tumor mass may form a safety concern, and strategies to tune the strength of T cell activation would be attractive.

The significant risk of treatment-induced toxicity upon adoptive T cell therapy has led a number of groups to develop genetically encoded suicide switches, such as the HSV-TK, iCas9, and CD20-based cell surface marker suicide switches, that can be triggered by drug administration at the moment that major toxicity is observed (Jones et al, Front Pharmacol. 2014 Nov 27;5:254).

Such suicide switches have primarily found use in the setting of DLI where the graft versus tumor (GvT) effect is correlated with graft versus host disease (GvHD), and a balance between these two effects is sought. However, the binary nature of these switches does not allow a titration of T cell functions and these systems have found limited use in the context of TCR/CAR-modified T cells.

In more recent work, safety switch technologies that can reversibly control chimeric antigen receptor (CAR) T cells have been engineered (Wu et al, Science. 2015 Oct 16;350(6258):aab4077, Ma et al, Proc Natl Acad Sci U S A. 2016 Jan 26;113(4):E450-8, Loureiro et al, Blood Cancer J. 2018 Sep; 8(9): 81), but these systems cannot be utilized to control the activity of either TCR modified or non-modified T cells. WO 2016/193696 A1 discloses the fusion of an SH2 domain to an ITIM containing domain. The fusion protein is used for modulating signal transduction pathways in T cells, for example via CAR.

Acute GvHD has also been observed in a clinical trial where patients were treated with T cell-depleted NK cell therapy, suggesting that a safety switch for controlling NK cell activity is also desirable (Shah et al, Blood. 2015 Jan 29;125(5):784-92).

In light of this, products, compositions, methods and uses for controlling TCR and CAR T cell function and natural killer (NK) cell function (i.e. NK cell receptor functioning), in particular in patients, would be highly desirable, but are not yet readily available. In particular there is a clear need in the art for reliable, efficient and reproducible products, compositions, methods and uses that allow to control TCR/CAR T cell and NK cell function, such as cytotoxic activity of such TCR or CAR comprising T cells, NK cell receptor (NKR) or CAR comprising NK cells and/or of controlling cytokine secretion by such T cells or NK cells. Accordingly, the technical problem underlying the present invention can be seen in the provision of such products, compositions, methods and uses for complying with any of the aforementioned needs. The technical problem is solved by the embodiments characterized in the claims and herein below.

### Detailed description of the invention

### Brief description of the drawings

Embodiments of the invention are further described hereinafter with reference to the accompanying drawings, in which:

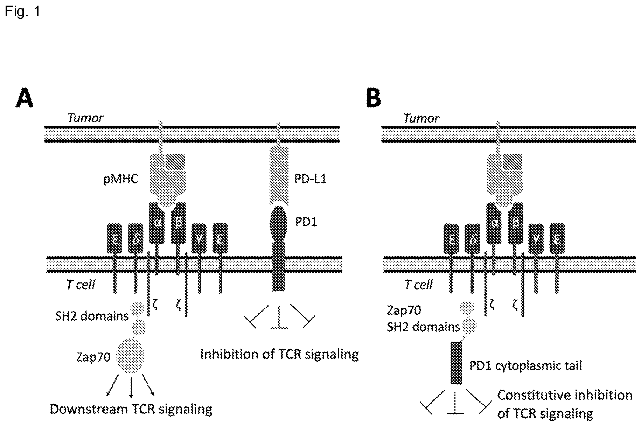

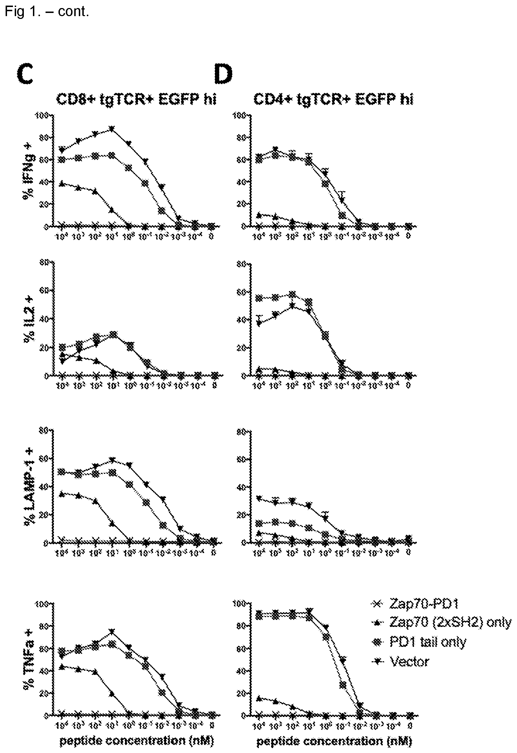

Figure 1: Efficient suppression of T cell function by Zap70 SH2-mediated PD1 tail recruitment. (A) Schematic diagram of early steps in the TCR signal transduction pathway, showing the recruitment of Zap70 to the CD3 ζ chain upon peptide MHC engagement and PD1 mediated inhibition of TCR signaling upon PD-L1 ligation. (B) Schematic diagram of Zap70 SH2 mediated recruitment of the PD1 tail to the activated TCR complex and resulting inhibition of TCR signaling. (C-D) Primary human T cells modified with the HLA class I restricted CDK4 TCR and either the Zap70-PD1, Zap70 2xSH2 domains, PD1 tail, or vector control were co-cultured with CDK4 peptide loaded T2 cells. (C, D) Data depict IFNγ, IL2, TNFα production and cell surface LAMP1 expression of CDK4 TCR+ EGFP high CD8+ (C) and CD4+ (D) T cells.

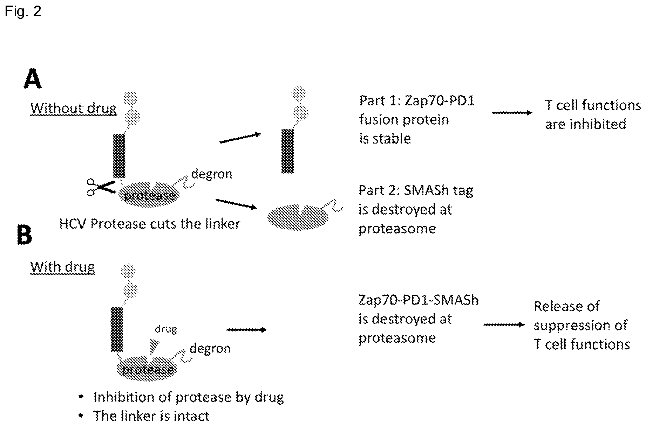

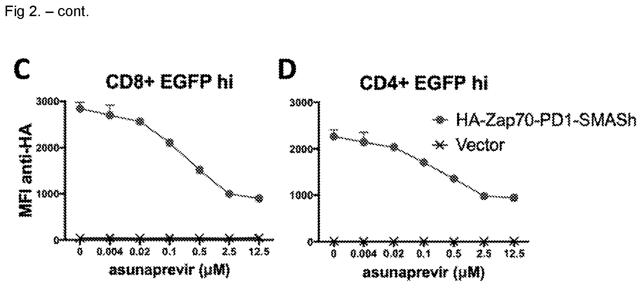

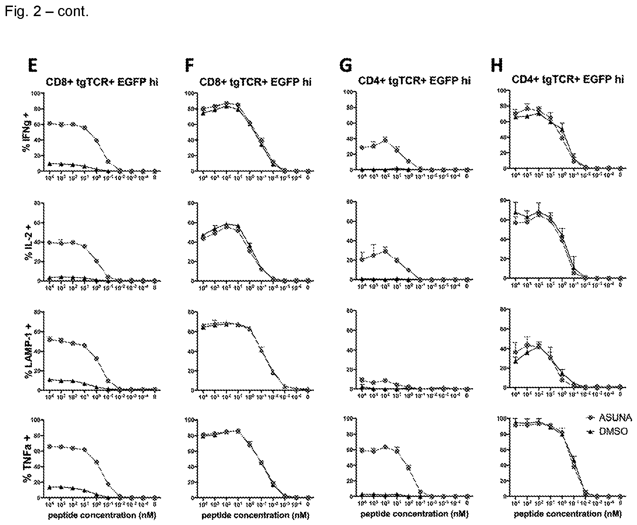

Figure 2: Control of T cell function. Schematic diagram of the Zap70-PD1-SMASh fusion protein in the absence (A) or presence (B) of asunaprevir. In the absence of asunaprevir, the HCV protease releases the Zap70-PD1 moiety to allow inhibition of TCR signaling. In the presence of asunaprevir, release is prevented and the fusion protein is targeted to the proteasome, thereby removing inhibition of T cell function. (C-D) Intracellular HA staining of primary human T cells modified with an N-terminal HA tagged Zap70-PD1-SMASh switch or vector control. Effect of prior asunaprevir exposure for 24 hours on HA signal is depicted for EGFP high CD8+ (C) and CD4+ (D) T cells. (E-H) Primary human T cells modified with the HLA class I restricted CDK4 TCR and either the Zap70-PD1-SMASh switch (E, G), or vector control (F, H), were pretreated with 10 µM asunaprevir or DMSO control. Data depict intracellular IFNγ, IL2, TNFα and cell surface LAMP1 expression of CDK4 TCR+ EGFP high CD8+ (E-F) and CD4+ (G-H) T cells upon co-culture with CDK4 peptide loaded T2 cells in the continued presence or absence of asunaprevir. Error bars represent standard deviation (n=3). Data are representative of two independent experiments.

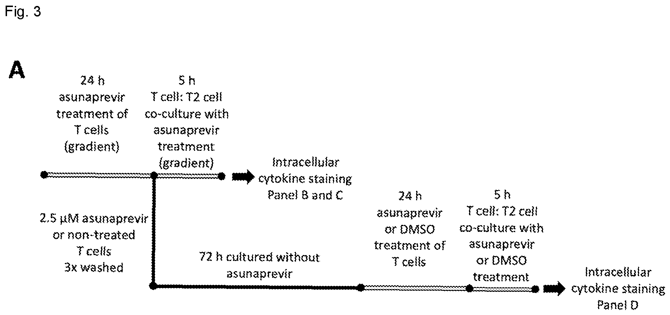

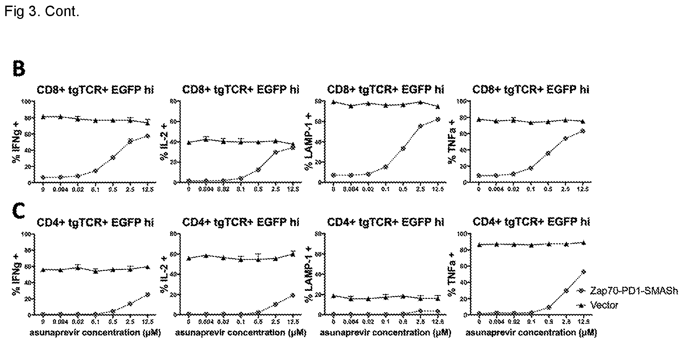

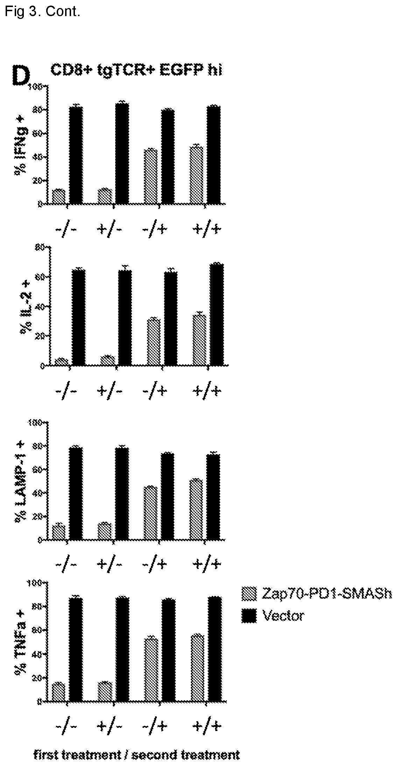

Figure 3: Titratable two-way control of T cell function. (A) Schematic diagram of experimental setup. Primary human T cells modified with the HLA class I restricted CDK4 TCR and either the Zap70-PD1 or vector control were pretreated for 24 hours with the asunaprevir concentrations indicated in B-D and then either used directly (B-C) or cultured for 72 hours in the absence of drug (D). (B-C) Effect of asunaprevir treatment on functional output of CD8+ (B) and CD4+ (C) T cells upon co-culture with T2 cells loaded with 10 nM CDK4 peptide in the continued presence or absence of the indicated asunaprevir concentrations. (D) T cells pre-treated with 2.5 µM asunaprevir or DMSO control were cultured for 72 hours in the absence of drug and were then either treated with 2.5 µM asunaprevir or DMSO control and co-cultured with 10 nM CDK4 peptide loaded T2 cells in the continued presence or absence of drug. Note that a first treatment with asunaprevir does not prevent subsequent inhibition of T cell function by the switch in the absence of drug (compare +/- and -/-), and that release of inhibition by asunaprevir is not influenced by prior release of inhibition (compare -/+ and +/+). (B-D) Data depict intracellular IFNγ, IL2, TNFα and cell surface LAMP1 expression. Error bars represent standard deviation (n=3). Data are representative of two independent experiments.

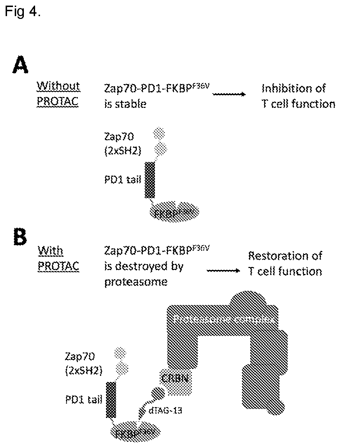

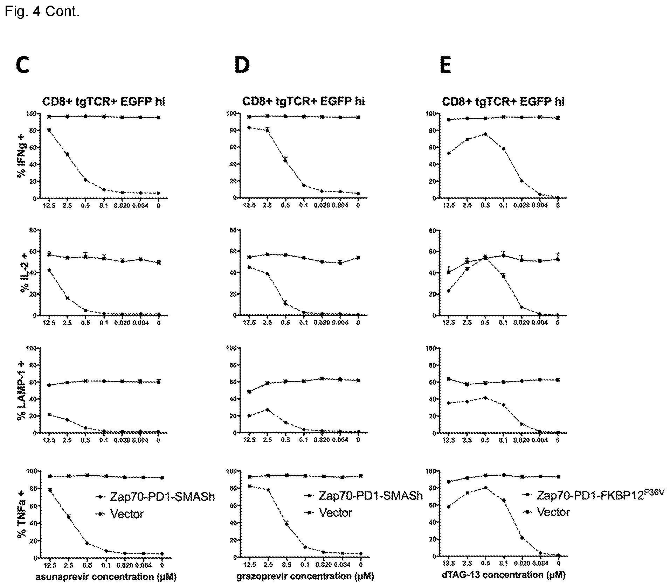

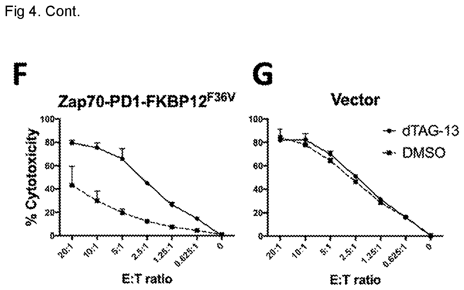

Figure 4: PROTACs can be used to regulate T cell activity in CRASH-IT platform. Schematic diagram of the Zap70-PD1-FKBP12^{F36V} fusion protein in the absence (A) or presence (B) of dTAG-13 PROTAC. (C-E) Primary human T cells modified with the CDK4 TCR and either Zap70-PD1-SMASh, Zap70-PD1-FKBP12^{F36V} or vector control were pretreated with indicated concentrations of HCV NS3/4A protease inhibitors asunaprevir (C) or grazoprevir (D), or dTAG-13 (E). Data depict intracellular IFNγ, IL2, TNFα and cell surface LAMP1 expression of CD8+ CDK4 TCR+ EGFP high T cells upon co-culture with NKIRTIL006 tumor cells that express the CDK4 epitope in the continued presence of indicated concentrations of asunaprevir, grazoprevir or dTAG-13. (F-G) Regulation of T cell cytotoxicity with a small-molecule induced Zap70-PD1-FKBP12^{F36V} switch. Primary human T cells modified with the HLA class I restricted CDK4 TCR and either Zap70-PD1-FKBP12^{F36V} (F) or vector control (G) were sorted for CD8+ and high EGFP expression, expanded by REP and pretreated with 0.5 µM dTAG-13 or DMSO. Data depict ⁵¹Cr release from labelled NKIRTIL006 tumor cells upon co-culture with sorted CD8+ CDK4 TCR+ EGFP high T cells in the continued presence or absence of dTAG-13. Error bars represent standard deviation (n=3). Data are representative of two independent experiments.

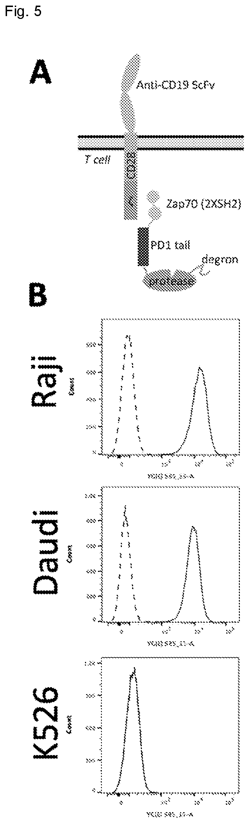

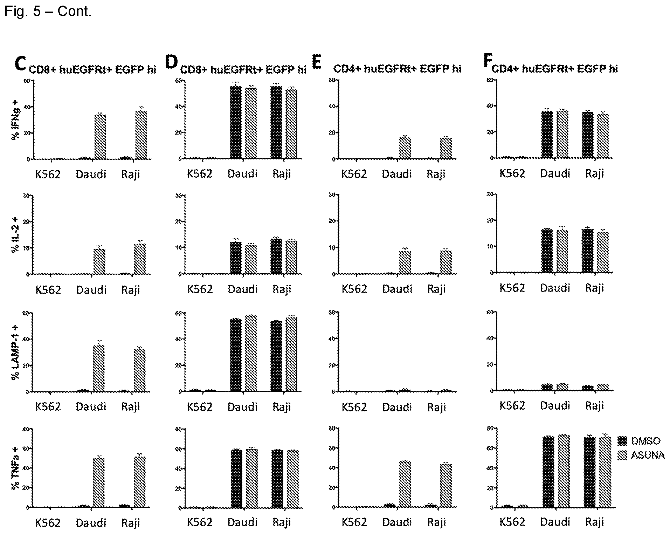

Figure 5: Control of CAR-T cell function by CRASH-IT. (A) Schematic diagram of second generation anti-CD19-CD28-CD3ζ CAR interacting with the Zap70-PD1-SMASh switch. (B) CD19 expression on K562, Daudi and Raji tumors. Cells were stained with anti-CD19-PE (solid line) or isotype control-PE (dashed line). (C-F) Primary human T cells modified with the anti-CD19-CD28-CD3ζ CAR and either Zap70-PD1-SMASh (C, E), or vector control (D, F) were pretreated with 10 µM asunaprevir or DMSO control. Data depict intracellular IFNγ, IL2, TNFα and cell surface LAMP1 expression of CAR+ EGFP high CD8+ (C-D) and CD4+ (E-F) T cells upon co-culture with CD19 negative K562, or CD19 positive Daudi or Raji tumor cells in the continued presence or absence of asunaprevir. Error bars represent standard deviation (n=3). Data are representative of two independent experiments.

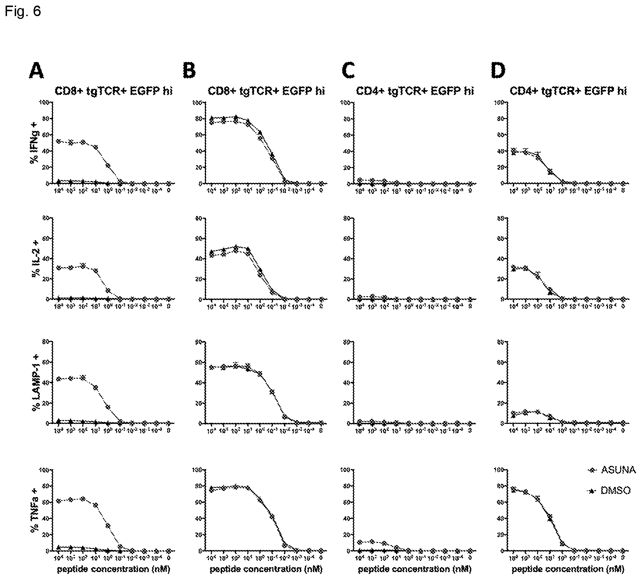

Figure 6: Control of NY-ESO-1 TCR T cell function by CRASH-IT. (A-D) Primary human T cells modified with the intermediate affinity HLA class I restricted NY-ESO-1 TCR and either the Zap70-PD1-SMASh switch (A, C), or vector control (B, D), were pretreated with 10 µM asunaprevir or DMSO control. Data depict intracellular IFNγ, IL2, TNFα and cell surface LAMP1 expression of NY-ESO-1 TCR+ EGFP high CD8+ (A-B) and CD4+ (C-D) T cells upon co-culture with NY-ESO-1 peptide loaded T2 cells in the continued presence or absence of asunaprevir. Error bars represent standard deviation (n=3). Data are representative of two independent experiments.

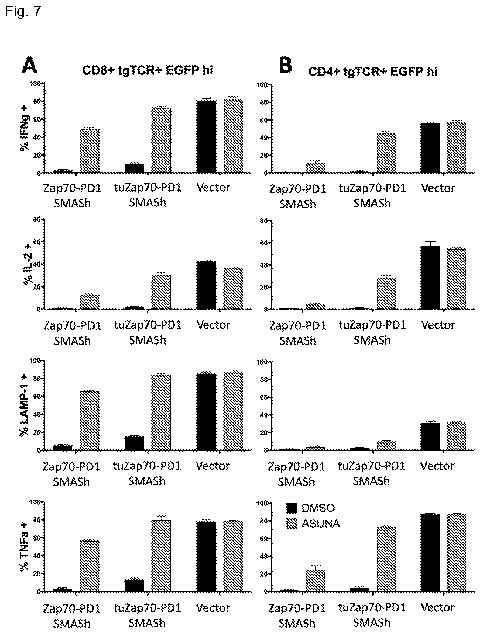

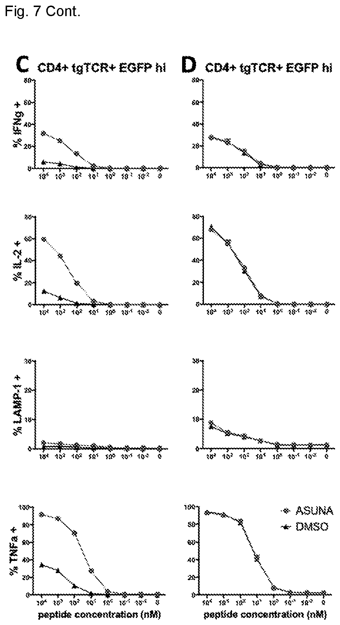

Figure 7: Tuning of inhibitory switches by N-terminal modification. (A-B) Primary human T cells modified with the HLA class I restricted CDK4 TCR and either Zap70-PD1-SMASh, N-terminal alanine added (tuned) tuZap70-PD1-SMASh or vector control were pretreated with 10 µM asunaprevir or DMSO control. Data depict intracellular IFNγ, IL2, TNFα and cell surface LAMP1 expression of CDK4 TCR+ EGFP high CD8+ (A) and CD4+ (B) T cells upon co-culture with NKIRTIL006 tumor cells in the continued presence or absence of asunaprevir. Note that the tuZap70-PD1-SMASh switch yields near-equivalent inhibition of CD4+ T cell function in the absence of asunaprevir and substantially increased restoration of T cell function in the presence of asunaprevir. (C-D) Primary human T cells modified with the HLA class II restricted CMV TCR and either tuZap70-PD1-SMASh (C), or vector control (D), were pretreated with 10 µM asunaprevir or DMSO control. Data depict intracellular IFNγ, IL2, TNFα and cell surface LAMP1 expression of CD4+ CMV TCR+ EGFP high T cells upon co-culture with CMV peptide loaded CBH 5477 cells in the continued presence or absence of asunaprevir. Error bars represent standard deviation (n=3). Data are representative of two independent experiments.

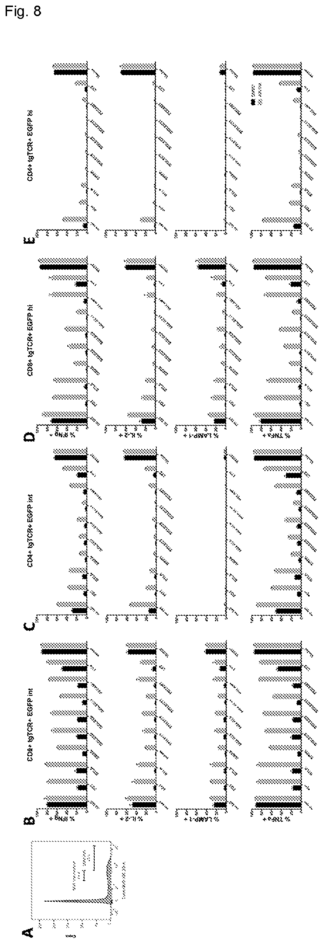

Figure 8: Different ITIM/ITSM containing inhibitory tails can be used in the CRASH-IT platform to control activity of TCR T cells. Primary human T cells modified with HLA class I restricted CDK4 TCR and CRASH-IT embodiments containing Zap70 (2xSH2)-X-SMASh, where X can be either no inhibitory tail, or inhibitory tails (or cytoplasmic domains/intracellular domains/endodomains) of PD1, BTLA, SIRPA, SIGLEC5, SIGLEC9, SIGLEC11, PECAM1 or LY9, or vector control were pretreated with 10 µM asunaprevir or DMSO control. (A) Positions of EGFP intermediate and EGFP high gating of T cells. Data depict intracellular IFNγ, IL2, TNFα and cell surface LAMP1 expression of CDK4 TCR+ EGFP intermediate (B-C) and EGFP high (D-E), CD8+ (B, D) and CD4+ (C, E) T cells upon co-culture with NKIRTIL006 tumor cells in the continued presence or absence of asunaprevir. Error bars represent standard deviation (n=3). Data are representative of two independent experiments.

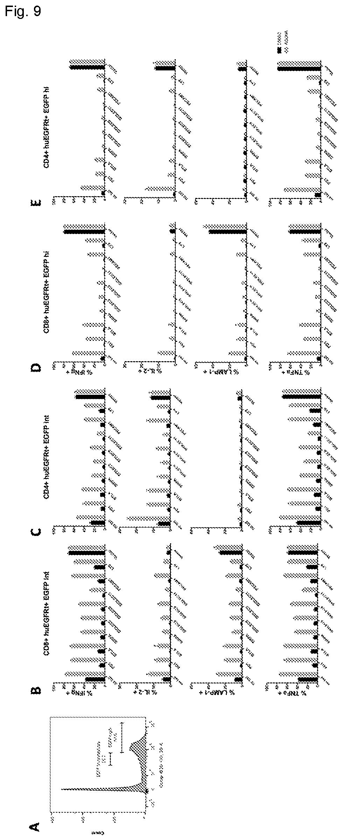

Figure 9. Different ITIM/ITSM containing inhibitory tails can be used in the CRASH-IT platform to control activity of CAR T cells. Primary human T cells modified with second generation anti-CD19-CD28-CD3ζ CAR and CRASH-IT embodiments containing Zap70 (2xSH2)-X-SMASh, where X can be either no inhibitory tail, or inhibitory tails (or cytoplasmic domains/intracellular domains/endodomains) of PD1, BTLA, SIRPA, SIGLEC5, SIGLEC9, SIGLEC11, PECAM1 or LY9, or vector control were pretreated with 10 µM asunaprevir or DMSO control. (A) Positions of EGFP intermediate and EGFP high gating of T cells. Data depict intracellular IFNγ, IL2, TNFα and cell surface LAMP1 expression of CDK4 TCR+ EGFP intermediate (B-C) and EGFP high (D-E), CD8+ (B, D) and CD4+ (C, E) T cells upon co-culture with Daudi tumor cells in the continued presence or absence of asunaprevir. Error bars represent standard deviation (n=3). Data are representative of two independent experiments.

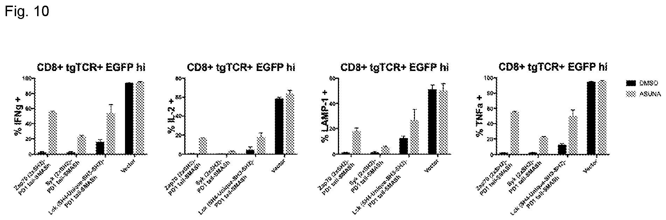

Figure 10: Alternative SH2 containing docking domains can be used in CRASH-IT platform. Primary human T cells modified with the HLA class I restricted CDK4 TCR and Zap70 (2xSH2)-PD1 tail-SMASh, Syk (2xSH2)-PD1 tail-SMASh, Lck (SH4-Unique-SH3-SH2)-PD1 tail-SMASh or vector control were pretreated with 10 µM asunaprevir or DMSO control. Data depict intracellular IFNγ, IL2, TNFα and cell surface LAMP1 expression of CD8+ CDK4 TCR+ EGFP high T cells upon co-culture with NKIRTIL006 tumor cells in the continued presence or absence of asunaprevir. Error bars represent standard deviation (n=3). Data are representative of two independent experiments.

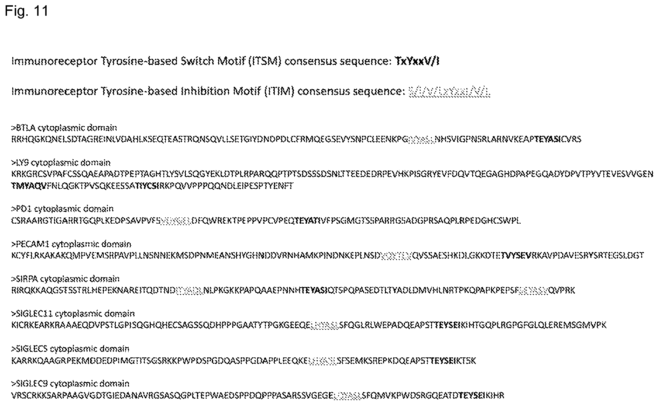

Figure 11: Comparison on Immunoreceptor Tyrosine-based Switch Motif (ITSM, depicted bold) and Immunoreceptor Tyrosine-based Inhibition Motif (ITIM, depicted underlined) sequences in the cytoplasmic domains of PD1, BTLA, SIRPA, SIGLEC5, SIGLEC9, SIGLEC11, PECAM1 and LY9. Consensus sequence of ITSM is TxYxxV/I whereas consensus sequence of ITIM is S/I/V/LxYxxI/V/L.

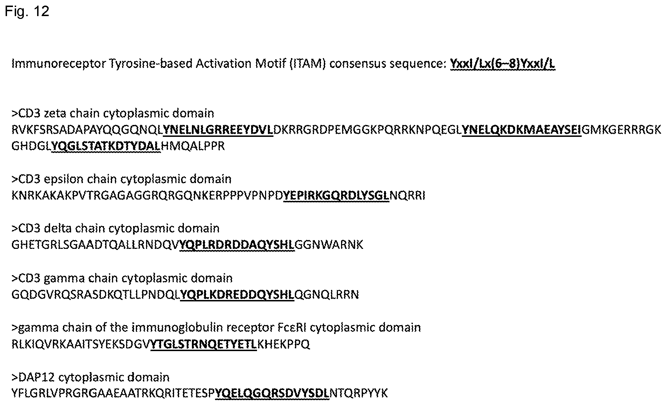

Figure 12: Comparison on Immunoreceptor Tyrosine-based Activation Motif (ITAM, depicted bold and underlined) sequences of CD3 zeta chain, CD3 epsilon chain, CD3 delta chain, CD3 gamma chain, gamma (γ) chain of the immunoglobulin receptor FcεRI and DAP12. Consensus sequence of ITAM is Yxxl/Lx(6-8)Yxxl/L.

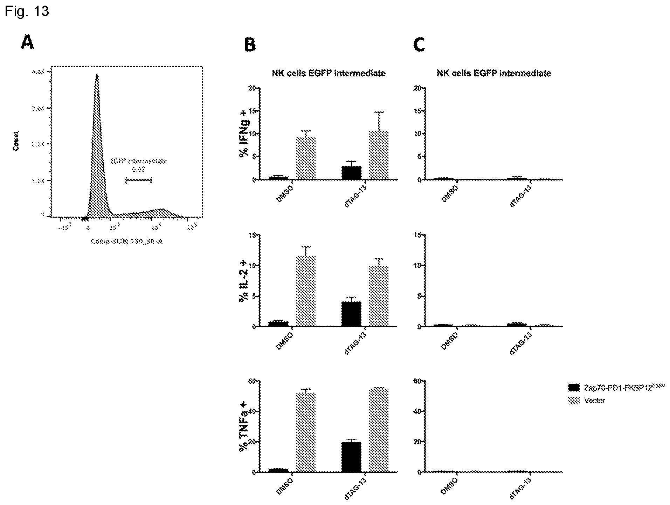

Figure 13: Control of NK cell function by CRASH-IT. The human NK cell line KHYG-1 modified with Zap70-PD1-FKBP12^{F36V} switch or vector control were pretreated with 0.5 µM dTAG-13 PROTAC or DMSO control. (A) Positions of EGFP intermediate gating of NKcells. Data depict intracellular IFNγ, IL2 and TNFα expression of EGFP intermediate NK cells upon co-culture with K562 tumor cells (B) or in the absence of K562 cells (C) in the continued presence or absence of dTAG-13. Error bars represent standard deviation (n=3). Data are representative of two independent experiments.

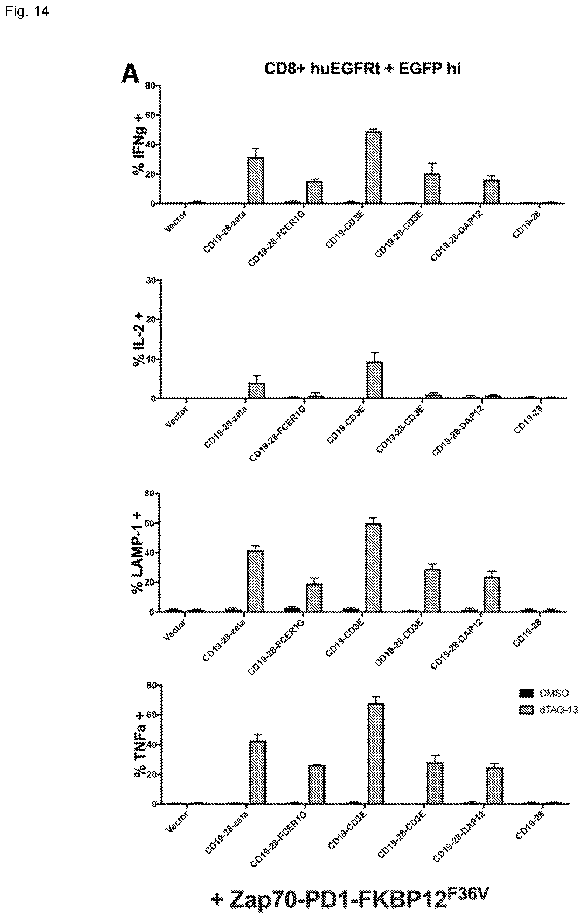

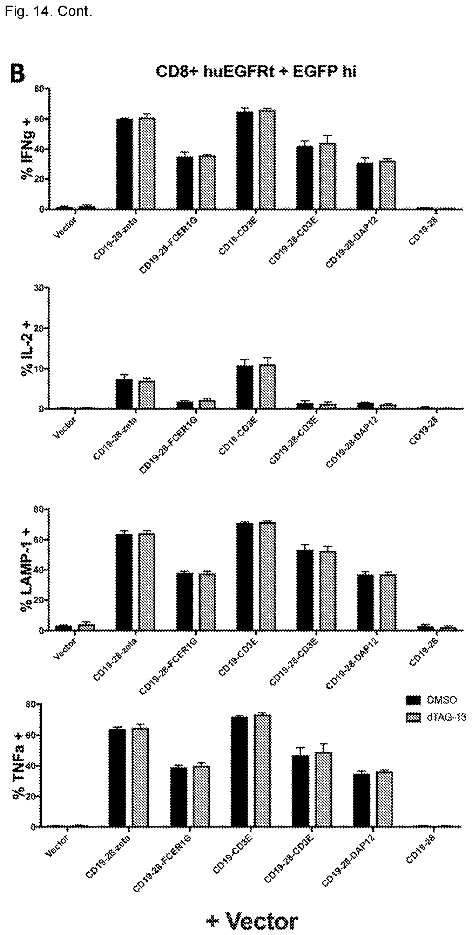

Figure 14: CRASH-IT switch can be combined with various ITAM containing CARs. Primary human T cells modified with (A) Zap70-PD1-FKBP12^{F36V} or (B) vector control and either CD19 ScFv-CD28 (hinge+TM)-CD3zeta chain, CD19 ScFv-CD28 (hinge+TM)-FCER1G, CD19 ScFv-CD3epsilon chain (full length), CD19 ScFv-CD28 (hinge+TM)-CD3 epsilon chain, or CD19 ScFv-CD28 (hinge+TM)-DAP12 CARs were pretreated with 0.5 µM dTAG-13 or DMSO control. (A-B) Data depict intracellular IFNγ, IL2, TNFα and cell surface LAMP1 expression of CAR+, EGFP high CD8+ T cells upon co-culture with Daudi tumor cells in the continued presence or absence of dTAG-13. Error bars represent standard deviation (n=3).

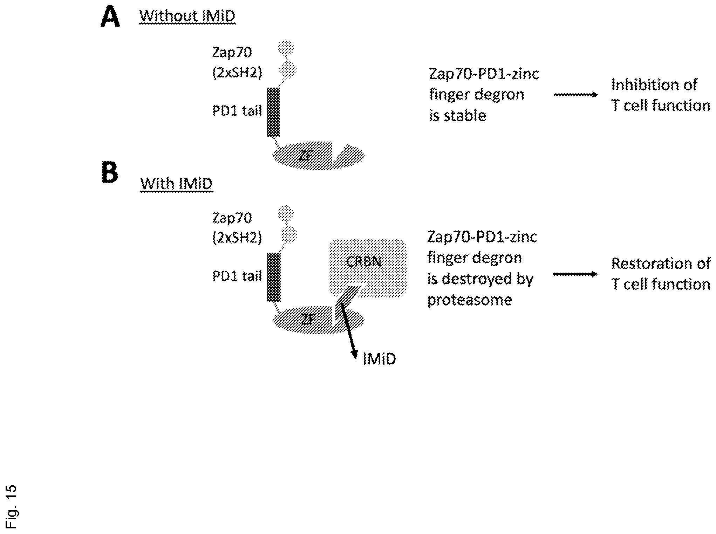

Figure 15: Schematic diagram of the Zap70-PD1-Zinc finger degron switch (as an example of embodiment according to the invention employing a CRBN polypeptide substrate domain capable of binding to the CRBN protein in response to a drug, thereby promoting ubiquitin pathway-mediated degradation of the chimeric polypeptide) in the absence (A) or presence (B) of IMiD. (A) In the absence of IMiD, such as thalidomide, lenalidomide or pomalidomide, the Zap70-PD1-Zinc finger fusion protein is stable and inhibits T cell functions. (B) Presence of IMiD induces degradation of the fusion protein via recruitment of the CRBN E3 ligase and thereby results in restoration of T cell activity.

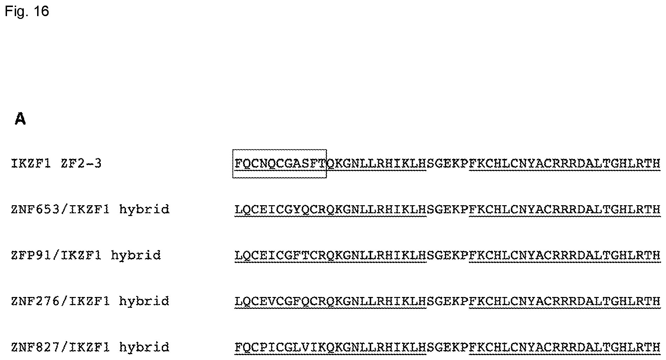

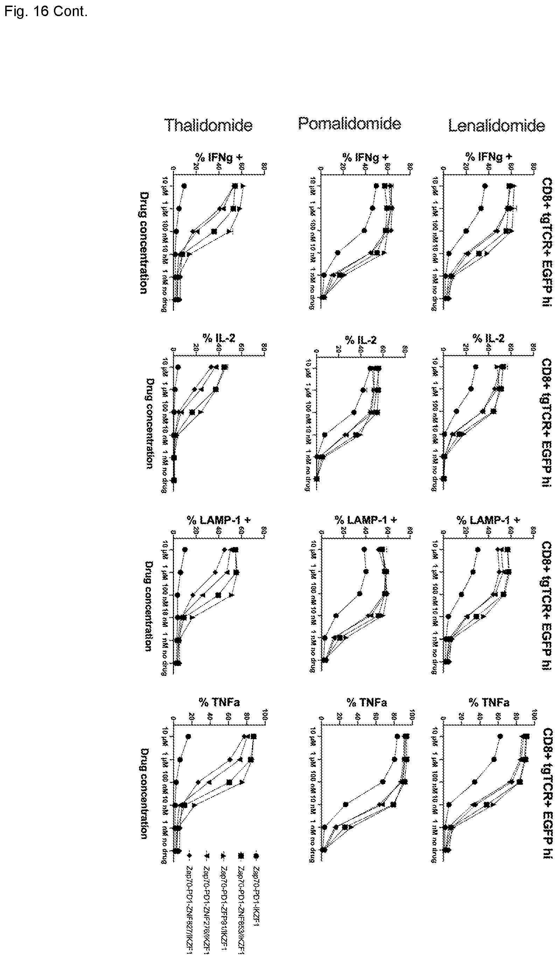

Figure 16: IMiD titration of zinc finger degron-based CRASH-IT switch expressing CD8 cells reveals designs with distinct drug sensitivity. (A) Amino acid sequences of zinc finger degrons used. The top zinc finger degron is derived from the IKZF1 ZF2-3 sequence. The IKZF1 ZF2 beta turn sequence (shown inside rectangle) was replaced with beta turn sequences from ZNF653 ZF4, ZFP91 ZF4, ZNF276 ZF4, or ZNF827 ZF1, to create the hybrid zinc fingers with improved IMiD sensitivity depicted below. Individual C2H2 zinc finger sequences are underlined. Primary human T cells modified with the CDK4 TCR and indicated Zap70-PD1-zinc finger degrons were pretreated with indicated concentrations of lenalidomide (B), pomalidomide (C), or thalidomide (D). Data depict intracellular IFNγ, IL2, TNFα and cell surface LAMP1 expression of CDK4 TCR+, EGFP high CD8+ T cells upon co-culture with NKIRTIL006 tumor cells in the continued presence of indicated concentrations of lenalidomide, pomalidomide or thalidomide. Error bars represent standard deviation (n=2).

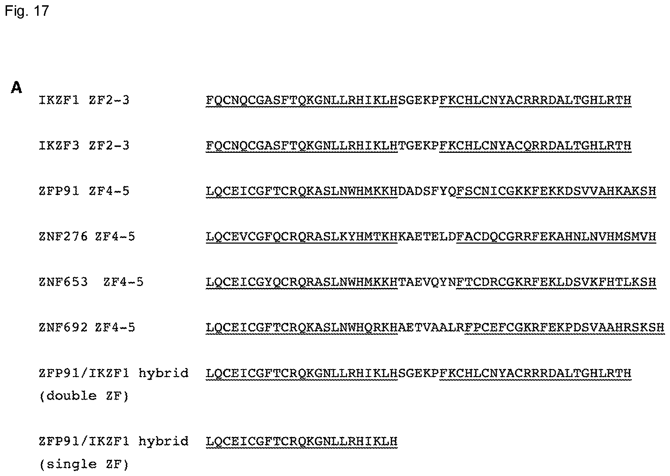

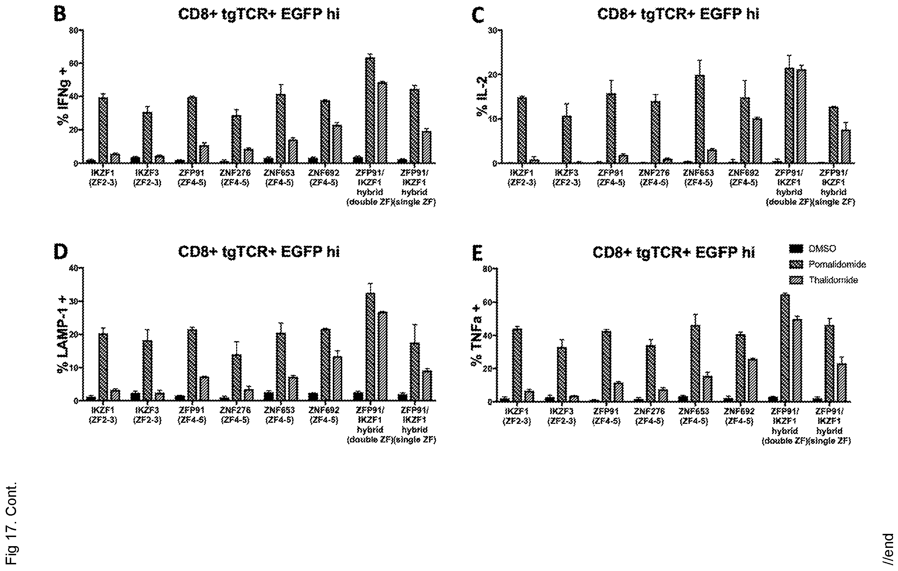

Figure 17: Hybrid ZFP91/IKZF1 (double ZF) based CRASH-IT switch is highly efficient in restoring T cell functions in the presence of IMiDs. (A) Amino acid sequences of zinc finger degrons used in the experiment. Indicated wild type zinc finger degrons derived from IKZF1, IKZF3, ZFP91, ZNF276, ZNF653, ZNF692 (all double ZF), or the hybrid ZFP91/IKZF1 zinc finger degron with or without IKZF1 ZF3 (double ZF and single ZF, respectively) were used in the experiment. Individual C2H2 zinc finger sequences are underlined. Primary human T cells modified with the CDK4 TCR and indicated Zap70-PD1-zinc finger degrons were pretreated with 0.5 µM pomalidomide, 0.5 µM thalidomide, or DMSO control. Data depict intracellular IFNγ, IL2, TNFα and cell surface LAMP1 expression of CDK4 TCR+, EGFP high CD8+ T cells upon co-culture with NKIRTIL006 tumor cells in the continued presence or absence of pomalidomide or thalidomide. Error bars represent standard deviation (n=2).

### Reference to a Sequence listing

The Sequence listing, which is a part of the present disclosure, includes a text file comprising nucleotide and/or amino acid sequences of the present invention. The subject matter of the Sequence listing is incorporated herein in its entirety. The information recorded in computer readable form is identical to the written sequence listing.

### Definitions

The section headings used herein are for organizational purposes only and are not to be construed as limiting the subject matter described.

A portion of this disclosure contains material that is subject to copyright protection (such as, but not limited to, diagrams, device photographs, or any other aspects of this submission for which copyright protection is or may be available in any jurisdiction.). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular form terms "a," "an," and "the" include plural referents unless the content clearly dictates otherwise. Thus, for example, reference to "a cell" includes a combination of two or more cells, and the like.

As used herein, the term "and/or" refers to a situation wherein one or more of the stated cases may occur, alone or in combination with at least one of the stated cases, up to with all of the stated cases.

As used herein, the term "at least" a particular value means that particular value or more. For example, "at least 2" is understood to be the same as "2 or more" i.e., 2, 3, 4, 5, 6, 7, 8, 9, 10, 11, 12, 13, 14, 15, etc. As used herein, the term "at most" a particular value means that particular value or less. For example, "at most 5" is understood to be the same as "5 or less" i.e., 5, 4, 3, ....-10, -11, etc.

As used herein, the term "comprising" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of'."

As used herein, "conventional techniques" or "methods known to the skilled person" refer to a situation wherein the methods of carrying out the conventional techniques used in methods of the invention will be evident to the skilled worker. The practice of conventional techniques in molecular biology, biochemistry, cell culture, genomics, sequencing, medical treatment, pharmacology, immunology and related fields are well-known to those of skill in the art, and are discussed, in various handbooks and literature references.

As used herein, the term "exemplary" means "serving as an example, instance, or illustration," and should not be construed as excluding other configurations disclosed herein.

As used herein, the term "cancer" refers to the physiological condition in mammals that is typically characterized by unregulated cell growth. The terms "cancer," "neoplasm," and "tumor" are often used interchangeably to describe cells that have undergone a malignant transformation that makes them pathological to the host organism. Primary cancer cells can be distinguished from non-cancerous cells by techniques known to the skilled person. A cancer cell, as used herein, includes not only primary cancer cells, but also cancer cells derived from such primary cancer cell, including metastasized cancer cells, and cell lines derived from cancer cells. Examples include solid tumors and non-solid tumors or blood tumors. Examples of cancers include, without limitation, leukemia, lymphoma, sarcomas and carcinomas (e.g. colon cancer, pancreatic cancer, breast cancer, ovarian cancer, prostate cancer, lung cancer, melanoma, lymphoma, non-Hodgkin lymphoma, colon cancer, (malignant) melanoma, thyroid cancer, papillary thyroid carcinoma, lung cancer, non-small cell lung carcinoma, and adenocarcinoma of lung.). As is well known, tumors may metastasize from a first locus to one or more other body tissues or sites. Reference to treatment for a "neoplasm, "tumors" or "cancer" in a patient includes treatment of the primary cancer, and, where appropriate, treatment of metastases.

As used herein, the terms "chimeric gene" or "chimeric nucleic acid" refer to any gene or nucleic acid which is not normally found in nature in a species, in particular a gene or nucleic acid in which one or more parts of the nucleotide sequence are not associated with each other in nature. For example the promoter is not associated in nature with part or all of the transcribed region or with another regulatory region, or different parts of the transcribed region are not associated in nature. The term "chimeric gene" is understood to include expression constructs in which a promoter or transcription regulatory sequence is operably linked to one or more coding sequences. A chimeric gene of chimeric nucleic acid can, in some embodiments, be used to make a chimeric protein.

As used herein, the terms "protein" and "polypeptide" refer to molecules consisting of a chain of amino acids, without reference to a specific mode of action, size, three dimensional structure or origin. A "fragment" or "portion" or "part" of a polypeptide may thus still be referred to as a "polypeptide". An "isolated protein" or isolated polypeptide" is used to refer to a protein or polypeptide which is no longer in its natural environment, for example in vitro or in a recombinant host cell.

As used herein the terms "chimeric polypeptide", "chimeric protein", or "fusion protein" refer to any polypeptide which is not normally found in nature is a species, in particular a polypeptide in which one or more part of the amino acids sequence are not associated with each other in nature. For example, the chimeric polypeptide may comprise an N-terminal part consisting of a first sequence of amino acids and a C-terminal part consisting of a second sequence of amino acids that are not associated with each other in nature and/or are not associated with each other in nature in this order. A chimeric polypeptide may for example be obtained from transcription and translation of a chimeric gene of nucleic acid.

As used herein the term "nucleic acid" or "polynucleotide" refers to any polymers or oligomers of (contiguous) nucleotides. The nucleic acid may be DNA or RNA, or a mixture thereof, and may exist permanently or transitionally in single-stranded or double-stranded form, including homoduplex, heteroduplex, and hybrid states. The present invention contemplates any deoxyribonucleotide, ribonucleotide or peptide nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. The term "isolated" thus means isolated from naturally occurring sources or artificially or synthetically produced.

As used herein, the term "pharmaceutical composition" refers to pharmaceutically acceptable compositions which comprise a therapeutically-effective amount of material formulated together with one or more pharmaceutically acceptable carriers (additives) and/or diluents.

As used herein, "therapy" or "treatment" refers to treatment of a tumor with a therapeutic agent (including biological materials and cells) or drug. A treatment may involve administration of more than one drug. A drug may be administered alone or in combination with other treatments, either simultaneously or sequentially dependent upon the condition to be treated. For example, the therapy may be a co-therapy involving administration of two drugs/agents, one or more of which may be intended to treat the tumor. The treatment regime may be a pre-determined timetable, plan, scheme or schedule of therapy administration which may be prepared by a physician or medical practitioner and may be tailored to suit the patient requiring treatment. The treatment regime may indicate one or more of: the type of therapy to administer to the patient; the dose of each drug; the time interval between administrations; the length of each treatment; the number and nature of any treatment holidays, if any etc. For a co-therapy a single treatment regime may be provided which indicates how each drug/agent is to be administered.

As used herein, the term "patient" or "individual" or "subject" refers to a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the patient, individual, or subject is a human. In some embodiments, the patient may be a "cancer patient," i.e. one who is suffering or at risk for suffering from one or more symptoms of cancer.

As used herein, the term "vector" as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### Detailed description

The invention is defined in the accompanying claims. It is contemplated that any method, use or composition described herein can be implemented with respect to any other method, use or composition described herein. Embodiments discussed in the context of methods, use and/or compositions of the invention may be employed with respect to any other method, use or composition described herein. Thus, an embodiment pertaining to one method, use or composition may be applied to other methods, uses and compositions of the invention as well.

As embodied and broadly described herein, the present invention is directed to the surprising finding that with a chimeric polypeptide as disclosed herein it has now become possible to control T cell activity, in particular to control cytotoxic activity of T cells and/or to control cytokine secretion by such T cells, for example CD4 or CD8 positive T cells, and to control natural killer cell activity, in particular to control cytotoxic activity of NK cells and/or to control cytokine secretion by such NK cells.

More in particular, the present inventors have developed an innovative system for modulating and/or manipulating signal transduction pathways in T cells and NK cells. The system allows for the time and/or dose dependent regulation of T cell activity as the consequence of signaling via T cell receptor (TCR) and/or chimeric antigen receptor (CAR) and for the time and/or dose dependent regulation of NK cell activity as the consequence of signaling via NK cell receptor (NKR) and/or chimeric antigen receptor (CAR). The system can suitably be used for any cell expressing a TCR and/or a CAR, including natural T cells or T cells manipulated to express a particular (modified) TCR and/or CAR and/or any cell expressing an NKR and/or a CAR, including natural or manipulated NK cells. Therefore, according to embodiments of the invention, the cell according to the invention is a lymphocyte, in particular a T cell or NK cell. Such T cell and/or NK cell is to be understood to include any "modified" T cell or NK cell, for example CAR T cells, CAR NK cells, multiple CAR T cells, multiple CAR NK cells, tandem CAR T cells, tandem CAR NK cells, transgenic TCR T cells, and transgenic TCR NK cells.

The current disclosure provides for tight regulation of T cell and NK cell activity (e.g. cytotoxic activity and/or cytokine secretion) by the chimeric polypeptide according to the invention due to the presence of a small molecule-regulated protein stability domain that is utilized to modulate (e.g., reduce or increase) in a time and/or dose dependent manner the expression of the chimeric polypeptide according to the invention.

The chimeric polypeptide is designed to interact with phosphorylated immunoreceptor tyrosine-based activation motifs (ITAM) in the TCR/CD3 complex and/or CAR and/or NK cell receptor (NKR) complexes that contain ITAM bearing signaling molecules such as DAP12, gamma (γ) chain of the immunoglobulin receptor FcεRI or CD3 zeta chain (Lanier et al, Nat Immunol. 2008 May; 9(5): 495-502).

The tyrosine residues within these ITAM motifs become phosphorylated following interaction of the receptor molecules with their ligands and form docking sites for other proteins involved in the signaling pathways of the cell. By the interaction of the chimeric polypeptide according to the invention with the TCR and/or CAR, the T cell activation (and subsequent cytotoxic effects and/or cytokine secretion) is inhibited. Similarly, by the interaction of the chimeric polypeptide according to the invention with the NKR and/or CAR in the NK cells, the NK cell activation (and subsequent cytotoxic effects and/or cytokine secretion) of the NK cells is inhibited.

According to a preferred embodiment, the chimeric polypeptide comprises, next to a small molecule-regulated protein stability domain and a domain that interacts with the phosphorylated ITAM motifs, also an immunoreceptor tyrosine-based switch motif (ITSM), preferably an ITSM and an immunoreceptor tyrosine-based inhibitory motif (ITIM). Such motifs are, for example, present in the inhibitory tail of PD1 and suggested to be implicated in its immunosuppressive effects of PD1 (Boussiotis et al, Cancer J. 2014 Jul-Aug; 20(4): 265-271). It was surprisingly found that the presence of such ITSM, preferably such ITSM and such ITIM, in the chimeric polypeptide, allows the chimeric polypeptide to effectively inhibit signal transduction by, for example, the TCR, NKR or CAR (upon ligand binding to the receptor). Here we show that use can be made of these ITSM, preferably ITSM and ITIM as present in, for example, the inhibitory tails of inhibitory immune receptor protein like PD1, to inhibit TCR and/or CAR signaling in T cells and NKR signaling in NK cells without the need for the presence of the extracellular domain of the inhibitory proteins to be present or to be interaction with their ligands (e.g. PD-L1 for PD1). In combination with the small molecule-regulated protein stability domain that allows for dose-dependent expression of the chimeric polypeptide in a cell, e.g. a T cell, the system thus provides for an efficient and reliable manner to precisely regulate T cell function, e.g. T cell activation, T cell cytotoxicity and/or T cell cytokine secretion. In this way, T cell function can be prevented/inhibited or be activated, leading to a maintained, regained, safe and controllable functionality of T cells, both in vitro and in vivo (i.e. in treatments of cancer or other conditions like autoimmune diseases that depend on the use of T cells, including T cells expressing a (modified) TCR and/or CAR)./pct

Similarly, NK cell function can be prevented/inhibited or be activated, leading to a maintained, regained, safe and controllable functionality of NK cells, both in vitro and in vivo (i.e. in treatments of cancer or other conditions like autoimmune diseases that depend on the use of NK cells, including NK cells expressing a (modified) NKR and/or CAR).

Therefore, as described herein, there is provided for a cell comprising a chimeric polypeptide, or a nucleic acid comprising a polynucleotide encoding a said chimeric polypeptide, wherein the chimeric polypeptide comprises:
a) a first part comprising an SH2-domain from a protein which binds a phosphorylated immunoreceptor tyrosine-based activation motif (ITAM); and
b) a second part comprising a small molecule-regulated protein stability domain.

The cell according to the invention may be any cell that can suitably comprise a chimeric polypeptide as disclosed herein or nucleic acid encoding such chimeric polypeptide. In some embodiments, the cell is a prokaryotic cell. In some embodiments, the cell is a eukaryotic cell. Preferably the cell is a eukaryotic cell, even more preferably a mammalian cell, such as a human cell. The cell may be a specialized cell, such as a T cell or NK cells, or be any other type of cell, including (undifferentiated) stem cells.

The polypeptide according to the invention is a chimeric polypeptide that comprises at least a first part and a second part. The order wherein the first and second part are present in the chimeric peptide is not critical. The invention is not limited by the location of the first part, second part, or third part in the chimeric polypeptide. For example, in some embodiments, the first part or second part is fused (e.g., genetically linked) on the N- or the C- terminus of the chimeric polypeptide, or present internally in the chimeric polypeptide. In other words, in the chimeric polypeptide, the first part and/or the second part may be at the C-terminus, at the N-terminus, or may be flanked at the C-terminus and/or at the N-terminus by additional parts. The first part may, relative to the second part, be more towards the C-terminus, or may, relative to the second part, be more towards the N-terminus.

The first and second part may be directly adjacent to each other or may be separated from each other by additional parts, i.e. additional (stretches of) amino acid residues.

The chimeric polypeptide as disclosed herein is characterized by the presence of a first part that comprises an SH2-domain from a protein which binds a phosphorylated immunoreceptor tyrosine-based activation motif (ITAM). As can be witnessed from the Examples, the SH2-domain can be any SH2-domain from a protein which can bind to a phosphorylated ITAM. The skilled person is well-aware of suitable SH2 domain for use in the chimeric polypeptide as disclosed herein and/or is readily capable of identifying such suitable SH2 domain or proteins that would comprise such SH2 domain capable of binding to a phosphorylated (ITAM). As will be understood by the skilled person a suitable SH2-domain may be selected in view of the ITAM that is comprised in, for example, the TCR/CD3 complex and/or CAR and/or NK cell receptor (NKR) complexes that the chimeric polypeptide according to the invention is designed for. In other words, the chimeric protein according to the invention comprises an SH2-domain from a protein which binds to a phosphorylated immunoreceptor tyrosine-based activation motif (ITAM), and wherein said ITAM is comprised in, for example, the TCR or CAR complex that is to be targeted in the context of the current invention.

As used herein the term "SH2 domain" refers to a SRC Homology 2 domain. The SH2 domain is a structurally conserved protein domain contained within the Src oncoprotein and in many other intracellular signal-transducing proteins. SH2 domains allow proteins containing those domains to dock to phosphorylated tyrosine residues on other proteins. SH2 domains are thus modular protein domains that serve as adaptors and mediate protein-protein interactions by binding to phosphorylated peptides in their respective protein binding partners.

SH2 domains typically bind a phosphorylated tyrosine residue in the context of a longer peptide motif within a target protein. SH2 domains as such lack any intrinsic catalytic activity but they serve to localize coupled functional domains in the polypeptide to the vicinity of appropriate substrates, activators or inhibitors (Ngoenkam et al, Immunology. 2018 Jan; 153(1): 42-50).

Some SH2 domains may, for example, interact with a protein having a phosphorylated immunoreceptor tyrosine-based activation motif (ITAM), while other SH2 domain interact with a protein having a phosphorylated immunoreceptor tyrosine-based inhibition motif (ITIM). In the current invention an SH2-domain from a protein which binds a phosphorylated immunoreceptor tyrosine-based activation motif (ITAM) is used. It was surprisingly found that, while SH2 domains from proteins that binds to a phosphorylated ITAM are important for regulation of T cell activity or NK cell activity by the chimeric peptide as disclosed herein, SH2 domains that interact with ITIM are not or to a lesser extent suitable for use in the chimeric polypeptide according to the invention.

In a preferred embodiment the SH2 domain is from a protein which binds a phosphorylated immunoreceptor tyrosine-based activation motif (ITAM) that is present in a TCR complex, an NKR complex and/or a CAR.

In some embodiments, the SH2 domain is an SH2 domain annotated as BetaA-AlphaA-BetaB-BetaC-BetaD-BetaE-BetaF-AlphaB-BetaG by Liu et al (Mol Cell. 2006;22(6):851-868. doi:10.1016/j.molcel.2006.06.001) for 120 known human SH2 domains; with Beta referring to a beta-strand and alpha referring to an alpha helix (see also Eck et al. Nature. 1993;362(6415):87-91. doi:10.1038/362087a0). SH2 domains can, for example, be found in various databases well-known to the skilled person (see e.g smart.embl.de/smart/do_annotation.pl?DOMAIN=SM00252 or www.ebi.ac.uk/interpro/entry/InterPro/IPR000980/).

As used here, the term "immunoreceptor tyrosine-based activation motif (ITAM)" refers to a conserved sequence of four amino acids that is repeated twice and is present in the cytoplasmic tails (i.e. endodomains) of certain cell surface proteins of the immune system. A half-ITAM comprises a tyrosine residue (Y) separated from a leucine residue (L) or isoleucine residue (I) by any two other amino acids. The consensus sequence of half-ITAM is YxxL/I. The two half-ITAMs are normally separated from each other by 6 to 8 amino acids to form a full ITAM. The consensus sequence of ITAM is YxxL/Ix(6-8)YxxL/I. ITAMs play an important role in signal transduction in immune cells and they are found in the cytoplasmic tails of cell signaling molecules in the T cell receptor complex (CD3 epsilon chain, CD3 delta chain, CD3 gamma chain and/or CD3 zeta chain). In NK cells, ITAMs are present in NK cell receptor complexes that contain CD3 zeta chain, gamma (γ) chain of the immunoglobulin receptor FcεRI and DAP12 (Lanier et al, Nat Immunol. 2008 May; 9(5): 495-502). ITAMs are also present in chimeric antigen receptor (CAR) complexes that comprise CD3 zeta chain (Abate-Daga et al, Mol Ther Oncolytics. 2016; 3: 16014), CD3 epsilon chain (Nolan et al, Clin Cancer Res. 1999 Dec;5(12):3928-41), gamma (γ) chain of the immunoglobulin receptor FcεRI (Ren-Heidenreich et al, Cancer Immunol Immunother. 2002 Oct;51(8):417-23) and DAP12 (Töpfer et al, J Immunol. 2015 Apr 1;194(7):3201-12).

The tyrosine residues in the ITAM motifs become phosphorylated following interaction of the receptor molecules with their ligands and form docking sites for other proteins involved in the signaling pathways of the cell. Once phosphorylated, SH2 domain comprising proteins which bind to such phosphorylated ITAM can interact with the phosphorylated ITAM, for example, present in CD3 zeta chain. Several proteins are known to contain endodomains with one or more ITAM motifs. Examples of such proteins include the CD3 gamma chain, the CD3 delta chain and the CD3 epsilon chain, CD3 zeta chain, gamma (γ) chain of the immunoglobulin receptor FcεRI and DAP12.

The chimeric polypeptide as disclosed herein is further characterized by the presence of a second part that comprises a small molecule-regulated protein stability domain. The small molecule-regulated protein stability domain may also be referred to as a regulatable destabilization domain. This small molecule-regulated protein stability domain or regulatable destabilization domain in the chimeric polypeptide of the invention is utilized to modulate (e.g., reduce or increase) in a time and/or dose dependent manner the expression of the chimeric polypeptide of the invention.

In more detail, the small molecule-regulated protein stability domain or regulatable destabilization domain in the chimeric polypeptide of the invention is a domain that can be regulated by the provision of a compound (e.g. small molecule) to the cell comprising the chimeric polypeptide. By the addition of the compound, the small molecule-regulated protein stability domain or regulatable destabilization domain in the chimeric polypeptide is modified to cause the breakdown of the chimeric polypeptide.

In other words, in response to such compound, and via interactions of the compound with the small molecule-regulated protein stability domain or regulatable destabilization domain in the chimeric polypeptide, the chimeric polypeptide according to the invention will be degraded, leading to a decrease in the level or concentration of the chimeric polypeptide in the cell. In turn, by reducing the level or concentration of the chimeric polypeptide in the cell, inhibition of T cell function, e.g. T cell activation, T cell toxicity and/or secretion of cytokines by the T cell as the consequence of signaling via the TCR and/or CAR is reversed.

In the same way, in NK cells by reducing the level or concentration of the chimeric polypeptide in the cell, inhibition of NK cell function, e.g. NK cell activation, NK cell toxicity and/or secretion of cytokines by the NK cell as the consequence of signaling via the NKR and/or CAR is reversed. In this way T cell function and/or NK cell function can be modulated by the chimeric polypeptide of the invention. In this way T cell function and/or NK cell function can be modulated by providing to the cell a compound that interacts with the small molecule-regulated protein stability domain or regulatable destabilization domain in the chimeric polypeptide, causing the breakdown of the chimeric polypeptide of the invention.

It was surprisingly found that with the chimeric polypeptide of the invention T cell function or NK cell function can, via the small molecule-regulated protein stability domain or regulatable destabilization domain in the chimeric polypeptide, be modulated in a reversible way and/or in a dose-dependent way (see Examples). In absence of a compound that interacts with the small molecule-regulated protein stability domain or regulatable destabilization domain, whose interaction causes the breakdown of the chimeric polypeptide, the chimeric polypeptide of the invention inhibits T cell function by inhibiting signaling via the TCR and/or CAR as the consequence of interaction of the chimeric polypeptide with the TCR/CD3 complex and/or CAR.

Similarly, in the absence of a compound that interacts with the small molecule-regulated protein stability domain or regulatable destabilization domain, and which in that case causes the breakdown of the chimeric polypeptide, the chimeric polypeptide of the invention inhibits NK cell function by inhibiting signaling via the NKR and/or CAR as the consequence of interaction of the chimeric polypeptide with the NKR complexes containing ITAM bearing signaling molecules such as CD3 zeta chain, gamma (γ) chain of the immunoglobulin receptor FcεRI and DAP12 and/or CAR. In the presence of such compound, the chimeric polypeptide will be directed towards the protein degradation systems in the cell, and therewith release the inhibition of T cell function and/or NK cell function; which release of inhibition of T cell function and/or NK cell function is dose-dependent, as can be witnessed from the Examples.

Alternatively, a small molecule-regulated protein stability domain or regulatable destabilization domain may be used that direct degradation of the chimeric protein of the invention in the absence of the small molecule. In such embodiments, the presence of the small molecule causes stabilization of the chimeric protein and thereby inhibition of T cell or NK cell function, and withdrawal of the small compound would restore activity of the T cell or NK cell.

The invention is not limited to any particular small molecule-regulated protein stability domain or regulatable destabilization domain. Indeed, any small molecule-regulated protein stability domain or regulatable destabilization domain that confers chimeric polypeptide stability (Röth et al, Cell Mol Life Sci. 2019 Jul;76(14):2761-2777) such that chimeric polypeptide degradation occurs when the small molecule-regulated protein stability domain or regulatable destabilization domain in the chimeric polypeptide is modified to direct the chimeric polypeptide to degradation by the presence of its cognate small molecule may be used in the invention. The skilled person is well-aware of such suitable small molecule-regulated protein stability domain or regulatable destabilization domain. A non-limiting example of small molecule-regulated protein stability domain or regulatable destabilization domain is a self-excising degron (SED), wherein the SED comprises a repressible protease, a cognate cleavage site, and a degron sequence (Chung et al, Nat Chem Biol. 2015 Sep;11(9):713-20) or a proteolysis-targeting chimera (Protac) binding domain. The Protac that in turn can bind to the Protac binding domain in the chimeric polypeptide according to the invention comprises a E3 ubiquitin ligase binding group (E3LB), a linker, and a protein binding group that binds to the Protac binding domain in the chimeric polypeptide (Nabet et al, Nat Chem Biol. 2018 May;14(5):431-441, An et al, EBioMedicine. 2018 Oct; 36: 553-562). A preferred example of a small molecule-regulated protein stability domain or regulatable destabilization domain for use in the current invention is broadly referred to as a CRBN polypeptide substrate domain capable of binding to the CRBN protein in response to a drug, thereby promoting ubiquitin pathway-mediated degradation of the chimeric polypeptide. Typical examples of such small molecule-regulated protein stability domain include so-called zinc finger degrons.

Small molecule IMiDs such as thalidomide, lenalidomide, and pomalidomide in vivo induce the ubiquitination and proteasomal degradation of transcription factors like Ikaros (IKZF1) and Aiolos (IKZF3) by recruiting these Cys2-His2 (C2H2) zinc finger domain containing proteins to Cereblon (CRBN), the substrate receptor of the CRL4CRBN E3 ubiquitin ligase. Such zinc finger domains (zinc finger degrons) can be used to target heterologous proteins for degradation in a time- and dose-dependent manner, both ex vivo and in vivo upon providing an IMiD to cells expressing such zinc-finger domain comprising heterologous protein (See Koduri et al PNAS (2019) 116 (7) 2539-2544; doi.org/10.1073/pnas. 1818109116).

In fact, a large number of possible zinc finger polypeptides and zinc finger domains have been implemented in mediating small molecule mediated, e.g. IMiDs (such as thalidomide, lenalidomide, and pomalidomide) mediated degradation of targeted proteins (see, for example, Sievers et al. Science. 2018 Nov 2; 362(6414): eaat0572.doi: 10.1126/science.aat0572). In view thereof, the skilled person is well acquainted with the use and design of such CRBN polypeptide substrate domains capable of binding to the CRBN protein in response to a drug, thereby promoting ubiquitin pathway-mediated degradation of the chimeric polypeptide, in particular wherein the CRBN polypeptide substrate domain is a C2H2 zinc finger protein or a fragment thereof that is capable of drug-inducible binding to the CRBN polypeptide, and as detailed herein.

According to the invention , there is provided for a cell of the invention wherein the chimeric polypeptide comprises a third part comprising an immunoreceptor tyrosine-based switch motif (ITSM), an immunoreceptor tyrosine-based inhibitory motif (ITIM), or preferably an ITSM and an immunoreceptor tyrosine-based inhibitory motif (ITIM). In some alternative embodiments, the third part comprises an immunoreceptor tyrosine-based switch motif (ITSM) and/or an immunoreceptor tyrosine-based inhibitory motif (ITIM), preferably an ITSM and an immunoreceptor tyrosine-based inhibitory motif(ITIM).

Preferably the third part comprises an immunoreceptor tyrosine-based switch motif (ITSM), preferably an ITSM and an immunoreceptor tyrosine-based inhibitory motif (ITIM).

The order wherein the first, second and third part are present in the chimeric peptide is not critical. The invention is generally not limited by the location of the first part, second part, or third part in the chimeric polypeptide. For example, in some embodiments, the first part, second part or third part is fused (e.g., genetically linked) on the N- or the C- terminus of the chimeric polypeptide, or present internally in the chimeric polypeptide. In other words, in the chimeric polypeptide, the first, second or third part may be at the C-terminus, at the N-terminus, or may be flanked at the C-terminus and/or at the N-terminus by other parts. Examples of suitable order of the first part (P1), second part (P2), and third part (P3) may in general be xP1xP2xP3x, xP1xP3xP2x, xP2xP1xP3x, xP2xP3xP1x, xP3xP1xP2x, or xP3xP2xP1x, wherein x at any position may refer, independently to the presence of no additional amino acid residue or the presence of one or more additional amino acid residues that do not form part of P1, P2 and/or P3. With respect to the order of the first and second part, and in analogy with the example above, examples of suitable order of the first part (P1) and the second part (P2) may in general be xP1xP2x, or xP2xP1x, wherein x at any position may refer, independently to the presence of no additional amino acid residue or the presence of one or more additional amino acid residues that do not form part of P1 and/or P2.

At the same time, it will be understood by the skilled person that the first part may, in addition to the SH2-domain from a protein which binds a phosphorylated immunoreceptor tyrosine-based activation motif (ITAM), comprise further domains or amino acids, for example one or several of the amino acids that normally flank (on one or on both sides) the SH2 domain in a protein which binds a phosphorylated immunoreceptor tyrosine-based activation motif (ITAM).

At the same time, it will be understood by the skilled person that the second part may, in addition to the small molecule-regulated protein stability domain, comprise further domains or amino acids on one or on both sides.

At the same time, it will be understood by the skilled person that the third part may, in addition to the immunoreceptor tyrosine-based switch motif (ITSM), preferably an ITSM and an immunoreceptor tyrosine-based inhibitory motif (ITIM), comprise further domains or amino acids, for example one or several of the amino acids that normally flank these motifs (on one or on both sides).

The skilled person will understand that the first, second and third part of the chimeric polypeptide of the invention may be present in the chimeric polypeptide in any order as long as the small molecule-regulated protein stability domain or regulatable destabilization domain is positioned in the chimeric polypeptide as such that upon contact with the compound that interacts with the small molecule-regulated protein stability domain or regulatable destabilization domain, the chimeric polypeptide is degraded and the inhibition of T cell function and/or NK cell function via interaction of the chimeric polypeptide with the TCR and/or CAR is released due to the breakdown of the chimeric polypeptide.

The terms "ITIM" and/or "ITSM" are known to the skilled person (Liu et al, Mol Cell Proteomics. 2015 Jul;14(7):1846-58).

As used herein, the term "immunoreceptor tyrosine-based inhibitory motif (ITIM)" refers generally to a conserved sequence of amino acids that is found in the cytoplasmic tails of many inhibitory receptors of the immune system. The ITIM motif comprises a serine residue (S), an isoleucine residue (I), a valine residue (V) or a leucine residue (L), separated by any other amino acid residue (x) from a tyrosine residue (Y), separated by any two other amino acids from an isoleucine residue (I), valine residue (V) or leucine residue (L). The consensus signature is S/I/V/LxYxxI/V/L. In vivo, ITIM-possessing inhibitory receptors interact with their ligand, causing the ITIM motif to become phosphorylated by enzymes of the Src kinases, allowing them to recruit SH2 containing protein tyrosine phosphatases (PTP) such as SHP-1 and SHP-2 (Coxon et al, Blood. 2017 Jun 29;129(26):3407-3418), and lipid phosphatases such as SHIP-1. PTPs oppose positive regulatory effect of protein tyrosine kinases (PTK) such as Lck and Zap70, thereby negatively regulate T cell signaling (Lorenz et al, Immunol Rev. 2009 Mar; 228(1): 342-359). By dephosphorylating ITAMs in TCRs, CARs, and other immune receptors, PTPs can reverse the activating effects of ITAM phosphorylation. Lipid phosphatases regulate cell signaling by modifying the concentrations of lipid phosphates versus their dephosphorylated products.

As used herein, the term "immunoreceptor tyrosine-based switch motif (ITSM)" refers to a conserved sequence of amino acids that is found in the cytoplasmic tails (or cytoplasmic domains or intracellular domain or endodomain; in other words, that part of the protein that is present in the cytoplasm of the cell (and not in the membrane and/or extracellular space) of many inhibitory receptors of the immune system. The ITSM motif comprises a threonine residue (T), separated by any other amino acid residue from a tyrosine residue (Y), separated by any other two amino acids from a valine residue (V) or an isoleucine residue (I). The consensus signature is TxYxxV/I. Similar to ITIM possessing inhibitory receptors, ITSM-possessing inhibitory receptors interact with their ligand, causing the ITIM motif to become phosphorylated by enzymes of the Src kinases, allowing them to recruit SH2 containing phosphates such as SHP-1 and SHP-2 (Lorenz et al, Immunol Rev. 2009 Mar; 228(1): 342-359). Some studies reported both ITIM and ITSM motifs contributed to inhibitory signaling of PD1 (Boussiotis et al, Cancer J. 2014 Jul-Aug; 20(4): 265-271, Peled et al, Proc Natl Acad Sci USA. 2018 Jan 16;115(3):E468-E477). Whereas in other studies, ITSM motif was shown to be primarily responsible for the inhibitory effect of PD1 while ITIM motif was had only limited effect (Chemnitz et al, J Immunol. 2004 Jul 15;173(2):945-54, Yokosuka et al, J Exp Med. 2012 Jun 4;209(6):1201-17).

According to the invention, the ITSM and the ITIM may be derived or obtained from the same protein (e.g. PD1) or may be obtained from two different proteins (e.g. the ITIM from PD1 and the ITSM from LY9). Preferably, the ITSM and the ITIM are derived or obtained from the same protein. Preferably, the ITSM and ITIM are separated from each other by 15-25 amino acids in the third part of the chimeric polypeptide according to the invention peptide. In some embodiments, the amino acids separating the ITSM and ITIM are the same amino acids as those in the protein from which the ITSM and ITIM were derived or obtained.

Preferably, in the chimeric polypeptide according to the invention, the first part, comprising a SH2-domain from a protein which binds a phosphorylated immunoreceptor tyrosine-based activation motif, consists of at least 80 adjacent amino acids, and/or at most 800 adjacent amino acids, preferably of at least 100 adjacent amino acids, and/or at most 400 amino acids, for example between 150 and 300 amino acids.

Preferably, in the chimeric polypeptide according to the invention, the third part, comprising an immunoreceptor tyrosine-based switch motif (ITSM), preferably an ITSM and an immunoreceptor tyrosine-based inhibitory motif (ITIM), consists of at least 30 adjacent amino acids, and/or at most 600 adjacent amino acids, preferably of at least 80 adjacent amino acids, and/or at most 200 amino acids, for example between 85 and 190 amino acids.

There is also provided for the cell according to the invention, wherein the small molecule-regulated protein stability domain is selected from the group consisting of a self-excising degron (SED), wherein the SED comprises a repressible protease, a cognate cleavage site, and a degron sequence; and a proteolysis-targeting chimera (Protac) binding domain wherein the Protac comprises a E3 ubiquitin ligase binding group (E3LB), a linker, and a protein binding group that binds to the Protac binding domain in the chimeric polypeptide, and a CRBN polypeptide substrate domain capable of binding to the CRBN protein in response to a drug, thereby promoting ubiquitin pathway-mediated degradation of the chimeric polypeptide. For example, the protein binding group of Protac may be AP1867 which binds to the Protac binding domain in the chimeric polypeptide of the invention, which may, for example be FKBP12^{F36V} (SEQ ID NO 35, Nabet et al, Nat Chem Biol. 2018 May;14(5):431-441). See Zou et al. Current Protocols (2019) V37:1: pp 21 -30; doi.org/10.1002/cbf.3369, for a recent review on PROTAC technology.

As will be understood by the skilled person, the small molecule-regulated protein stability domain (or regulatable destabilization domain) may suitably be any domain that confers chimeric polypeptide stability such that chimeric polypeptide degradation occurs when the small molecule-regulated protein stability domain (or regulatable destabilization domain) in the chimeric polypeptide is modified/targeted to direct the chimeric polypeptide to degradation by the presence of its cognate small molecule.

Preferably, the small molecule-regulated protein stability domain is a CRBN polypeptide substrate domain capable of binding to the CRBN protein in response to a drug, thereby promoting ubiquitin pathway-mediated degradation of the chimeric polypeptide, preferably wherein the CRBN polypeptide substrate domain is a C2H2 zinc finger protein or a fragment thereof that is capable of drug-inducible binding to the CRBN polypeptide.

Preferably the small molecule-regulated protein stability domain is a proteolysis-targeting chimera (Protac) binding domain. The domain can bind to the cognate Protac.

Preferably the small molecule-regulated protein stability domain is a self-excising degron (SED), wherein the SED comprises a repressible protease, a cognate cleavage site, and a degron sequence. Such self-excising degron are well-known to the skilled person (Chung et al, Nat Chem Biol. 2015 Sep;11(9):713-20).

The term "self-excising degron" (SED) as used herein, refers to a polypeptide or protein complex that comprises a repressible protease, a cognate cleavage site, and a degron sequence (or degradation sequence). The self-excising degron is part of the chimeric polynucleotide of the invention such that the protease is capable of cleaving the chimeric polypeptide of the invention to separate the degron sequence from other parts of the chimeric polypeptide.

In some embodiments, the cleaving of the chimeric polypeptide of the invention by the protease separates at least the first part of the chimeric polypeptide and comprising an SH2-domain from a protein which binds a phosphorylated immunoreceptor tyrosine-based activation motif (ITAM) from the degron sequence (that is part of the second part in the chimeric polypeptide).

In some embodiments, the cleaving of the chimeric polypeptide of the invention by the protease separates at least the first part of the chimeric polypeptide and comprising an SH2-domain from a protein which binds a phosphorylated immunoreceptor tyrosine-based activation motif (ITAM), and the third part of the chimeric polypeptide of the invention and comprising an immunoreceptor tyrosine-based switch motif (ITSM), preferably an ITSM and an immunoreceptor tyrosine-based inhibitory motif (ITIM) from the degron sequence (that is part of the second part in the chimeric polypeptide).

The protease itself may or may not be removed from the part of the chimeric polypeptide that is separated from the degron sequence.

The term "degron" as used herein, refers to a protein or a part thereof that is important in regulation of protein degradation rates. Various degrons known in the art, including but not limited to short amino acid sequences, structural motifs, and exposed amino acids, can be used in various embodiments of the present disclosure. Degrons identified from a variety of organisms can be used.

The term "degron sequence" or "degradation sequence" as used herein in the context of the SED, refers to a sequence that promotes degradation of an attached protein through either the proteasome or autophagy- lysosome pathways. Many different degradation sequences/signals (e.g., of the ubiquitin- proteasome system) are known in the art, any of which may be used as provided herein. For a discussion of degradation sequences and their function in protein degradation, see, e.g., Kanemaki et al, Pflugers Arch. 2013 Mar;465(3):419-25, Erales et al, Biochim Biophys Acta. 2014 Jan;1843(1):216-21.

The term "cognate cleavage site" as used herein, refers to a specific sequence or sequence motif recognized by and cleaved by the repressible protease in the SED. A cleavage site for a protease includes the specific amino acid sequence or motif recognized by the protease during proteolytic cleavage and typically includes the flanking one to six amino acids on either side of the scissile bond, which bind to the active site of the protease and are used for recognition as a substrate.

The term "repressible protease" as used herein, refers to a protease that can be inactivated by the presence of a specific agent or compound, e.g. a small compound (e.g., that binds to the protease) (Leuw et al, GMS Infect Dis. 2017; 5: Doc08, Lv et al, HIV AIDS (Auckl). 2015; 7: 95-104). In some embodiments, a repressible protease is active (cleaves a cognate cleavage site) in the absence of the specific agent and is inactive (does not cleave a cognate cleavage site) in the presence of the specific agent. In some embodiments, the specific agent is a protease inhibitor. In some embodiments, the protease inhibitor specifically inhibits a given repressible protease of the present disclosure.

In an embodiment, the SED is small molecule-assisted shutoff (SMASh) technology, i.e. a Small Molecule-Assisted Shutoff tag (SMASh tag) (Chung et al, Nat Chem Biol. 2015 Sep;11(9):713-20). It includes a degradation signal (i.e., degron sequence) and a protease cleavage site that cleaves the degron from other parts of the chimeric polypeptide of the invention. However, in the presence of a protease inhibitor, this cleavage can be blocked and the degron induces rapid degradation of the chimeric polypeptide. In some embodiments, the SMASh can comprise a hepatitis C virus-derived NS3/4A protease (inhibited, for example, by asunaprevir or grazoprevir) and flanked by a degron domain inducing proteasomal degradation. HCV NS3/4A protease inhibitors include asunaprevir, grazoprevir, glecaprevir, voxilaprevir, paritaprevir, simeprevir, boceprevir and telaprevir (Majumdar et al, Aliment Pharmacol Ther. 2016 Jun;43(12):1276-92, Ahmed et al, World J Hepatol. 2018 Oct 27; 10(10): 670-684).

In another embodiment the small molecule-regulated protein stability domain (or regulatable destabilization domain) comprises a proteolysis-targeting chimera (Protac) binding domain wherein the Protac comprises a E3 ubiquitin ligase binding group (E3LB), a linker, and a protein binding group that binds to the Protac binding domain in the chimeric polypeptide.

One of the cell's major degradation pathways is the ubiquitin-proteasome system. In this system, a protein is marked for degradation by the proteasome by ubiquitinating the protein. The ubiquitination of the protein is accomplished by an E3 ubiquitin ligase that binds to a protein and adds ubiquitin molecules to the protein. The E3 ubiquitin ligase is part of a pathway that includes E1 ubiquitin-activating enzyme and E2 ubiquitin-conjugating enzyme, which make ubiquitin available to the E3 ubiquitin ligase to add to the protein.

To make use of this degradation pathway, Protacs have been developed. Protacs bring together an E3 ubiquitin ligase with a protein that is to be targeted for degradation. To facilitate a protein for degradation by the proteasome, the Protac comprised of a group that binds to an E3 ubiquitin ligase and a group that binds to the protein one wishes to degrade (i.e. to the Protac binding domain in the polypeptide). These groups are typically connected with a linker. This molecular construct can bring the E3 ubiquitin ligase in proximity with the targeted protein so that it is ubiquitinated and marked for degradation.

As used herein, the term "Protac" refers to proteolysis-targeting chimera molecules having generally three components, an E3 ubiquitin ligase binding group (E3LB), a linker, and a protein binding group. Protacs and Protac binding domains for use in the chimeric polypeptide according to the invention are well-known to the skilled person (An et al, EBioMedicine. 2018 Oct; 36: 553-562).

As used herein, the term "linker", means a chemical moiety comprising a chain of atoms that covalently attaches a component of a Protac to another component of the Protac. In various embodiments, a linker is typically 8 - 20 atoms in length (see also Cyrus et al, Mol Biosyst. 2011 Feb; 7(2): 10.1039/c0mb00074d, Nabet et al, Nat Chem Biol. 2018 May;14(5):431-441). Commercial linker can be, for example, those provided by Medchemexpress (www.medchemexpress.com).

The protein binding group is a group which binds to a target protein, here to the Protac binding domain present in the chimeric polypeptide. The protein binding group may, for example, be any moiety which binds to a protein specifically (binds to a target protein) and includes the following non-limiting examples of small molecule target protein moieties: Hsp90 inhibitors, kinase inhibitors, MDM2 inhibitors, compounds targeting Human BET Bromodomain-containing proteins, HDAC inhibitors, human lysine methyltransferase inhibitors, angiogenesis inhibitors, immunosuppressive compounds, and compounds targeting the aryl hydrocarbon receptor (AHR), among numerous others (see US2014/0356322 and US2016/0045607).

In some embodiments, the protein binding group in the Protac is AP1867 (www.medchemexpress.com/AP1867.html) and the Protac binding domain in the chimeric polypeptide of the invention is FKBP12^{F36V} (Nabet et al, Nat Chem Biol. 2018 May; 14(5):431-441).
In another embodiment the small molecule-regulated protein stability domain (or regulatable destabilization domain) is a CRBN polypeptide substrate domain capable of binding to the CRBN protein in response to a drug, thereby promoting ubiquitin pathway-mediated degradation of the chimeric polypeptide, preferably wherein the CRBN polypeptide substrate domain is a C2H2 zinc finger protein or a fragment thereof that is capable of drug-inducible binding to the CRBN polypeptide (herein also refered to as "zinc finger degron"). Also provided is for the cell according to the invention comprising a chimeric protein according to the invention comprising such small molecule-regulated protein stability domain. In such embodiment the second part of the chimeric protein according to the invention comprises a small molecule-regulated protein stability domain that is capable of interacting and binding with the CRBN protein in the presence of a drug. For example, various IMiD's, including those described herein, have been shown to bind to the CRBN protein, thereby promoting interaction between the CRBN protein and its target (see also Buhimschi et al. Biochemistry 2019, 58, 861-864; DOI: 10.1021/acs.biochem.8b01307 for a recent review of the technology), ubiquitination and subsequent degradation of the target protein.

CRBN (Cereblon) is a 442 amino acid protein that forms an E3 ubiquitin ligase complex with damaged DNA binding protein 1 (DDB1), Cullin-4A (CUL4A) and regulator of cullins 1 (ROC1 ; Angers et al. Nature 443 : 590-593). This complex ubiquitinates a number of other proteins. It was shown that thalidomide, lenalidomide, pomalidomide, CC-122 (avadomide), CC-220 (iberdomide) and CC-885 each bind to CRBN (see, for example Lopez - Girona et al. Leukemia 26: 2326-2335).

In a preferred embodiment the CRBN polypeptide substrate domain is a C2H2 zinc finger protein or a fragment thereof that is capable of drug-inducible binding to the CRBN polypeptide. Also provided is for the cell according to the invention comprising a chimeric protein according to the invention comprising such small molecule-regulated protein stability domain. The Cys2His2-like fold group (C2H2) is a well characterized class of zinc fingers that is extremely common in mammalian transcription factors. These domains adopt a simple ββα fold, forming two short β-strands connected by a turn (zinc knuckle; beta turn) followed by a short helix and have the amino acid sequence motif (Pabo et al. Annual Review of Biochemistry (2001). 70: 313- 40).

X2-Cys-X2,4-Cys-X12-His-X3,4,5-His

In another preferred embodiment, the chimeric protein comprises a CRBN polypeptide substrate domain that comprises one or more zinc fingers. Also provided is for the cell according to the invention comprising a chimeric protein according to the invention comprising such small molecule-regulated protein stability domain.

Although not in particular limited to a particular CRBN polypeptide substrate domain, in particular a C2H2 zinc finger protein, or a fragment thereof (zinc finger domain) that is capable of binding to the CRBN protein in response to a drug, thereby promoting ubiquitin pathway-mediated degradation of the chimeric polypeptide, in a preferred embodiment the CRBN polypeptide substrate domain is selected from the group consisting of IKZF1, IKZF3, ZFN654, ZNF787, ZNF653, ZFP91, ZNF276, ZNF827, or a fragment thereof that is capable of small molecule-inducible binding to the CRBN polypeptide, preferably wherein said fragment is selected from the group consisting of IKZF1 ZF2-3 (SEQ ID NO:41), IKZF3 ZF2-3 (SEQ ID NO:42), ZFP91 ZF4-5 (SEQ ID NO: 43), ZNF276 ZF4-5 (SEQ ID NO:44), ZNF653 ZF4-5 (SEQ ID NO: 45), and ZNF692 ZF4-5 (SEQ ID NO:46) (See examples). Also provided is for the cell according to the invention comprising a chimeric protein according to the invention comprising such small molecule-regulated protein stability domain.

In another preferred embodiment, the CRBN polypeptide substrate domain comprises a hybrid fusion polypeptide wherein the hybrid fusion polypeptide comprises at least a first fragment of a first C2H2 zinc finger protein and a second fragment from a second C2H2 zinc finger protein, wherein the combination of said first fragment and said second fragment in the hybrid fusion polypeptide is capable of drug-inducible binding to the CRBN polypeptide. For example, in some embodiments, a beta-turn (formed by two short beta-strands) from a first C2H2 zinc finger protein may be fused to an alpha-helix of a second C2H2 zinc finger protein. Also provided is for the cell according to the invention comprising a chimeric protein according to the invention comprising such small molecule-regulated protein stability domain.

Although the invention is not in particular limited to specific hybrid fusion polypeptides that may form or may be comprised in the CRBN polypeptide substrate domain, in a preferred embodiment, the hybrid fusion polypeptide comprises a first fragment selected from the beta-turn of ZFP91 ZF4 (LQCEICGFTCR; SEQ ID NO: 52), ZFN653 ZF4 (LQCEICGYQCR; SEQ ID NO 53), ZNF276 ZF4 (LQCEVCGFQCR: SEQ ID NO: 54), ZNF827 ZF1 (FQCPICGLVIK; SEQ ID NO:55) and a second fragment selected from the alpha-helix of IKZF1 ZF2 (QKGNLLRHIKLH; SEQ ID NO: 56), and in any possible combination. Preferably the hybrid fusion polypeptide comprises the beta-turn of ZFP91 ZF4 and the alpha-helix of IKZF1 ZF2, preferably wherein the hybrid fusion polypeptide comprises one selected from SEQ ID NO 47 - 51. Also provided is for the cell according to the invention comprising a chimeric protein according to the invention comprising such small molecule-regulated protein stability domain.

According to another embodiment, the CRBN polypeptide substrate binding domain used in the method of the invention comprises or further comprises IKZF1 ZF3 (FKCHLCNYACRRRDALTGHLRTH; SEQ ID NO: 57), preferably wherein the CRBN polypeptide substrate binding domain comprises the beta-turn of ZFP91 ZF4, the alpha-helix of IKZF1 ZF2, and IKZF1 ZF3. Preferably the IKZF1 ZF3 is at the C-terminus of the second part of the chimeric protein according to the invention. Also provided is for the cell according to the invention comprising a chimeric protein according to the invention comprising such small molecule-regulated protein stability domain.

As will be understood by the skilled person, in some embodiments, one or more CRBN polypeptide substrate domain(s) capable of binding CRBN in response to drug, thereby promoting ubiquitin pathway-mediated degradation of the chimeric protein of the invention are comprised in the chimeric protein of the invention. The zinc finger degron polypeptide domains (CRBN polypeptide substrate domain(s) capable of binding CRBN in response to drug, thereby promoting ubiquitin pathway-mediated degradation of the chimeric protein) can be included as single degron polypeptide domains or as multiple degron polypeptide domains, optionally where multiple degron polypeptide domains are joined in a series or an array, optionally using polypeptide linkers, such as those known in the art.

As will also be understood by the skilled person, within a CRBN polypeptide substrate binding domain used in the method of the invention, different parts (e.g. a beta-turn and an alpha-helix) may be directly adjacent to each other, or may, be linked using polypeptide linkers (including small stretches of amino acids), such as those known in the art.

Drugs (e.g. small molecules) suitable for regulation of degradation of the chimeric proteins according to the invention comprising one or more of such C2H2 zinc finger proteins, fragments or domains include the so-called immunomodulatory imide drugs (IMiDs), including but not limited to thalidomide, lenalidomide, pomalidomide, CC-122 (avadomide), CC-220 (iberdomide) and CC-885 (see for example, Matyskiela et al. J. Med. Chem. 2018, 61, 2, 535-542; 2017; doi.org/10.1021/acs.jmedchem.6b01921 and Gao et al. Biomarker Research (2020) 8:2; doi.org/10.1186/s40364-020-0182-y). The skilled person understands how to select a suitable drug, e.g. a suitable IMiD for use in the method according to the invention.

Therefore, there is provided for the cell according to any one of the previous claims wherein the drug that allows the CRBN polypeptide substrate domain to bind to the CRBN protein, thereby promoting ubiquitin pathway-mediated degradation of the chimeric polypeptide, is an IMiD, preferably selected from the group consisting of thalidomide, lenalidomide, pomalidomide, CC-122 (avadomide), CC-220 (iberdomide) and CC-885.

Therefore, there is provided for a cell according to any one of the previous claims further comprising a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, preferably wherein said drug is bound to the CRBN polypeptide substrate domain.

As will be understood by the skilled person, the chimeric polypeptide of the invention can be present in different forms in the cell of the invention. For example, in case the small molecule-regulated protein stability domain (or regulatable destabilization domain) is a SED, depending on the presence of the cognate small compound/protease inhibitor, the chimeric polypeptide can be present in the cell with or without the degron sequence.

Also provided is for the cell according to the invention wherein the ITAM is an ITAM comprised in a T cell receptor (TCR) complex and/or a chimeric antigen receptor (CAR) and/or an NKR complex, preferably an ITAM comprised in a CD3 zeta chain, a CD3 epsilon chain, a CD3 delta chain, CD3 gamma chain, gamma chain of the immunoglobulin receptor FcεRI and DAP12.

As discussed, the SH2 domain may be from a protein which binds a phosphorylated immunoreceptor tyrosine-based activation motif (ITAM).

ITAMs are found in the intracellular domains of cell signaling molecules such as the CD3 zeta (ζ), CD3 epsilon (ε), the CD3 gamma (γ) and the CD3 delta (δ) chains of the T cell receptor complex and certain Fc receptors (Love et al, Cold Spring Harb Perspect Biol. 2010 Jun; 2(6): a002485).

In NK cells, certain activating NK cell receptors (NKR) form complexes with ITAM bearing signaling molecules such as CD3 zeta chain, gamma (γ) chain of the immunoglobulin receptor FcεRI and DAP12. For example, NK cell receptors (NKR) NKp46 and NKp30 associate with the gamma (γ) chain of the immunoglobulin receptor FcεRI and the CD3ζ chain while NKp44 associates with the signaling adaptor DAP12 (Barrow et al, Front Immunol. 2019; 10: 909). Therefore, in one embodiment of the current invention, the cell according to the invention is a NK cell.

ITAM bearing domains are also used in chimeric antigen receptor (CAR) designs. CD3 zeta chain contains three ITAMs while CD3 epsilon chain, gamma (γ) chain of the immunoglobulin receptor FcεRI and DAP12 signaling domains contain one ITAM and they are used in various CAR designs (Ren-Heidenreich et al, Cancer Immunol Immunother. 2002 Oct;51(8):417-23, Nolan et al, Clin Cancer Res. 1999 Dec;5(12):3928-41, Töpfer et al, J Immunol. 2015 Apr 1;194(7):3201-12).

Two tyrosine residues within ITAM are phosphorylated by Src-kinase family members such as Lck. Phosphorylated ITAMs act as a docking platform for SH2 domains of Zap70 and Syk (Long et al, Annu Rev Immunol. 2013; 31: 10.1146/annurev-immunol-020711-075005).

The half-ITAM signature can be easily recognized as a tyrosine separated from a leucine or isoleucine by any two other amino acids, giving the signature YxxL/I. Two of these signatures are separated by between 6 and 8 amino acid to constitute ITAM consensus sequence of YxxL/Ix(6-8)YxxL/I. In a preferred embodiment the ITAM-containing domain may be or comprise a CD3 zeta chain domain. In another preferred embodiment the ITAM-containing domain may be or comprise a CD3 epsilon chain domain. Yet, in another preferred embodiment the ITAM-containing domain may be or comprise a gamma (γ) chain of the immunoglobulin receptor FcεRI. Yet, in another preferred embodiment the ITAM-containing domain may be or comprise a DAP12 domain.

Also provided is for the cell according to the invention wherein the cell further comprises a T cell receptor (T cell receptor complex) and/or a chimeric antigen receptor (CAR) or an NK cell receptor (NK cell receptor complex), preferably wherein the cell is a T cell, CAR T cell, NK cell and/or CAR NK cell.

Preferably the cell is a T cell expressing a TCR complex and/or CAR complex. Preferably the TCR complex or the CAR complex comprise a CD3 zeta chain domain comprising an ITAM, or any other ITAM bearing domain disclosed herein.

Preferably the cell is a NK cell expressing an NKR complex and/or CAR complex. Preferably the NKR complex or the CAR complex comprise a CD3 zeta chain domain comprising an ITAM, or any other ITAM bearing domain disclosed herein.

The skilled person is well aware of T cells and/or CAR T cells. T cells or T lymphocytes play a central role in cell-mediated immunity. They can be distinguished from other lymphocytes, such as B cells and natural killer cells (NK cells), by the presence of a T cell receptor (TCR) on the cell surface.

There are various types of T cells, including but not limited T helper cells (TH cells), cytolytic T cells and regulatory T cells.TH cells express CD4 on their surface and become activated when they are presented with peptide antigens on the surface of antigen presenting cells (APCs). These cells can differentiate into one of several subtypes which secrete different cytokines to facilitate different types of immune responses.

Cytolytic T cells (TC cells, or CTLs) destroy virally infected cells and tumor cells, and are also implicated in transplant rejection. CTLs express CD8 at their surface. These cells recognize their targets by binding to antigen associated with MHC class I, which is present on the surface of all nucleated cells.

Regulatory T cells (Tregs) inhibit immune reactions, for instance by secretion of molecules such as IL-10, and these cells are characterized by expression of the transcription factor FOXP3.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory cells may be either CD4+ or CD8+.

Preferably the T cell is a CD4 positive T cell. Preferably the T cell is a CD8 positive T cell. A CAR T cell is a T cell expressing a CAR complex.

Natural Killer Cells (or NK cells) are a type cytolytic cells that are part of the innate immune system. NK cells provide responses to innate signals from virally infected cells in a peptide MHC independent manner.

NK cells are defined as large granular lymphocytes and constitute the third kind of cells differentiated from the common lymphoid progenitor generating B and T lymphocytes. NK cells are known to differentiate and mature in e.g. bone marrow, lymph node, spleen, tonsils and thymus.

The cells of the invention may be any type of cell, in particular a T cell, a CAR T cell, an NK cell or a CAR NK cell.

The T cells (including CAR T cells) or NK cells (including CAR NK cells) expressing the molecules of the invention may either be created ex vivo e.g. from a patient's own peripheral blood, or from donor peripheral blood.

Also provided is for the cell according to the invention wherein the SH2-domain is from a protein selected from the group consisting of Zap70, Syk, and Lck.

It was found that in particular chimeric polypeptides according to the invention comprising a first part comprising SH2-domains from Zap70, Syk, and Lck are suitable according to the current invention.

ZAP70 is a protein normally expressed near the cell membrane of T cells and natural killer cells. It plays a critical role in T cell signaling. Its molecular weight is 70 kDa, and it is composed of 2 N-terminal SH2 domains and a C-terminal kinase domain. It is a member of the protein-tyrosine kinase family. Human ZAP70 protein has the UniProtKB accession number P43403. This sequence is 619 amino acids in length and is shown as SEQ ID NO 37.

Syk is expressed thymocytes, intraepithelial gammadelta T cells, naive alphabeta T cell and B cells (Latour et al, Mol Cell Biol. 1997 Aug; 17(8): 4434-4441). Syk is highly homologous to ZAP70 and has the same domain structure of 2 N-terminal SH2 domains and a C-terminal kinase domain. In B cells, deficiency of Syk can be reconstituted by Zap70 (Kong et al, Immunity. 1995 May;2(5):485-92). Similarly, ZAP70 deficiency can be reconstituted by Syk in T cells (Williams et al, Mol Cell Biol. 1998 Mar;18(3):1388-99). Human Syk protein has the UniProtKB accession number P43405. This sequence is 635 amino acids in length and is shown as SEQ ID NO 38.

Lck (also known as p56-LCK) is expressed in lymphocytes. Lck plays a critical role in TCR signal transduction pathway. In T cells, it constitutively associates with cytoplasmic domains of CD4 and CD8 co-receptors. Activation of TCR by peptide-MHC complex brings Lck to close proximity of TCR complex and thereby ITAM residues in CD3 subunits are phosphorylated by Lck. Phosphorylated ITAMs function as docking sites for SH2 domains of Zap70 (Simeoni, Oncotarget. 2017 Nov 28; 8(61): 102761-102762). Domain structure of Lck is SH4-Unique domain (UD)-SH3-SH2-kinase domain. SH2 domain is required for interaction with phosphorylated ITAMs while SH4 is required for membrane association (Ngoenkam et al, Immunology. 2018 Jan; 153(1): 42-50). Human Lck protein has the UniProtKB accession number P06239. This sequence is 509 amino acids in length and is shown as SEQ ID NO 39.

Preferably Zap70, Syk, and Lck are human Zap70, Syk, and Lck.

Also provided is for a cell according to the invention wherein the chimeric polypeptide comprises more than one SH2-domain from a protein which binds a phosphorylated immunoreceptor tyrosine-based activation motif.

Although according to the invention the presence of one SH2 domain in the chimeric polypeptide is suitable, in some embodiments more than one SH2 domains that can bind to a phosphorylated ITAM, preferably a phosphorylated ITAM that is present in a T cell receptor (complex) or CAR (complex) or NKR (complex) may be comprised in the first part of the chimeric polypeptide (As explained herein elsewhere, the first part of the chimeric polypeptide refers to a part comprising a SH2-domain from a protein which binds a phosphorylated immunoreceptor tyrosine-based activation motif (ITAM). As disclosed herein, this first part may be present anywhere in the chimeric polypeptide according to the invention; "first part" does not necessarily imply "at the N-terminal". The same applies to the second and third part of the chimeric polypeptide as defined and disclosed herein, and as discussed herein). The more than one SH2 domain may be from the same (natural) protein or may be from two or more different proteins.

As will be understood by the skilled person it is contemplated that next to, for example, the SH2 domains, other domains may be comprised in the first part of the chimeric polypeptide, for example such domains as are present in the chimeric polypeptides tested in the Examples.

Also provided is for a cell according to the invention wherein the ITIM and/or ITSM is from an inhibitory receptor protein, preferably an inhibitory immune receptor protein, preferably from a protein selected from the group consisting of PD1, BTLA, SIRPalpha, SIGLEC5, SIGLEC9, SIGLEC11, PECAM1 or LY9. Preferably the inhibitory receptor protein, the inhibitory immune receptor protein, or protein selected from the group consisting of PD1, BTLA, SIRPalpha, SIGLEC5, SIGLEC9, SIGLEC11, PECAM1 or LY9 is from human.

PD1 (also known as PD-1) is encoded by PDCD1 gene. PD1 is a type I transmembrane protein. Interaction with its ligands PD-L1/PD-L2 causes downregulation of effector functions of cytotoxic T cells. Human PD1 has UniProtKB accession number Q15116. This sequence is 288 amino acids in length. Cytoplasmic domain of PD1 contains an ITIM and an ITSM motif. Phosphorylated ITSM in cytoplasmic domain of PD1 recruits SHP-2 phosphatase, which dephosphorylates key signaling molecules in TCR signaling pathway such as ZAP70, PKCtheta and CD3 zeta (CD247) and cause downregulation of TCR signaling (Bardhan et al, Front Immunol. 2016; 7: 550) and CD28 mediated co-stimulation (Hui et al, Science. 2017 Mar 31;355(6332):1428-1433). For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 17 (representing the cytoplasmic domain of PD1).

B- and T-lymphocyte attenuator (BTLA) is mainly expressed in T cells, B cells and mature lymphocytes (Yue et al, Front Immunol. 2019; 10: 617). It is an immune regulator receptor playing a critical role in immune tolerance. Similar to PD1, BTLA is a type I transmembrane glycoprotein. Engagement of BTLA receptor induce SHP-1/SHP-2 recruitment and downregulation of IL-2 secretion in T cells (Watanabe et al, Nat Immunol. 2003 Jul;4(7):670-9). Human BTLA protein has the UniProtKB accession number Q7Z6A9. This sequence is 289 amino acids in length. Cytoplasmic domain of BTLA contains an ITIM and an ITSM motif.

For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 15 (representing the cytoplasmic domain of BTLA).

SIRPA (also known as SIRPalpha, SIRPα, BIT, MFR, MYD1, PTPNS1, SHPS1, SIRP) is expressed in myeloid cells. Upon engagement with is ligand CD47, it negatively regulates phagocytosis, mast cell activation and dendritic cell activation (Timms et al, Curr Biol. 1999 Aug 26;9(16):927-30, Latour et al, J Immunol. 2001 Sep 1;167(5):2547-54, Matlung et al, Immunol Rev. 2017 Mar;276(1):145-164. doi: 10.1111/imr.12527). In macrophages, SIRPA primarily associate with SHP-1 (Veillette et al, J Biol Chem. 1998 Aug 28;273(35):22719-28). SIRPA is a type I transmembrane protein. Human SIRPA protein has the UniProtKB accession number P78324. This sequence is 504 amino acids in length. Cytoplasmic domain of SIRPA contains two ITIMs and an ITSM motif.

For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 19 (representing the cytoplasmic domain of SIRPa).

PECAM1 (also known as PECAM-1, CD31) is expressed in T cell, B cells, platelets, monocytes, macrophages and neutrophils (Newton-Nash et al, J Immunol. 1999 Jul 15;163(2):682-8). PECAM1 inhibits T cell and B cell signaling via recruitment of SHIP1, SHP-1 and SHP-2 (Marelli-Berg et al, J Cell Sci. 2013 Jun 1;126(Pt 11):2343-52). In macrophages, ligand binding to PECAM1 leads to recruitment of SHP-1 and SHP2, downregulation of TNF-alpha, IL-6, and IFN-beta production and TLR4 signaling (Rui et al, J Immunol. 2007 Dec 1;179(11):7344-51). PECAM1 negatively regulates platelet signaling pathway (Jones et al, FEBS Lett. 2009 Nov 19;583(22):3618-24). PECAM1 is a type I transmembrane protein. Human PECAM1 protein has the UniProtKB accession number P16284. This sequence is 738 amino acids in length. Cytoplasmic domain of PECAM1 contains an ITIM and an ITSM motif. For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 18 (representing the cytoplasmic domain of PECAM1).

Sialic acid-binding immunoglobulin-type lectins (Siglecs) are a group of immune regulatory receptors, mainly expressed on the cells of the hematopoietic system (Bornhöfft et al, Dev Comp Immunol. 2018 Sep;86:219-231). SIGLEC5 (also known as CD33L2, OBBP2) is expressed in monocytes, neutrophils and B cells, SIGLEC9 is expressed in neutrophils, monocytes, dendritic cells and NK cells while SIGLEC11 is expressed in macrophages (Macauley et al, Nat Rev Immunol. 2014 Oct; 14(10): 653-666). Most Siglecs have inhibitory ITIM/ITSM motifs that recruit SHP1 and SHP2 and act as negative regulators of immune system (Crocker et al, Nat Rev Immunol. 2007 Apr;7(4):255-66, Avril et al, J Biol Chem. 2005 May 20;280(20):19843-51, Haas et al, Cancer Immunol Res. 2019 May;7(5):707-718, Angata et al, J Biol Chem. 2002 Jul 5;277(27):24466-74). SIGLEC5, SIGLEC9 and SIGLEC11 are type I transmembrane proteins. They contain ITIM and ITSM motifs in their cytoplasmic domains. Human SIGLEC5 protein has the UniProtKB accession number O15389. This sequence is 551 amino acids in length. Human SIGLEC9 protein has the UniProtKB accession number Q9Y336. This sequence is 463 amino acids in length. Human SIGLEC11 protein has the UniProtKB accession number Q96RL6. This sequence is 698 amino acids in length. For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 21, SEQ ID NO 22, or SEQ ID NO 20 (representing the cytoplasmic domains of SIGLEC 5, 9, and 11, respectively).

T-lymphocyte surface antigen Ly-9 (also known as LY9, SLAMF3, CD229) is expressed in thymocytes and in mature T and B lymphocytes (de la Fuente et al, Blood. 2001 Jun 1;97(11):3513-20). It has been reported to interact with SHIP-1 and SHP-2 (Puñet-Ortiz et al, Front Immunol. 2018 Nov 16;9:2661) and contribute to peripheral cell tolerance by functioning as a negative regulator of immune response (de Salort et al, Front Immunol. 2013; 4: 225). LY9 is a type I transmembrane protein. Human LY9 protein has the UniProtKB accession number Q9HBG7. This sequence is 655 amino acids in length. Cytoplasmic domain of LY9 contains two ITSM motifs. For example, a suitable third part comprising an immunoreceptor tyrosine-based switch motif (ITSM), preferably an ITSM and an immunoreceptor tyrosine-based inhibitory motif (ITIM) that could be used in the chimeric polypeptide according to the invention is characterized by SEQ ID NO 16 (representing the cytoplasmic tail of LY9).

In some embodiments the ITSM and ITIM are obtained or derived from the same inhibitory protein, in other embodiments the ITSM and ITIM are each derived from a different inhibitory protein.

Also provided is for a cell according to the invention wherein the chimeric polypeptide comprises an ITSM and an ITIM.

It was surprisingly found that the presence of both an ITSM and an ITIM in the third part comprised in the chimeric polypeptide of the invention is in particular advantageous (see examples).

Also provided is for a cell according to the invention wherein, preferably the first part is located at the N-terminus of the chimeric polypeptide, and wherein the second part is located at the C-terminus of the chimeric polypeptide, and preferably wherein the third part is located between the first part and the second part.

Also disclosed herein elsewhere, the first part and the third part may be directly adjacent to each other or may be separated by additional stretches of amino acids. As disclosed herein elsewhere, the second part and the third part may be directly adjacent to each other or may be separated by additional stretches of amino acids. Although not limited by any particular order it was found that this particular order of the first, second and third part may be advantageous.

Also disclosed is a cell according to the invention further comprising a protease inhibitor bound to the repressible protease or further comprising a Protac bound to the proteolysis-targeting chimera (Protac) binding domain, or further comprising a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, as disclosed and discussed herein elsewhere. In a preferred embodiment the protease inhibitor is a protease inhibitor as disclosed herein. In a preferred embodiment the Protac is a Protac as disclosed herein. In a preferred embodiment the CRBN polypeptide substrate domain capable of binding to the CRBN protein in response to a drug, thereby promoting ubiquitin pathway-mediated degradation of the chimeric polypeptide is one as disclosed herein, preferably wherein the drug is an IMiD, preferably selected from the group consisting of thalidomide, lenalidomide, pomalidomide, CC-122 (avadomide), CC-220 (iberdomide) and CC-885

Also disclosed is the cell according to the invention wherein
a) the SH2-domain comprises or consists of an amino acid sequence having at least 80% identity to an amino acid sequence according to SEQ ID NO 7-11 or comprises or consists of an amino acid sequence having at least 80% identity to an amino acid sequence according to SEQ ID NO 12 - 14;
b) the first part of the chimeric polypeptide comprises or consists of an amino acid sequence having at least 80% identity to an amino acid sequence according to SEQ ID NO 7 - 11 or comprises or consists of an amino acid sequence having at least 80% identity to an amino acid sequence according to SEQ ID NO 12 - 14 ;
c) the ITAM is YxxL/Ix(6-8)YxxL/I;
d) the SH2-domain is a SH2-domain that binds to a phosphorylated ITAM comprised in SEQ ID NO 1 - 6, or that binds to an amino acid sequence having 80% or more identity to an amino acid sequence according to SEQ ID NO 1- 6;
e) the ITIM is S/I/V/LxYxxI/V/L, and/or the ITSM is TxYxxV/I;
f) the ITSM and/or ITIM is an ITSM and/or ITIM that is comprised in SEQ ID No 15 - 22;
g) the third part of the chimeric polypeptide comprises or consists of an amino acid sequence having at least 80% identity to an amino acid sequence according to SEQ ID NO 15 - 22;
h) the second part of the chimeric polypeptide comprises or consists of an amino acid second according to SEQ ID NO:35 or 40 or SEQ ID NO: 41 - 57; and/or
i) the chimeric polypeptide comprises an amino acid sequence having 80% or more identity to an amino acid sequence according to SEQ ID NO 23 - 34, SEQ ID NO 36, or SEQ ID NO 58 - 68.

The skilled person will understand that any defined amino acid sequence relevant for the first part of the chimeric polypeptide may be combined with any defined amino acid sequence relevant for the third part of the chimeric polypeptide, and/or the second part of the chimeric polypeptide. In others words, any first part as disclosed herein, any second part as disclosed and any third part as disclosed herein may be combined to form, respectively, the first, second and third part of the chimeric polypeptide according to the invention. In a preferred embodiment, the first part of the chimeric polypeptide comprises an amino acid sequence as defined under a), b), c) and/or d) and the third part of the chimeric polypeptide comprises an amino acid sequence as defined under e), f), or g). As can be witnessed from the disclosure, different first parts of the chimeric proteins may be combined with different third parts of the chimeric polypeptide.

The skilled person will also understand that, in addition to the amino acid sequence in the first, second and third part of the chimeric proteins, as defined herein, the chimeric polypeptide according to the invention may also comprise additional parts. In other words, the chimeric polypeptides as disclosed herein are not limited to only chimeric polypeptides that only consist of the first, second and third part as defined herein. The chimeric polypeptides according to the invention may comprises additional (stretches) of amino acids or additional (functional) parts.

Thus, the SH2-domain, as is present in the first part of the chimeric polypeptide, may in a preferred embodiment comprises or consists of an amino acid sequence having at least 80% identity, preferably at least 81, 83, 87, 90, 91, 92, 93, 94, 95, 96, 97, 98, 99, or 100% identity, to an amino acid sequence according to SEQ ID NO 7-11 or comprises or consists of an amino acid sequence having at least 80% identity, preferably at least 81, 83, 87, 90, 91, 92, 93, 94, 95, 96, 97, 98, 99, or 100% identity to an amino acid sequence according to SEQ ID NO 12 - 14. As will be understood by the skilled person also included are those sequence defined above and wherein 1, 2, 3, 4, 5, 6, 10 amino acids have been deleted, replaced or inserted.

Thus the first part of the chimeric polypeptide may in a preferred embodiment comprises or consists of an amino acid sequence having at least 80% identity, preferably at least 81, 83, 87, 90, 91, 92, 93, 94, 95, 96, 97, 98, 99, or 100% identity, to an amino acid sequence according to SEQ ID NO 7-11 or comprises or consists of an amino acid sequence having at least 80% identity, preferably at least 81, 83, 87, 90, 91, 92, 93, 94, 95, 96, 97, 98, 99, or 100% identity, to an amino acid sequence according to SEQ ID NO 12 - 14. As will be understood by the skilled person also included are those sequence defined above and wherein 1, 2, 3, 4, 5, 6, 10 amino acids have been deleted, replaced or inserted. The first part may also comprise additional (stretches) of amino acids adjacent to the defined amino acids above as long as the first part of the chimeric polypeptide remains functional within the context of the current invention.

Thus the ITAM to which the SH2 domain of the first part of the chimeric polypeptide bind when the ITAM is phosphorylated is YxxL/Ix(6-8)YxxL/I, as explained above.

Thus, the SH2-domain, as is present in the first part of the chimeric polypeptide, may in a preferred embodiment be an SH2-domain that binds to a phosphorylated ITAM comprised in SEQ ID NO 1 - 6, or that binds to an amino acid sequence having at least 80% identity, preferably at least 81, 83, 87, 90, 91, 92, 93, 94, 95, 96, 97, 98, 99, or 100% identity, to an amino acid sequence according to SEQ ID NO 1- 6.

Thus, third part of the chimeric polypeptide, comprising an ITSM, preferably an ITSM and an ITIM may comprise an ITSM and/or ITIM wherein the ITIM is S/I/V/LxYxxI/V/L, and/or the ITSM is TxYxxV/I.

Thus the ITSM and/or ITIM that is comprised in the third part of the chimeric polypeptide may be an ITSM and/or ITIM that is comprised in SEQ ID No 15 - 22.

Thus, the third part of the chimeric polypeptide may comprise or consists of an amino acid sequence having at least 80% identity, preferably at least 81, 83, 87, 90, 91, 92, 93, 94, 95, 96, 97, 98, 99, or 100% identity, to an amino acid sequence according to SEQ ID NO 15 - 22. As will be understood by the skilled person also included are those sequence defined above and wherein 1, 2, 3, 4, 5, 6, 10 amino acids have been deleted, replaced or inserted. The third part may also comprise additional (stretches) of amino acids adjacent to the defined amino acids above as long as the third part of the chimeric polypeptide remains functional within the context of the current invention.

Thus the second part of the chimeric polypeptide according to the invention may comprise or consist of an amino acid sequence according to SEQ NO 35 or SEQ ID NO 40 or SEQ ID NO: 41 - 57. As will be understood by the skilled person also included are those sequence defined above and wherein 1, 2, 3, 4, 5, 6, 10 amino acids have been deleted, replaced or inserted. The third part may also comprise additional (stretches) of amino acids adjacent to the defined amino acids above as long as the third part of the chimeric polypeptide remains functional within the context of the current invention. Also contemplated are those amino acid sequence that have at least 80% identity, preferably at least 81, 83, 87, 90, 91, 92, 93, 94, 95, 96, 97, 98, 99, or 100% identity, to an amino acid sequence according to SEQ ID NO 35 or SEQ ID NO:40 or SEQ ID NO: 41 - 57.

Thus, the chimeric polypeptide comprises or consist of an amino acid sequence having at least 80% identity, preferably at least 81, 83, 87, 90, 91, 92, 93, 94, 95, 96, 97, 98, 99, or 100% identity, to an amino acid sequence according to SEQ ID NO 23 - 34, SEQ ID NO 36, or SEQ ID NO 58 - 68. As will be understood by the skilled person also included are those sequence defined above and wherein 1, 2, 3, 4, 5, 6, 10, 15 amino acids have been deleted, replaced or inserted. The chimeric polypeptide may also comprise additional (stretches) of amino acids adjacent to the defined amino acids above as long as the chimeric polypeptide according to the invention remains functional within the context of the current invention.

According to another aspect of the current invention there is also provided for the chimeric protein as defined herein. The chimeric protein may be present in a cell, or may be present in any other form.

According to another aspect of the current invention there is also provided for a nucleic acid comprising a polynucleotide encoding a chimeric polypeptide according to the invention.

As will be understood the nucleic acid according to the invention may suitably be introduced in a cell, for example a T cell or CAR T cell or NK cell, and be brought to expression in such cell, therewith expressing the chimeric polypeptide according to the invention in such cell. The nucleic acid be introduced in the form of a vector or in the form of a plasmid. In some embodiment, the nucleic acid of the invention is integrated in the genome of a cell, for example a T cell or CAR T cell or NK cell. Such T cell may, for example, be used to introduce a modified T cell receptor and/or CAR.

In some embodiments the nucleic acid of the invention is introduced to a T cell or NK before, after or at the same time as a nucleic acid encoding a (modified) T cell receptor or CAR. The nucleic acids according to the invention and a nucleic acid encoding a (modified) T cell receptor or CAR may be introduced using two separate vector or using one vector comprising both nucleic acids. Similarly, in some embodiments the nucleic acid of the invention is introduced to an NK cell before, after or at the same time as a nucleic acid encoding a (modified) NK cell receptor or CAR. The nucleic acids according to the invention and a nucleic acid encoding a (modified) NK cell receptor or CAR may be introduced using two separate vector or using one vector comprising both nucleic acids.

Therefor also provided is for a vector comprising the nucleic acid according to the invention.

Therefor also provided is for a cell comprising the nucleic acid according to the invention.

Also provided is for a pharmaceutical composition comprising a cell according to the invention or comprising a nucleic acid according to the invention. Preferably the cell is a T cell or CAR T cell or NK cell or CAR NK cell. The T cells may be derived from the patient to be treated or may be allogeneic.

Immunotherapy, which involves the transfer of autologous antigen-specific T cells generated ex vivo, is a promising strategy to treat viral infections and cancer. The T cells used for immunotherapy can be generated either by expansion of antigen-specific T cells or redirection of T cells through genetic engineering. Antigen-specific T cells for use in immunotherapy have been successfully generated through the genetic transfer of T cell receptors or chimeric antigen receptors (CARs).

With the current invention it has now become possible to provide to a patient in need thereof immune cell therapy or immune therapy (e.g. treatment with T cells, CAR T cells, NK cells and CAR NK cells) by providing to the patient T cells, CAR T cells, NK cells or CAR NK cells, and wherein T cell or NK cell function (e.g. T cell or NK cell cytotoxic activity and/or cytokine secretion) of these cells can be regulated as disclosed herein. The skilled person is well aware of the various methods of immune cell therapy, using Chimeric Antigen Receptor Engineered T cells or (genetically modified) T cell receptors for example as reviewed by Rosenberg et al, Science. 2015 Apr 3;348(6230):62-8 and June et al, Science. 2018 Mar 23;359(6382):1361-1365) and unmodified or CAR engineered NK cells as reviewed by Hu et al, Front Immunol. 2019; 10: 1205, Kloess et al, Transfus Med Hemother. 2019 Feb; 46(1): 4-13 and Suen et al, Cancer Invest. 2018;36(8):431-457.

The cells according to the invention, the chimeric polypeptides according to the invention and/or the nucleic acids according to the invention can, as the skilled person will understand based on the current disclosure, be suitably used in such immune cell therapies.

Therefor also provided is for the cell according to the invention for use as a medicament.

Therefor also provided is for the cell according to the invention for use in the treatment of cancer in a subject. Preferably the cancer is a cancer selected from the group consisting of hematological cancers, in particular B cell cancers, melanoma, breast cancer, colorectal cancers, lung cancers, renal cell cancers, and prostate cancers.

Preferably the cell for use as a medicament or for use in the treatment of cancer is a T cell or a CAR T cell or an NK cell or a CAR NK cell.

Also provided is a cell for use as a medicament, preferably for use in the treatment of cancer in a subject according to the invention, wherein the treatment comprises
a) administering to the subject a population of cells according to the invention;
b) optionally, administering to the subject an inhibitor of the repressible protease or a Protac that binds to the proteolysis-targeting chimera (Protac) binding domain or a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally, if step b) is performed, increasing or reducing the concentration of the inhibitor of the repressible protease or the Protac or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885.

As disclosed herein elsewhere, T cell function or NK cell function of the cell according to the invention can be regulated by the expression of the chimeric polypeptide according to the invention. In the absence of the inhibitor of the repressible protease (in case a SED is comprised in the second part of the chimeric polypeptide) or in the absence of a Protac that binds to the Protac binding domain in the second part of the chimeric polypeptide of the invention, or in the absence of the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, T cell function is inhibited by inhibition of TCR or CAR signaling by the chimeric polypeptide according to the invention or NK function is inhibited by inhibition of NKR or CAR signaling by the chimeric polypeptide according to the invention. In the presence of such inhibitor or such Protac or presence of the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, the chimeric polypeptide according to the invention will be degraded, causing the release of the inhibition of T cell or NK cell function (cytokine release/cytotoxicity). Since the regulation of T cell function or NK function is reversible and dose-dependent, T cell function or NK function can be regulated as part of the treatment of the subject.

Also provided is a method of providing a cell according to the invention wherein the method comprises contacting the cell with a nucleic acid according to the invention, preferably wherein the nucleic acid is contacted with the cell ex vivo.

Also provided is a method of controlling expression of a chimeric polypeptide in a cell, wherein the method comprises contacting the cell expressing the chimeric polypeptide according to invention with an inhibitor of the repressible protease or with a Protac that binds to the proteolysis-targeting chimera (Protac) binding domain or with the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, preferably wherein the cell is contacted with the inhibitor of the repressible protease or with the Protac or with the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885 ex vivo or, preferably, in vivo.

Expression of the chimeric polypeptide according to the invention is controlled by modulating the level or concentration of the chimeric polypeptide according to the invention by directing the chimeric polypeptide according to the invention from or towards degradation, as disclosed herein elsewhere.

Also provided is for a method of controlling cytotoxic activity of T cells and/or NK cells and/or of controlling cytokine secretion by T cells and/or NK cells wherein the method comprises
a) expressing a chimeric polypeptide according to the invention in the T cell and/or NK cell;
b) contacting the T cell and/or NK cell with an inhibitor of the repressible protease or with a Protac that binds to the proteolysis-targeting chimera (Protac) binding domain or with a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally, increasing or reducing the concentration of the inhibitor of the repressible protease or the Protac, or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885.

The skilled person is well aware of methods to determine cytotoxic activity of T cells and NK cells and/or cytokine secretion by such T cells, e.g. IFNγ, IL-2, TNFα, IL-10, IL-4 and NK cells, e.g. IFNγ, IL-2, TNFα. The T cells and/or NK cells may be derived from the patient to be treated or may be allogeneic.

Also described herein is a method of the treatment of cancer in a subject, wherein the method comprises
a) providing to the subject cells according to the invention;
b) optionally, administering to the subject an inhibitor of the repressible protease or a Protac that binds to the proteolysis-targeting chimera (Protac) binding domain, or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally, if step b) is performed, increasing or reducing the concentration of the inhibitor of the repressible protease or the Protac, or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885. The T cells and/or NK cells may be derived from the patient to be treated, or may be allogeneic.

Also described is a method of controlling cytotoxic activity of T cells and/or NK cells in a subject and/or of controlling cytokine secretion by T cells and/or NK cells in a subject, wherein the method comprises
a) providing cells according to the invention to the subject;
b) administering to the subject an inhibitor of the repressible protease or a Protac that binds to the proteolysis-targeting chimera (Protac) binding domain, or a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally increasing or reducing the concentration of the inhibitor of the repressible protease or the Protac, or a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885.

The T cells and/or NK cells may be derived from the patient to be treated or may be allogeneic.

It will be understood that all details, embodiments and preferences discussed with respect to one aspect of embodiment of the invention is likewise applicable to any other aspect or embodiment of the invention and that there is therefore not need to detail all such details, embodiments and preferences for all aspect separately.

Having now generally described the invention, the same will be more readily understood through reference to the following examples which is provided by way of illustration and is not intended to be limiting of the present invention. Further aspects and embodiments will be apparent to those skilled in the art.

### Examples

### General introduction

To develop a system that can reversibly control the activity of T cells that express any antigen receptor of interest, we designed a strategy in which an inhibitory signal is brought to the CAR or TCR signaling complex in a titratable manner. A shared property of CAR and TCR antigen receptors is that signaling through these receptors leads to the phosphorylation of immunoreceptor tyrosine-based activation motifs (ITAM). Engagement of antigen receptor by an agonist ligand activates Lck which then phosphorylates ITAMs in CD3 complex. Upon phosphorylation CD3 zeta chain, Zap70 protein is recruited to phosphorylated ITAMs in CD3 zeta chain via its SH2 domains.

We realized that when signaling domains as comprised in inhibitory receptors would be shuttled to activated antigen receptors by fusion to ITAM-interacting SH2 domains, such as the Zap70 SH2 domain or the SH2 domain of other proteins which binds a phosphorylated immunoreceptor tyrosine-based activation motif (ITAM), like Syk and Lck this could result in attenuation of TCR/ CAR signaling.

Upon binding of their natural ligands, a variety of inhibitory receptors, including PD1, BTLA, SIRPalpha, SIGLEC5, SIGLEC9, SIGLEC11, PECAM1 and LY9 have been shown to be able to interfere with immune cell activation.

To provide support for concept of SH2 domain-assisted inhibitory domain delivery we initially decided to focus on the PD1 intracellular domain, as signaling on cytokine production and cytotoxicity is reversible (Barber et al, Nature. 2006 Feb 9;439(7077):682-7), and as the physical proximity of PD1 to TCR microclusters has been shown to be crucial for T cell suppression (Yokosuka et al, J Exp Med. 2012 Jun 4;209(6):1201-17). Based on these observations, we generated a Zap70 2xSH2 domains-PD1 tail fusion protein (from here on abbreviated as Zap70-PD1), with the aim to bring the inhibitory domain of PD1 in close proximity to the TCR complex (Figure 1A-B). Zap70 2xSH2 domains bind to phosphorylated CD3 ζ chain and initiate TCR signal transduction pathway by phosphorylating downstream targets (Wang et al, Cold Spring Harb Perspect Biol. 2010 May;2(5):a002279).

To assess the effect of Zap70-PD1 on T cell activation, primary human T cells were transduced with a high affinity CDK4 neoantigen-specific class I-restricted TCR (Stronen et al, Science. 2016 Jun 10;352(6291):1337-41), together with either the Zap70-PD1 fusion, the Zap70 SH2 domains without inhibitory tail (Zap70 2xSH2), the PD1 intracellular domain (PD1 tail), or a vector control.

Analysis of T cell cytokine production (IFNγ, IL2, TNFα) or T cell degranulation (LAMP-1 cell surface expression) upon incubation with T2 tumor cells loaded with the CDK4 neoantigen demonstrated that expression of the free PD1 tail did not substantially alter T cell functionality. Expression of the Zap70 2xSH2 domains without the PD1 tail resulted in a modest inhibition of T cell functions (Figure 1C-D) that may be explained by competition with endogenous wild type Zap70.

Notably, when the intracellular domain of PD1 was linked to the Zap70 SH2 domains, a highly efficient suppression of both T cell cytokine production and T cell degranulation was observed (reduction in percentage responding cells when T cells are co-cultured with 10 nM peptide loaded T2 tumor cells: IFNγ: 104-fold; IL-2: 110-fold; TNFα: 81-fold; cell surface LAMP1: 40-fold, all relative to vector control, Figure 1C-D).

Inhibition of T cell functionality was both observed in CD8 and CD4 T cells. Furthermore, comparison of the degree of inhibition of T cell activity in gene-modified cell populations with different levels of EGFP reporter expression revealed that the level of Zap70-PD1 expression was correlated with inhibitory activity, suggesting that regulation of Zap70-PD1 protein levels could be used to modulate T cell function.

To achieve pharmacological control over Zap70-PD1 protein levels we subsequently generated fusion proteins with a small molecule-regulated protein stability domain (e.g. a self-excising degron (SED), wherein the SED comprises a repressible protease, a cognate cleavage site, and a degron sequence, or a proteolysis-targeting chimera (Protac) binding domain). The combination of a phosphorylated ITAM binding SH2 domain, an immunoreceptor tyrosine-based switch motif (ITSM), preferably an ITSM and an immunoreceptor tyrosine-based inhibitory motif (ITIM), and a small molecule-regulated protein stability domain is herein also referred to as CRASH-IT (Chemically Regulated and SH2-delivered Inhibitory Tail). A CRASH-IT embodiment containing Small Molecule-Assisted Shutoff tag (SMASh tag, Chung et al, Nat Chem Biol. 2015 Sep;11 (9):713-20) as SED is illustrated in Figure 2A-B. The SMASh tag consists of the HCV NS3/4A protease plus a degron that results in rapid protein degradation by the proteasome. In the absence of HCV protease inhibitor, the HCV protease cleaves the linker between the protein of interest and the degron, thereby preventing protein degradation. In contrast, in the presence of HCV NS3/4A protease inhibitor the entire fusion protein is targeted to proteasome and hence degraded.

To test whether the level of Zap70-PD1 fusion protein can be controlled in this manner, we generated an N-terminal HA-tagged Zap70-PD1-SMASh fusion protein and introduced this into human T cells. Analysis of fusion protein levels by intracellular staining revealed that inhibition of protease activity by the HCV NS3/4A protease inhibitor asunaprevir resulted in a reduction in fusion protein levels in both primary human CD8 and CD4 cells, with half maximal inhibition observed around 0.2 µM (Figure 2C-D).

Importantly, the Zap70-PD1-SMASh fusion protein retained the capacity of the Zap70-PD1 fusion protein to prevent T cell activation, and this inhibition of T cell function could now be reverted by addition of asunaprevir for both primary human CD8 T cells (Figure 2E) and CD4 T cells (Figure 2G).

Specifically, relative to DMSO-treated cells, CD8+ T cell activation by peptide-loaded target cells (10nM) was boosted 8.8x, 11.5x, 6.3x, and 6.6x-fold for IFNγ, IL2, TNFα and cell surface LAMP1 expression, respectively. As a control, effector functions of Zap70-PD1-SMASh fusion protein-negative T cells (vector control) were unaltered by protease inhibitor (Figure 2F and Figure 2H). We note that the effect of asunaprevir on the function of Zap70-PD1-SMASh-modified T cells is profound (Figure 2E) even when its effects on fusion protein levels are modest (Figure 2C), potentially reflecting the signal amplification properties of the TCR signaling pathway that makes it highly sensitive to signal strength.

A safety switch used in adoptive T cell therapies should ideally be titratable and reversible. A dose dependent recovery of T cell functionality was demonstrated by analyzing the effect of increasing asunaprevir concentrations in co-cultures of antigen loaded target cells and CDK4-specific T cells modified with the Zap70-PD1-SMASh switch (Figure 3A-C), demonstrating that T cells can be tuned to reach a desired antigen sensitivity. Next, to understand whether the CRASH-IT platform can act as a reversible regulator of T cells, to switch them from an active to an inactive state and back, asunaprevir treated and control treated T cells were washed and cultured for 72 hours in the absence of drug. Subsequently, T cells were again treated with asunaprevir or left untreated and were then exposed to antigen loaded target cells (experimental scheme in Figure 3A). Notably, Zap70-PD1-SMASh modified T cells only showed substantial activity when exposed to asunaprevir during the time of tumor co-culture, and regardless of whether the cells had previously been exposed to asunaprevir or not. In other words, prior triggering of the CRASH-IT switch does not alter outcome during subsequent use, demonstrating reversibility of the platform (Figure 3D).

Small molecule-induced recovery of T cell function was also demonstrated in co-culture experiments using the Mel526 and NKIRTIL006 melanomas that endogenously express the mutant CDK4 neoantigen, while control MM90904 melanoma cells that express the wild type CDK4 gene were unaffected (data not shown).

To characterize the essential protein domains to achieve reversible T cell inhibition, we compared SMASh tagged versions of Zap70 2xSH2 and Zap70-PD1 and also a SMASh tagged version of Zap70-PD1 that lacked the degron domain (data not shown). As was observed for protein switches that lacked the SMASh domain (Figure 1), the presence of the used PD1 tail was important to achieve tight control of TCR signaling, and restoration of T cell activity upon triggering of the CRASH-IT switch required the presence of the degron that induces proteasomal degradation.

Next, we explored PROTAC mediated activation of CRASH-IT platform by replacing SMASh tag with an FKBP12^{F36V} domain (Figure 4A-B). Presence of heterobifunctional dTAG-13 molecule leads to rapid proteasomal degradation of FKBP12^{F36V} fusion proteins by inducing dimerization with CRBN E3 ligase complex (Nabet et al, Nat Chem Biol. 2018 May;14(5):431-441). Small-molecule titration experiments revealed that activation of CRASH-IT platform using dTAG-13 PROTAC peaks around 0.5 µM whereas substantially higher concentrations of HCV NS3/4A inhibitors were required for similar activation levels (Figure 4C-E). In addition, replacing SMASh tag with FKBP12^{F36V} domain reduced basal T cell activation in tumor co-culture in the absence of small-molecule. The reason of improved stringency could be higher transgene expression level, as FKBP12^{F36V} is 591 bp shorter than SMASh tag. By sorting CDK4 TCR and Zap70-PD1-FKBP12^{F36V} (EGFP) co-transduced cells based on EGFP and CD8 expression and subsequently expanding in dTAG-13 and high IL-2 containing media, we generated T cell pools for use in cytotoxicity assays. Sorted CD8 cells showed increased killing of ⁵¹Cr loaded NKIRTIL006 tumor cells upon dTAG-13 treatment, whereas the activity of control vector-modified T cells was unaltered (Figure 4F-G).

We next tested the flexibility of the CRASH-IT switch system using T cells that were modified with a second generation CAR. When anti-CD19-CD28-CD3ζ chain CAR (Figure 5A) (Brentjens et al, Clin Cancer Res. 2007 Sep 15;13(18 Pt 1):5426-35) modified human T cells were co-cultured with CD19 positive Raji or Daudi (Figure 5B) cytokine production and degranulation of these cells was efficiently suppressed by the Zap70-PD1-SMASh, and these T cell functions were recovered upon addition of asunaprevir (Figure 5C-F).

By the same token, the functional activity of CD8 T cells modified with an NY-ESO-1 shared antigen specific TCR (Linnemann et al, Nat Med. 2013 Nov;19(11):1534-41) was prevented by the CRASH-IT switch and restored by addition of asunaprevir (Figure 6).

Finally, when endogenous TCR complexes in primary human T cells were stimulated by anti-CD3 or anti-CD3/CD28 antibody coated plates, T cell functionality was again suppressed by CRASH-IT and regained by drug addition (data not shown).

The efficiency of asunaprevir-induced protein degradation was higher in CD8 cells than in CD4 cells. Potentially because of this, asunaprevir-induced restoration of T cell function was more profound for CD8+ T cells than for CD4+ T cells. To start exploring the feasibility of creating variant CRASH-IT switch systems with an optimized dynamic range for different immune cell types, we created a modified version of Zap70-PD1-SMASh (Figure 7A) that showed slightly less stringent T cell suppression in the absence of asunaprevir in CD8 cells. The tuned tuZap70-PD1-SMASh switch retained the capacity to efficiently suppress the function of CD4 T cells modified with the CDK4 TCR, but recovery of T cell functions upon asunaprevir addition was remarkably improved (Figure 7B). To test whether this switch system could also be used to control CD4+ T cell recognition via HLA class II, we co-transduced primary human CD4+ T cells with the tuZap70-PD1-SMASh switch and an HLA class II restricted CMV TCR, and co-cultured the resulting cells with peptide loaded CBH 5477 target cells. Antigen sensitivity was reduced by approximately 1000-fold by introduction of the tuZap70-PD1-SMASh switch, and asunaprevir treatment resulted in a near-complete recovery of CD4+ T cell functions (Figure 7C-D) demonstrating how optimized CRASH-IT systems can be created for specific cell types. The tuZap70-PD1-SMASh was obtained by adding an alanine amino acid residue encoding DNA N-terminally after start codon (a valine amino acid residue was also tested and showed similar results).

From a conceptual point of view, CRASH-IT contains functional elements that induce proximity to the antigen receptor, provide the inhibitory signal, and offer the possibility to regulate strength of this inhibitory signal. In the design that we have developed, these functional elements are formed by the Zap70 SH2 domains, the PD1 tail and a small molecule-regulated protein stability domain (SMASh tag or FKBP12^{F36V}), respectively.

Furthermore, the inclusion of different inhibitory domains from ITIM and ITSM containing receptors offer the potential to both vary the level of T cell suppression and to direct such suppression to specific T cell output signals. Figure 8 and Figure 9 show that indeed constructs comprising different ITSM motifs and ITIM motifs from other inhibitory tails can improve stringency of the chimeric polypeptides and system according to the invention. All of the tested domains comprise both an ITSM and an ITIM except for LY9, which only comprises two ITSM domains and no ITIM domain (see also Figure 11). In addition, Figure 10 shows that alternative SH2 containing docking domains may be used in the invention. All of the alternative SH2 domains tested interact with phosphorylated ITAMs (see also Figure 12) within the context of the current invention (Katsuyama et al, Front Immunol. 2018; 9: 1088, Ngoenkam et al, Immunology. 2018 Jan; 153(1): 42-50, Koch et al, Trends Immunol. 2013 Apr;34(4):182-91).

The broad applicability of CRASH-IT platform was further demonstrated in NK cells as well. The NK cell line KHYG-1 can effectively recognize and kill HLA class I and class II deficient K562 tumor using its endogenous NK cell activation receptors (Suck et al, Exp Hematol. 2005 Oct;33(10):1160-71). Expression of Zap70-PD1-FKBP12^{F36V} switch efficiently suppressed IFNγ, IL2 and TNFα production in NK cells co-cultured with K562 tumor in the absence of drug, and the suppression was reversed in the presence of dTAG-13 PROTAC (Figure 13).

While current CAR designs frequently comprise ITAM containing CD3 zeta chain signaling domains, alternative ITAM-containing-signaling-domains such as fragments from the CD3 epsilon chain (Nolan et al, Clin Cancer Res. 1999 Dec;5(12):3928-41), gamma (γ) chain of the immunoglobulin receptor FcεRI (Ren-Heidenreich et al, Cancer Immunol Immunother. 2002 Oct;51(8):417-23) and DAP12 (Töpfer et al, J Immunol. 2015 Apr 1;194(7):3201-12) were also reported. We tested compatibility of the CRASH-IT switch with a panel of CARs containing alternative ITAM containing signaling domains and showed inhibition of T cell functions in the absence of drug, and drug induced restoration of T cell functions upon addition of drug in T cells co-expressing different CAR and the CRASH-IT switch (Figure 14).

The CRASH-IT embodiment that utilizes the SMASh domain contains HCV-derived protein sequences that may potentially be immunogenic in immunocompetent hosts. A further embodiment of CRASH-IT platform, based on the FKBP12^{F36V} domain, can be regulated by PROTAC molecules such as dTAG-13. However, the size of dTAG-13 (molecular weight: 1049.18) may limit its oral availability.

We therefore also assessed whether it was possible to combine the CRASH-IT switch system with an alternative protein stability control domain that can be regulated by small molecules that do show oral bioavailability and that are used clinically. We found that CRASH-IT embodiments that comprised a CRBN polypeptide substrate domain capable of binding to the CRBN protein in response to a drug, thereby promoting ubiquitin pathway-mediated degradation of the chimeric polypeptide may successfully be employed in the current invention. This was shown using CRASH-IT embodiments that comprised, next to the first part comprising a SH2-domain from a protein which binds to a phosphorylated immunoreceptor tyrosine-based activation motif and, preferably, a third part, comprising an immunoreceptor tyrosine-based switch motif (ITSM), preferably an ITSM and an immunoreceptor tyrosine-based inhibitory motif (ITIM), and a second part (i.e. the part comprising a small molecule-regulated protein stability domain) containing zinc finger based degrons. Such CRASH-IT embodiments can be successfully regulated by Immunomodulatory imide drugs (IMiDs, also known as Cereblon modulators) that include clinically approved, orally available molecules, such as thalidomide, lenalidomide and pomalidomide.

Thalidomide (molecular weight: 258.23), lenalidomide (molecular weight: 259.26) and pomalidomide (molecular weight: 273.24) are immunomodulatory drugs (IMiDs) that are clinically used for the treatment of multiple myeloma. Prior work has demonstrated that the activity of these compounds relies on the CRBN E3 ligase-dependent degradation of, for example, the IKZF1 protein. Moreover, it has been shown that a 23-amino-acid-long zinc finger motif (IKZF1 zinc finger 2, ZF2) within the IKZF1 sequence constitutes the minimal degron (or the a CRBN polypeptide substrate domain) for thalidomide-, lenalidomide- and pomalidomide-dependent protein degradation. Combination of the IKZF1 ZF2 sequence with the adjacent zinc finger 3 (IKZF1 ZF3) that interacts with CRBN-IKZF1 ZF2 interface, yields a ~57 amino acids long improved IKZF1-derived degron (Sievers et al, Science. 2018 Nov 2;362(6414). pii: eaat0572). This improved degron sequence can, like other CRBN polypeptide substrate domain capable of binding to the CRBN protein in response to a drug, for example, be fused to a protein of interest such that stability of resultant protein can be regulated by addition of thalidomide, lenalidomide or pomalidomide or any other IMiDs (Koduri et al, Proc Natl Acad Sci USA. 2019 Feb 12;116(7):2539-2544).

Pomalidomide and lenalidomide are second generation IMiDs that differ from thalidomide by an aniline group in the solvent exposed phthalimide ring at the C4 position. Lenalidomide and pomalidomide are thought to show improved efficacy compared to thalidomide due to the presence of this aniline group. Both the therapeutic and adverse effects of these 3 drugs (myelosuppression, anti-inflammatory activity, T cell co-stimulation, NK cell proliferation, anti-angiogenic and teratogenic effects) are overlapping but differ in magnitude between the 3 drugs. The recommended starting doses of thalidomide, lenalidomide and pomalidomide are 200 mg/day, 25 mg/day and 4 mg/day, respectively, reflecting their differences in potency.

In order to regulate activity levels of T cells with IMiDs, a CRASH-IT switch comprising the Zap70 2xSH2 domains, the PD1 signaling domain and the IKZF1 ZF2-ZF3 optimal degron was created (Figures 15-16). When primary T cells were co-transduced with a CDK4 neoantigen-specific TCR plus the IKZF1-based CRASH-IT switch and co-cultured with NKIRTIL006 tumor cells that endogenously express the CDK4 neoantigen, cytokine production and cell surface LAMP-1 expression was tightly suppressed in the absence of IMiD, but activity levels were robustly restored in the presence of pomalidomide or lenalidomide, but lesser in the presence of thalidomide (Figure 16).

The anti-inflammatory effects of IMiDs could potentially interfere with the activity of cellular therapies in case safety switch inactivation would require high drug concentrations. For this reason, it is desirable to create engineered degrons that display a higher affinity to IMiDs, such that safety switch regulation can be achieved at drug concentrations at which degradation of endogenous targets is minimal. It has previously been reported that a hybrid zinc finger motif containing the beta turn sequence of ZFP91 ZF4 and the alpha helix sequence of IKZF1 ZF2 displays a higher affinity towards thalidomide, as compared to the parental ZFP91 ZF4 and IKZF1 ZF2 sequences (Sievers et al, Science. 2018 Nov 2;362(6414). pii: eaat0572). In this analysis, the IKZF1 ZF3 sequence was not included in the zinc finger degron, although it was separately reported that the IKZF1 ZF2-3 sequence shows superior protein degradation potential as compared to the IKZF1 ZF2 sequence.

To generate degrons that interact with thalidomide with high affinity, we replaced the IKZF1 ZF2 beta turn sequence from the IKZF1 ZF2-3 degron with the beta turn sequences from various zinc fingers (ZNF653 ZF4, ZFP91 ZF4, ZNF276 ZF4, ZNF827 ZF1). Analysis of T cell activity as a function of drug concentration revealed that hybrid zinc fingers containing ZNF653, ZFP91, ZNF276 and ZNF827 beta turn grafts could be inactivated at a reduced concentration of either lenalidomide, pomalidomide, and thalidomide, with a particularly profound improvement in drug sensitivity for the latter (Figure 16). Specifically, the thalidomide concentration required to achieve the same level of cytokine production (IFNγ, IL-2, orTNFα), or surface LAMP-1 expression levels was reduced around 1,000-fold (Figure 16D) forT cells expressing the chimeric degrons as compared to the parental IKZF1 ZF2-3 degron. Thus, Zn finger-based CRASH-IT switches such as Zap70-PD1-ZFP91/IKZF1 can be used to regulate T cell functions at thalidomide concentrations at which thalidomide binding of its normal ligand is minimal.

Notably, in these IMiD titration experiments effective T cell reactivation was also observed at high drug concentrations. In contrast, the use of high drug concentrations in FKBP12^{F36V}/dTAG-13 based switches results in a suboptimal reactivation of T cell function. A suboptimal degradation of protein targets at high PROTAC concentrations has been reported previously, and has been attributed to a so-called "hook effect" (An et al, EBioMedicine. 2018 Oct;36:553-562), in which E3 ligase-PROTAC and PROTAC-target protein (such as FKBP12^{F36V}) dimers dominate over the intended E3-ligase-PROTAC-target protein trimer. The lack of a noticeable hook effect in zinc finger/IMiD combinations facilitates the clinical use of IMiDs, by allowing optimal T cell reactivation at both the Cmax and Cmin. Finally, the small size of zinc finger based degrons (~57 amino acids) as compared to e.g. the FKBP12^{F36V} domain (107 amino acids) enables compact vector design, including the design of single vector systems in which expression of CAR/TCR and the CRASH-IT switch are linked.

Finally, we compared efficiency of Zap70-PD1-Zinc finger constructs that contain ZFP91/IKZF1 hybrid zinc finger with or without IKZF1 ZF3 (double ZF and single ZF, respectively), or wild type double zinc fingers derived from IKZF1, IKZF3, ZFP91, ZNF276, ZNF653 (Figure 17A). The results show that activity of T cells expressing the CDK4 TCR and the Zap70-PD1-ZFP91/IKZF1 hybrid zinc finger double ZF degron can be restored most efficiently in the presence of IMiDs (Figure 17B-E).

The chimeric polypeptides and system according to the current invention, CRASH-IT, is a titratable and reversible T cell and NK cell safety switch platform that is agnostic to the nature of the activating antigen receptor, as shown by its use together with high and intermediate affinity class I-restricted TCRs, class II restricted TCRs and CARs in the context of CD4 T cells, CD8 T cells and NK cells.

The flexible nature of CRASH-IT will be of value in settings in which fine control over T cell and NK cell sensitivity is desirable. Furthermore, combination of CRASH-IT with existing CARs does not require a structural re-design of CARs, and this is of particular value for CAR designs that have already been evaluated in clinical studies.

### Materials and Methods

### Retroviral DNA constructs

All DNA constructs were generated in the MP71 retroviral expression vector backbone (Engels et al, Hum Gene Ther. 2003 Aug 10;14(12):1155-68). In brief, codon optimized DNA sequences were synthesized by IDT as gene fragments and cloned into the MP71 vector by Gibson assembly (Gibson et al, Nat Methods. 2009 May;6(5):343-5). To generate the MP71-Zap70 2xSH2-PD1 tail IRES-EGFP vector, codon optimized sequences encoding a human Zap70 fragment (P43403, 1-264 aa), a GGS linker, the human PD1 intracellular domain (Q15116, 192-288 aa), followed by a stop codon and an IRES-EGFP reporter were cloned into MP71.

Other constructs containing the MP71 IRES-EGFP backbone were created from following coding sequences: MP71 Zap70 - 2xSH2 domains-IRES-EGFP: human Zap70 (P43403, 1-264 aa), MP71 PD1-tail-IRES-EGFP: MV coding sequence (i.e. start codon methionine plus an additional valine to generate a Kozak sequence), and the human PD1 intracellular domain (Q15116, 192-288 aa). MP71- Zap70-PD1-SMASh-IRES-EGFP: human Zap70 (P43403, 1-264 aa), a GGS linker, the human PD1 intracellular domain (Q15116, 192-288 aa), an SGGGS linker, and the 304 aa SMASh tag (Chung et al, Nat Chem Biol. 2015 Sep;11(9):713-20). MP71-Zap70 2xSH2-SMASh-IRES-EGFP: human Zap70 (P43403, 1-264 aa), an SGGGS short linker, and the 304 aa SMASh tag. MP71-IRES-EGFP vector control: the irrelevant 248 aa Tet-On 3G trans-activator (Clontech). MP71-HA-Zap70-PD1-SMASh tag-IRES-EGFP: the HA tag (MVYPYDVPDYAGSGV) coding sequence followed by the Zap70-PD1-SMASh coding sequence. MP71-tuZap70-PD1-SMASh-IRES-EGFP: an additional alanine residue encoding DNA was added after the start codon in MP71-Zap70-PD1-SMASh-IRES-EGFP. MP71-Zap70-PD1-SMASh-delta-IRES-EGFP (lacking the degron sequence): DNA encoding the last 78 aa of the SMASh tag from MP71-Zap70-PD1-SMASh-iresEGFP construct were deleted. MP71 CD19 ScFv-CD28-CD3 ζ CAR-IRES-huEGFRt: a second generation CD19 specific CAR was subcloned into the MP71 backbone from the SFG-19-28z vector (Brentjens et al, Clin Cancer Res. 2007 Sep 15;13(18 Pt 1):5426-35) together with an IRES truncated human EGFR (huEGFRt) reporter (Wang et al. 2011) by Gibson assembly. MP71-IRES-huEGFRt vector control: the CAR insert in MP71 CD19 ScFv-CD28-CD3 ζ CAR-IRES-huEGFRt vector was replaced by the irrelevant 248 aa Tet-On 3G trans-activator protein coding sequence (Clontech). PD1 cytoplasmic domain (PD1 tail) coding sequence within MP71- Zap70-PD1-SMASh-IRES-EGFP vector was replaced by DNA sequences encoding cytoplasmic domains of BTLA (Q7Z6A9, 179-289 aa), SIRPA (P78324, 395-504 aa), SIGLEC5 (015389, 463 - 551 aa), SIGLEC9 (Q9Y336, 370-463 aa), SIGLEC11 (Q96RL6, 585-698 aa), PECAM1 (P16284-1, 621-738 aa) and LY9 (Q9HBG7, 477-655 aa) to construct CRASH-IT embodiments encoding corresponding inhibitory cytoplasmic domains. Zap70 2xSH2 domains encoding sequence (P43403, 1-264 aa) within MP71- Zap70-PD1-SMASh-IRES-EGFP vector was replaced by DNA sequences encoding Syk 2xSH2 (P43405-1, 1-287 aa) or Lck SH4-Unique-SH3-SH2 (P06239-1, 1-254 aa) sequences to construct CRASH-IT embodiments encoding corresponding SH2 domains. SMASh domain encoding sequence within MP71- Zap70-PD1-SMASh-IRES-EGFP vector was replaced by DNA sequence encoding FKBP^{F36V} to construct CRASH-IT embodiment that can be controlled by dTAG-13 PROTAC (Nabet et al, Nat Chem Biol. 2018 May;14(5):431-441).

To generate a CAR-T panel encoding alternative ITAM containing domains, the CD3 ζ chain in MP71 CD19 ScFv-CD28-CD3 ζ CAR-IRES-huEGFRt vector was replaced with ITAM containing cytoplasmic domains of either the gamma chain of the immunoglobulin receptor FcεRI (FCER1G) (P30273, 45-86 aa), CD3 epsilon chain (P07766, 153-207 aa), or DAP12 (043914, 62-113 aa). Alternatively, the CD3 ζ chain was deleted to generate a CAR construct without ITAM containing domain (negative control). The CD28-CD3 ζ encoding sequence in MP71 CD19 ScFv-CD28-CD3 ζ CAR-IRES-huEGFRt vector was replaced with full length CD3 epsilon chain sequence CD3E (P07766, 25-207 aa) to generate MP71 CD19 ScFv-CD3E CAR-IRES-huEGFRt.

Constructs containing various zinc finger degrons in MP71 Zap70-PD1-Zinc finger IRES-EGFP format were created using IKZF1 ZF2-3 degron containing sequence (Q13422, 141-197 aa), or IKZF3 ZF2-3 degron containing sequence (Q9UKT9, 142-198 aa), or ZFP91 ZF4-5 degron containing sequence (Q96JP5, 396-455 aa), ZNF276 ZF4-5 degron containing sequence (Q8N554, 520-579 aa), ZNF653 ZF4-5 degron containing sequence (Q96CK0, 552-611 aa), or ZNF692 ZF4-5 degron containing sequence (Q9BU19, 413-473 aa). For CRASH-IT switches encoding hybrid zinc finger degrons, the IKZF1 ZF2 beta turn sequence (FQCNQCGASFT) was replaced with beta turn sequences from ZNF653 ZF4 (LQCEICGYQCR), ZFP91 ZF4 (LQCEICGFTCR), ZNF276 ZF4 (LQCEVCGFQCR), or ZNF827 ZF1 (FQCPICGLVIK). For the CRASH-IT switch encoding ZFP91/IKZF1 hybrid zinc finger without IKZF1 ZF3 (single ZF), the IKZF1 ZF3 containing sequence (Q13422, 170-197 aa) was deleted from the Zap70-PD1-ZFP91/IKZF1 hybrid zinc finger (double ZF) construct.

The HLA class I-restricted CDK4 TCR (TCR 17, Strønen et al, Science. 2016 Jun 10;352(6291):1337-41) and NY-ESO-1 TCR (TCR 1, Linnemann et al, Nat Med. 2013 Nov;19(11):1534-41) have been described previously. The variable domain sequences of the HLA class II-restricted CMV-pp65 TCR (van Loenen et al, PLoS One. 2013 May 30;8(5):e65212) were kindly provided by M.H. Heemskerk (LUMC, NL) and were cloned into the TCR flex MP71 vector (Linnemann et al, Nat Med. 2013 Nov;19(11):1534-41).

### Cell lines and cell culture

FLYRD18, T2, MM90904 (a kind gift from Marco Donia, Herlev Hospital, Denmark), Mel526 (Stronen et al, Science. 2016 Jun 10;352(6291):1337-41), NKIRTIL006 (Kvistborg et al, Oncoimmunology. 2012 Jul 1; 1(4): 409-418), K562, Daudi, Raji and CBH 5477 (a kind gift from M.H. Heemskerk) cells were cultured in IMDM (Invitrogen, #21980065), supplemented with 8 % FCS (Invitrogen, #F7524-500ML) and penicillin-streptomycin (100 IU/ml penicillin, 100 µg/ml streptomycin, Sigma-Aldrich, #11074440001). FLYRD18, MM90904, Mel526 and NKIRTIL006 cells were passaged every 2-3 days with trypsin-EDTA (Invitrogen, # 15400054).

Human NK cell line KHYG-1 (DSMZ, Leibniz, Germany) was cultured in RPMI supplemented with 8% FBS and penicillin-streptomycin (100 IU/ml penicillin, 100 µg/ml streptomycin) containing 500 IU/ml IL-2 (Novartis). All cell lines were tested for mycoplasma using PCR based screening (Young et al, Nat Protoc. 2010 May;5(5):929-34) and found negative.

### Retrovirus production

Retroviral particles were produced in FLYRD18 packaging cells. In brief, 700,000 FLYRD18 packaging cells were plated per 10 cm dish one day prior to transfection. The next day, cell culture medium was refreshed with IMDM supplemented with 8% FCS without antibiotics. 25 µl of X-tremeGENE 9 (Roche, #6365809001) was mixed with 800 µl Opti-MEM (Invitrogen, #11058-021) and incubated for 5 minutes. Subsequently, the Optimem-X-tremeGENE 9 mixture was added on top of 10 µg retroviral plasmid DNA dissolved in water and incubated for 15 minutes, and the resulting transfection mixture was added dropwise onto the packaging cells. The supernatant containing retrovirus was harvested 48 hours after transfection and immediately used or snap-frozen in liquid nitrogen.

### T cell isolation and activation

Peripheral blood mononuclear cells (PBMC) were isolated from buffy coats from healthy donors (Sanquin (Amsterdam, NL) by Ficoll-Isopaque density centrifugation (Linnemann et al, Nat Med. 2013 Nov;19(11):1534-41) and were stored frozen until further use. To generate activated T cell populations, PBMC were thawed in PBS containing 5 % FCS, counted, and mixed with CD3/CD28 Dynabeads (CTS, #40203D) at a 1: 1 cell to bead ratio, at a density of 10⁷ cells/ml.

Following a 30 min incubation at room temperature on a tumbler, the mixture was put on a magnet and unbound cells were removed. Bead bound T cells were subsequently resuspended in RPMI supplemented with 10% human serum (Sigma-Aldrich, #H3667-100ML) and penicillin-streptomycin containing 100 IU/ml IL-2 (Novartis) and 5 ng/ml IL-15 (Peprotech, #200-15), and plated at a density of 0.75 × 10⁶ cells/ml.

### Spin-transduction of T cells and NK cells

24-well non treated cell culture plates were covered with 10µg/ml retronectin (Takara, #T100B) overnight at 4 °C. The next day, the retronectin solution was removed and wells were blocked with 2% BSA (Sigma-Aldrich, A9418-500g) in PBS for 30 minutes. Activated T cells (0.25 × 10⁶ cells/ml in RPMI/10% human serum/penicillin-streptomycin/200 IU/ml IL-2 and 10 ng/ml IL-15) or KHYG-1 NK cells (0.25 × 10⁶ cells/ml in RPMI/8% FCS/penicillin-streptomycin/1000 IU/ml IL-2) were then mixed with retroviral supernatant at a 1:1 ratio (volume/volume) in retronectin coated 24 well plates and centrifuged at 2,000 RPM for 90 minutes at room temperature.

### Peptide loading

For use as T cell targets, T2 and CBH 5477 cells were loaded with the indicated concentrations of HLA-A*02:01 restricted mutant CDK4 peptide (ALDPHSGHFV), HLA A*02:01 restricted NY-ESO-1 peptide (SLLMWITQA), or HLA-DR1 restricted CMV peptide (KYQEFFWDANDIYRI) in IMDM for 1 hour at 37 °C. Cells were then washed once and used in co-culture experiments.

### Cytokine release assay and antibody staining

T cells and NK cells were pretreated with the indicated concentrations of asunaprevir (MedChemExpress, # HY-14434), grazoprevir (MedChemExpress, # HY-15298), dTAG-13 (Tocris, #6605) or DMSO control in T cell media (RPMI/10 % human serum/penicillin-streptomycin, 100 IU/ml IL2 and 5 ng/ml IL15) or NK cell media (RPMI/8 % FCS/penicillin-streptomycin, 500 lU/ml IL2), respectively, for 24 hours before co-culture experiments. 100,000 T cells or NK cells and 100,000 of indicated tumor cells were mixed in T cell or NK cell media supplemented with golgi-plug (1:1000 dilution, BD, #51-2301KZ) and anti-LAMP1-APC (1:100 dilution, Biolegend, #328620) in the presence of indicated drugs or DMSO control in 96-well plates and incubated for 5 hours at 37 °C.

After incubation, cells were washed once with PBS and stained with IR dye (Invitrogen, #L34976) at 1:400 dilution for 5 minutes at 4 °C. Subsequently, cells were washed once with FACS buffer (PBS plus 0.5 % BSA) and stained with anti-CD8-PerCP Cy5.5 (1:20 dilution, BD, #341050), anti-CD4 BV711 (1:50 dilution, Biolegend, #317440), anti-murine constant TCR-PE (in experiments with transduced TCRs, 1:200 dilution, BD, #553172) or cetuximab-PE (in experiments with transduced CAR construct containing truncated human EGFR reporter, 1:200 dilution, R&D Systems, #FAB9577P) for 20 minutes at 4 °C. Cells were then washed once with FACS buffer and fixed with BD fixation and permeabilization solution (#51-2090KZ) for 20 minutes at 4 °C.

Following permeabilization, cells were washed twice with BD perm/wash buffer (#51-2091KZ) and stained with anti-IFNy-BV421 (1:100 dilution, BD, #564791), anti-IL-2-PE-Cy7 (1:100 dilution, BD, #560707) and anti-TNFα-BV650 (1:100 dilution, Biolegend, #502938) diluted in perm/wash buffer for 20 min at 4 °C. Cells were then washed twice, resuspended in 100 µl FACS buffer and analyzed directly on a Fortessa Special Order analyzer. Data were analyzed using FlowJo and Prism 7 software.

Similarly, T cells were intracellularly stained with anti-HA-AF647 (1:200 dilution, Cell Signaling Technology, #3444S), and K562, Raji and Daudi tumor cells were cell surface stained with anti-CD19-PE (1:200 dilution, BD, #345789) or isotype control (Biolegend, #400111) as described above.

### Sorting and rapid expansion of T cells

Culture media of Zap70-PD1-FKBP^{F36V} expressing cells is supplemented with 0.5 µM dTAG-13 PROTAC starting one day before cell sorting until 4 days before ⁵¹Cr assay. Primary human T cells modified with the CDK4 TCR plus either the Zap70-PD1-FKBP^{F36V} switch or IRES-EGFP vector control were sorted on a Beckman Coulter Moflo Astrios using an 80 µM nozzle. Cells were cultured in standard T cell culturing conditions in RPMI/10 % human serum/penicillin-streptomycin 100 IU/ml IL-2 and 5 ng/ml IL-15 for 7 days.

After this, cells were expanded using a rapid expansion protocol (REP). In brief, a mixture of 2 × 10⁸ feeder cells from 3 donors was generated by irradiation at 4,000 rad (Gammacell 40 Exactor) and resulting feeder cells were then mixed with 1 × 10⁶ sorted T cells, and 4.5 µg OKT3 (Invitrogen, #16-0037-85), IL-2 (3000 lU/ml final concentration) in 150 ml 20/80 T cell Mixed Media (Invitrogen, #041-96658P) supplemented with penicillin-streptomycin (100 IU/ml penicillin, 100 µg/ml streptomycin). At day 6, culture media is refreshed with 3000 IU/ml IL-2 containing media and cultures were split into 2 every 3 days with media containing IL-2 (3000 IU/ml final concentration). At day 12, cells were switched to standard T cell culturing conditions (RPMI/10 % human serum/penicillin-streptomycin/100 IU/ml IL-2 and 5 ng/ml IL-15) for 3 days, until use in ⁵¹Cr assays.

### ⁵¹Cr assay

One day prior to ⁵¹Cr assay, T cells were pretreated with 0.5 µM dTAG-13 PROTAC or DMSO in standard T cell culturing conditions in RPMI/10 % human serum/penicillin-streptomycin/100 IU/ml IL-2 and 5 ng/ml IL-15. 5 × 10⁵ tumor cells were resuspended in 100 µl media, mixed gently with 100 µCi⁵¹Cr and then incubated at 37 °C for 45 minutes. In parallel, 100 µl of dilutions of T cells treated with either dTAG-13 or DMSO control were aliquoted into 96 well plates. 100 µl medium only (spontaneous release) and 100 µl 1 % Triton solution (maximal release) were used as controls.

After labeling, target cells were washed three times with 1 ml medium. Labeled target cells were resuspended at 50,000 cells/ml, and target cells were added to the 96 well plates at 100 µl per well. Subsequently, plates were centrifuged at 900 rpm for 2 minutes and incubated at 37 °C for 4 hours. After incubation, 50 µl of supernatant was added onto a Lumaplate-96 (Packard Bioscience, #6005164) and, following overnight drying, counts were determined using a PerkinElmer, TopCount NXT. Experimental values were normalized using spontaneous and maximal release controls.

Having now fully described this invention, it will be appreciated by those skilled in the art that the same can be performed within a wide range of equivalent parameters, concentrations, and conditions without departing from the scope of the invention as defined in the claims and without undue experimentation.

Reference to known method steps, conventional methods steps, known methods or conventional methods is not in any way an admission that any aspect, description or embodiment of the present invention is disclosed, taught or suggested in the relevant art.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

1. A cell comprising a chimeric polypeptide, or comprising a nucleic acid comprising a polynucleotide encoding said chimeric polypeptide, wherein the chimeric polypeptide comprises:
a) a first part comprising a SH2-domain from a protein which binds to a phosphorylated immunoreceptor tyrosine-based activation motif (ITAM);
b) a second part comprising a small molecule-regulated destabilization domain; and
c) a third part comprising an immunoreceptor tyrosine-based switch motif (ITSM), and/or an immunoreceptor tyrosine-based inhibitory motif (ITIM), preferably an ITSM and an ITIM.

2. The cell according to claim 1, wherein the small molecule-regulated destabilization domain is selected from the group consisting of
i)a self-excising degron (SED), wherein the SED comprises a repressible protease, a cognate cleavage site, and a degron sequence;
ii) a proteolysis-targeting chimera (Protac) binding domain wherein the Protac comprises a E3 ubiquitin ligase binding group (E3LB), optionally a linker, and a protein binding group that binds to the Protac binding domain in the chimeric polypeptide; and
iii) a CRBN polypeptide substrate domain capable of binding to the CRBN protein in response to a drug, thereby promoting ubiquitin pathway-mediated degradation of the chimeric polypeptide.

3. The cell according to any one of the previous claims wherein the ITAM is an ITAM comprised in a T cell receptor (TCR) complex and/or a NK cell receptor (NKR) complex and/or a chimeric antigen receptor (CAR), preferably an ITAM derived from a CD3 zeta chain, a CD3 epsilon chain, a CD3 delta chain, CD3 gamma chain, gamma chain of the immunoglobulin receptor FcεRI and DAP12, and/or wherein the cell further comprises a T cell receptor and/or a chimeric antigen receptor (CAR) and/or an NK cell receptor (NKR), preferably wherein the cell is a T cell and/or CAR T cell and/or NK cell and/or CAR NK cell.

4. The cell according to any one of the previous claims wherein the SH2-domain is from a protein selected from the group consisting of Zap70, Syk, and Lck, and/or wherein the chimeric polypeptide comprises more than one SH2-domain from a protein which binds a phosphorylated immunoreceptor tyrosine-based activation motif.

5. The cell according to any one of the previous claims wherein the ITIM and/or ITSM is from an inhibitory receptor protein, preferably an inhibitory immune receptor protein, preferably from a protein selected from the group consisting of PD1, BTLA, SIRPalpha, SIGLEC5, SIGLEC9, SIGLEC11, PECAM1 or LY9.

6. The cell according to any one of the previous claims wherein the CRBN polypeptide substrate domain is a C2H2 zinc finger protein or a fragment thereof that is capable of drug-inducible binding to the CRBN polypeptide, and/or wherein the CRBN polypeptide substrate domain, is selected from the group consisting of IKZF1, IKZF3, ZFN654, ZNF787, ZNF653, ZFP91, ZNF276, ZNF827, or a fragment thereof that is capable of drug- inducible binding to the CRBN polypeptide, preferably wherein said fragment is selected from the group consisting of IKZF1 ZF2-3 (SEQ ID NO:41), IKZF3 ZF2-3 (SEQ ID NO:42), ZFP91 ZF4-5 (SEQ ID NO: 43), ZNF276 ZF4-5 (SEQ ID NO:44), ZNF653 ZF4-5 (SEQ ID NO: 45), and ZNF692 ZF4-5 (SEQ ID NO:46) and/or wherein the CRBN polypeptide substrate domain comprises a hybrid fusion polypeptide wherein the hybrid fusion polypeptide comprises at least a first fragment of a first C2H2 zinc finger protein and a second fragment from a second C2H2 zinc finger protein, wherein the combination of said first fragment and said second fragment in the hybrid fusion polypeptide is capable of drug-inducible binding to the CRBN polypeptide, preferably wherein the hybrid fusion polypeptide comprised in the CRBN polypeptide substrate domain comprises a first fragment selected from the beta-turn of ZFP91 ZF4 (LQCEICGFTCR; SEQ ID NO: 52), ZFN653 ZF4 (LQCEICGYQCR; SEQ ID NO 53), ZNF276 ZF4 (LQCEVCGFQCR: SEQ ID NO: 54), ZNF827 ZF1 (FQCPICGLVIK; SEQ ID NO:55) and a second fragment selected from the alpha-helix of IKZF1 ZF2 (QKGNLLRHIKLH; SEQ ID NO: 56), preferably the hybrid fusion polypeptide comprises the beta-turn of ZFP91 ZF4 and the alpha-helix of IKZF1 ZF2, preferably wherein the hybrid fusion polypeptide comprises one selected from SEQ ID NO 47-51.

7. The cell according to any one of the previous claims wherein the CRBN polypeptide substrate binding domain comprises or further comprises IKZF1 ZF3 (FKCHLCNYACRRRDALTGHLRTH; SEQ ID NO: 57), preferably wherein the CRBN polypeptide substrate binding domain comprises the beta-turn of ZFP91 ZF4, the alpha-helix of IKZF1 ZF2, and IKZF1 ZF3, and/or wherein the drug that allows the CRBN polypeptide substrate domain to bind to the CRBN protein, thereby promoting ubiquitin pathway-mediated degradation of the chimeric polypeptide, is an IMiD, preferably selected from the group consisting of thalidomide, lenalidomide, pomalidomide, CC-122 (avadomide), CC-220 (iberdomide) and CC-885.

8. The cell according to any one of the previous claims wherein
a) the SH2-domain comprises or consists of an amino acid sequence having at least 80% identity to an amino acid sequence according to SEQ ID NO 7-11 or comprises or consists of an amino acid sequence having at least 80% identity to an amino acid sequence according to SEQ ID NO 12 - 14;
b) the first part of the chimeric polypeptide comprises or consists of an amino acid sequence having at least 80% identity to an amino acid sequence according to SEQ ID NO 7 - 11 or comprises or consists of an amino acid sequence having at least 80% identity to an amino acid sequence according to SEQ ID NO 12 - 14;
c) the ITAM is YxxL/Ix(6-8)YxxL/I;
d) the SH2-domain is a SH2-domain that binds to a phosphorylated ITAM comprised in SEQ ID NO 1 - 6, or that binds to an amino acid sequence having 80% or more identity to an amino acid sequence according to SEQ ID NO 1- 6;
e) the ITIM is S/I/V/LxYxxI/V/L, and/or the ITSM is TxYxxV/I;
f)the ITSM and/or ITIM is an ITSM and/or ITIM that is comprised in SEQ ID No 15-22;
g) the third part of the chimeric polypeptide comprises or consists of an amino acid sequence having at least 80% identity to an amino acid sequence according to SEQ ID NO 15-22;
h) the second part of the chimeric polypeptide comprises or consists of an amino acid second according to SEQ ID NO:35 or 40 or SEQ ID NO: 41 - 57; and/or
i)the chimeric polypeptide comprises an amino acid sequence having 80% or more identity to an amino acid sequence according to SEQ ID NO 23 - 34, SEQ ID NO 36, or SEQ ID NO 58-68.

9. A chimeric polypeptide as defined in any one of claims 1 - 8, and/or a nucleic acid comprising a polynucleotide encoding a chimeric polypeptide as defined in any one of claims 1-8, and/or a vector comprising said nucleic acid and/or a cell comprising said vector.

10. A pharmaceutical composition comprising the cell of any one of claim 1-8, the chimeric polypeptide, the nucleic acid and/or the vector of claim 9.

11. A cell according to claims 1 - 8 for use as a medicament, preferably for use in the treatment of cancer in a subject, preferably wherein the treatment comprises
a) administering to the subject a population of cells according to any one of claims 1 - 8;
b) optionally, administering to the subject an inhibitor of the repressible protease or a Protac that binds to the proteolysis-targeting chimera (Protac) binding domain or a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally, if step b) is performed, increasing or reducing the concentration of the inhibitor of the repressible protease or a Protac that binds to the proteolysis-targeting chimera (Protac) binding domain or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885.

12. A method of providing a cell according to any one of claims 1 - 8 wherein the method comprises contacting the cell with a nucleic acid or the vector of claim 9, wherein the nucleic acid or vector is contacted with the cell ex vivo.

13. A method of controlling expression of a chimeric polypeptide in a cell, wherein the method comprises contacting the cell expressing the chimeric polypeptide according to claim 9 with an inhibitor of the repressible protease, with a Protac that binds to the proteolysis-targeting chimera (Protac) binding domain, or with the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885, and wherein the cell expressing the chimeric polypeptide is contacted with the inhibitor ex vivo.

14. A method of controlling cytotoxic activity of T cells and/or NK cells and/or of controlling cytokine secretion by T cells and/or NK cells wherein the method comprises
a) expressing a chimeric polypeptide according to claim 9 in the T cell and/or NK cell;
b) ex vivo contacting the cells with an inhibitor of the repressible protease, with a Protac that binds to the proteolysis-targeting chimera (Protac) binding domain or with a drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885;
c) optionally, increasing or reducing the concentration of the inhibitor of the repressible protease, the Protac, or the drug that allows the CRBN polypeptide substrate domain to bind to the CRBN protein, thereby promoting ubiquitin pathway-mediated degradation of the chimeric polypeptide, preferably wherein the drug is an IMiD, preferably selected from the group consisting of thalidomide, lenalidomide, pomalidomide, CC-122 (avadomide), CC-220 (iberdomide) and CC-885.

## Patentansprüche

1. Zelle, umfassend ein chimäres Polypeptid oder umfassend eine Nukleinsäure, die ein Polynukleotid umfasst, das das chimäre Polypeptid codiert, wobei das chimäre Polypeptid Folgendes umfasst:
a) einen ersten Teil, der eine SH2-Domäne aus einem Protein umfasst, das an ein phosphoryliertes Immunrezeptor-Tyrosin-basiertes Aktivierungsmotiv (ITAM) bindet,
b) einen zweiten Teil, der eine durch ein kleines Molekül regulierte Destabilisierungsdomäne umfasst, und
c) einen dritten Teil, der ein Immunrezeptor-Tyrosin-basiertes Schaltermotiv (ITSM) und/oder ein Immunrezeptor-Tyrosin-basiertes inhibitorisches Motiv (ITIM), vorzugsweise ein ITSM und ein ITIM, umfasst.

2. Zelle nach Anspruch 1, wobei die durch ein kleines Molekül regulierte Destabilisierungsdomäne aus der Gruppe ausgewählt ist, bestehend aus
i) einem selbstexzidierenden Degron (SED), wobei das SED eine reprimierbare Protease, eine zugehörige Spaltstelle und eine Degronsequenz umfasst,
ii) einer auf Proteolyse abzielende Chimäre(proteolysis-targeting chimera, Protac)-Bindungsdomäne, wobei die Protac eine E3-Ubiquitinligase-Bindungsgruppe (E3LB), optional einen Linker und eine Proteinbindungsgruppe umfasst, die an die Protac-Bindungsdomäne im chimären Polypeptid bindet, und
iii) einer CRBN-Polypeptid-Substrat-Domäne, die in der Lage ist, als Reaktion auf einen Arzneistoff an das CRBN-Protein zu binden, wodurch der durch den Ubiquitinweg vermittelte Abbau des chimären Polypeptids gefördert wird.

3. Zelle nach einem der vorhergehenden Ansprüche, wobei das ITAM ein in einem T-Zell-Rezeptor(TCR)-Komplex und/oder einem NK-Zell-Rezeptor(NKR)-Komplex und/oder einem chimären Antigenrezeptor (CAR) enthaltenes ITAM ist, vorzugsweise ein von einer CD3-zeta-Kette, einer CD3-epsilon-Kette, einer CD3-delta-Kette, CD3-gamma-Kette, gamma-Kette des Immunglobulinrezeptors FcεRI und DAP12 stammendes ITAM ist und/oder wobei die Zelle ferner einen T-Zell-Rezeptor und/oder einen chimären Antigenrezeptor (CAR) und/oder einen NK-Zell-Rezeptor (NKR) umfasst, wobei vorzugsweise die Zelle eine T-Zelle und/oder CAR-T-Zelle und/oder NK-Zelle und/oder CAR-NK-Zelle ist.

4. Zelle nach einem der vorhergehenden Ansprüche, wobei die SH2-Domäne aus einem Protein stammt, das aus der aus Zap70, Syk und Lck bestehenden Gruppe ausgewählt ist, und/oder wobei das chimäre Polypeptid mehr als eine SH2-Domäne aus einem Protein umfasst, das ein phosphoryliertes Immunrezeptor-Tyrosin basiertes Aktivierungsmotiv bindet.

5. Zelle nach einem der vorhergehenden Ansprüche, wobei das ITIM und/oder ITSM von einem inhibitorischen Rezeptorprotein, vorzugsweise einem inhibitorischen Immunrezeptorprotein, vorzugsweise von einem aus der aus PD1, BTLA, SIRPalpha, SIGLEC5, SIGLEC9, SIGLEC11, PECAM1 oder LY9 bestehenden Gruppe ausgewählten Protein stammt.

6. Zelle nach einem der vorhergehenden Ansprüche, wobei die CRBN-Polypeptid-Substrat-Domäne ein C2H2-Zinkfingerprotein oder ein Fragment davon ist, das zu einer Arzneistoff-induzierbaren Bindung an das CRBN-Polypeptid in der Lage ist, und/oder wobei die CRBN-Polypeptid-Substrat-Domäne aus der Gruppe ausgewählt ist, bestehend aus IKZF1, IKZF3, ZFN654, ZNF787, ZNF653, ZFP91, ZNF276, ZNF827 oder einem Fragment davon, das zu einer Arzneistoff-induzierbaren Bindung an das CRBN-Polypeptid in der Lage ist, wobei vorzugsweise das Fragment aus der aus IKZF1 ZF2-3 (SEQ ID NO:41), IKZF3 ZF2-3 (SEQ ID NO:42), ZFP91 ZF4-5 (SEQ ID NO: 43), ZNF276 ZF4-5 (SEQ ID NO:44), ZNF653 ZF4-5 (SEQ ID NO: 45) und ZNF692 ZF4-5 (SEQ ID NO:46) bestehenden Gruppe ausgewählt ist, und/oder wobei die CRBN-Polypeptid-Substrat-Domäne ein Hybrid-Fusionspolypeptid umfasst, wobei das Hybrid-Fusionspolypeptid mindestens ein erstes Fragment eines ersten C2H2-Zinkfingerproteins und ein zweites Fragment aus einem zweiten C2H2-Zinkfingerprotein umfasst, wobei die Kombination des ersten Fragments und des zweiten Fragments im Hybrid-Fusionspolypeptid zur Arzneistoff-induzierbaren Bindung an das CRBN-Polypeptid in der Lage ist, wobei vorzugsweise das in der CRBN-Polypeptid-Substrat-Domäne enthaltene Hybrid-Fusionspolypeptid ein erstes Fragment, ausgewählt aus dem beta-Turn von ZFP91 ZF4 (LQCEICGFTCR; SEQ ID NO: 52), ZFN653 ZF4 (LQCEICGYQCR; SEQ ID NO 53), ZNF276 ZF4 (LQCEVCGFQCR: SEQ ID NO: 54), ZNF827 ZF1 (FQCPICGLVIK; SEQ ID NO: 55), und ein zweites Fragment, ausgewählt aus der alpha-Helix von IKZF1 ZF2 (QKGNLLRHIKLH; SEQ ID NO: 56), umfasst, wobei vorzugsweise das Hybrid-Fusionspolypeptid den beta-Turn von ZFP91 ZF4 und die alpha-Helix von IKZF1 ZF2 umfasst, wobei vorzugsweise das Hybrid-Fusionspolypeptid ein aus SEQ ID NO 47 - 51 ausgewähltes umfasst.

7. Zelle nach einem der vorhergehenden Ansprüche, wobei die CRBN-Polypeptid-Substrat-Bindungsdomäne IKZF1 ZF3 (FKCHLCNYACRRRDALTGHLRTH; SEQ ID NO: 57) umfasst oder ferner umfasst, wobei vorzugsweise die CRBN-Polypeptid-Substrat-Bindungsdomäne den beta-Turn von ZFP91 ZF4, die alpha-Helix von IKZF1 ZF2 und IKZF1 ZF3 umfasst und/oder wobei der Arzneistoff, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, ein IMiD ist, das vorzugsweise aus der aus Thalidomid, Lenalidomid, Pomalidomid, CC-122 (Avadomid), CC-220 (Iberdomid) und CC-885 bestehenden Gruppe ausgewählt ist.

8. Zelle nach einem der vorhergehenden Ansprüche, wobei
a) die SH2-Domäne eine Aminosäuresequenz umfasst oder daraus besteht, die mindestens 80 % Identität mit einer Aminosäuresequenz gemäß SEQ ID NO 7 - 11 aufweist, oder eine Aminosäuresequenz umfasst oder daraus besteht, die mindestens 80 % Identität mit einer Aminosäuresequenz gemäß SEQ ID NO 12 - 14 aufweist;
b) der erste Teil des chimären Polypeptids eine Aminosäuresequenz umfasst oder daraus besteht, die mindestens 80 % Identität mit einer Aminosäuresequenz gemäß SEQ ID NO 7 - 11 aufweist, oder eine Aminosäuresequenz umfasst oder daraus besteht, die mindestens 80 % Identität mit einer Aminosäuresequenz gemäß SEQ ID NO 12 - 14 aufweist;
c) das ITAM YxxL/Ix(6-8)YxxL/I ist;
d) die SH2-Domäne eine SH2-Domäne ist, die an ein in SEQ ID NO 1 - 6 enthaltenes phosphoryliertes ITAM bindet oder die an eine Aminosäuresequenz bindet, die 80 % oder mehr Identität mit einer Aminosäuresequenz gemäß SEQ ID NO 1-6 aufweist;
e) das ITIM S/I/V/LxYxxI/V/L ist und/oder das ITSM TxYxxV/I ist;
f) das ITSM und/oder das ITIM ein in SEQ ID No 15 - 22 enthaltenes ITSM und/oder ITIM ist;
g) der dritte Teil des chimären Polypeptids eine Aminosäuresequenz umfasst oder daraus besteht, die mindestens 80 % Identität mit einer Aminosäuresequenz gemäß SEQ ID NO 15 - 22 aufweist;
h) der zweite Teil des chimären Polypeptids eine Aminosäuresequenz gemäß SEQ ID NO:35 oder 40 oder SEQ ID NO: 41 - 57 umfasst oder daraus besteht und/oder
i) das chimäre Polypeptid eine Aminosäuresequenz umfasst, die 80 % oder mehr Identität mit einer Aminosäuresequenz gemäß SEQ ID NO 23 - 34, SEQ ID NO 36 oder SEQ ID NO 58 - 68 aufweist.

9. Chimäres Polypeptid nach einem der Ansprüche 1 - 8 und/oder Nukleinsäure, umfassend ein Polynukleotid, das ein chimäres Polypeptid nach einem der Ansprüche 1 - 8 codiert, und/oder Vektor, der die Nukleinsäure umfasst, und/oder Zelle, die den Vektor umfasst.

10. Pharmazeutische Zusammensetzung, umfassend die Zelle nach einem der Ansprüche 1 - 8, das chimäre Polypeptid, die Nukleinsäure und/oder den Vektor nach Anspruch 9.

11. Zelle nach einem der Ansprüche 1 - 8 zur Verwendung als Arzneimittel, vorzugsweise zur Verwendung bei der Behandlung von Krebs bei einem Individuum, wobei die Behandlung vorzugsweise Folgendes umfasst
a) Verabreichen einer Population von Zellen nach einem der Ansprüche 1 - 8 an das Individuum;
b) optional Verabreichen eines Inhibitors der reprimierbaren Protease oder einer Protac, die an die auf Proteolyse abzielende Chimäre(Protac)-Bindungsdomäne bindet, oder eines Arzneistoffs, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, wobei vorzugsweise der Arzneistoff ein IMiD ist, der vorzugsweise aus der aus Thalidomid, Lenalidomid, Pomalidomid, CC-122 (Avadomid), CC-220 (Iberdomid) und CC-885 bestehenden Gruppe ausgewählt ist;
c) optional, wenn Schritt b) durchgeführt wird, Erhöhen oder Verringern der Konzentration des Inhibitors der reprimierbaren Protease oder einer Protac, die an die auf Proteolyse abzielende Chimäre(Protac)-Bindungsdomäne bindet, oder des Arzneistoffs, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, wobei der Arzneistoff vorzugsweise ein IMiD ist, der vorzugsweise aus der aus Thalidomid, Lenalidomid, Pomalidomid, CC-122 (Avadomid), CC-220 (Iberdomid) und CC-885 bestehenden Gruppe ausgewählt ist.

12. Verfahren zum Bereitstellen einer Zelle nach einem der Ansprüche 1 - 8, wobei das Verfahren das Inkontaktbringen der Zelle mit einer Nukleinsäure oder dem Vektor nach Anspruch 9 umfasst, wobei die Nukleinsäure oder der Vektor mit der Zelle ex vivo in Kontakt gebracht wird.

13. Verfahren zum Kontrollieren der Expression eines chimären Polypeptids in einer Zelle, wobei das Verfahren das Inkontaktbringen der Zelle, die das chimäre Polypeptid nach Anspruch 9 exprimiert, mit einem Inhibitor der reprimierbaren Protease, mit einer Protac, die an die auf Proteolyse abzielende Chimäre(Protac)-Bindungsdomäne bindet, oder mit dem Arzneistoff, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, umfasst, wobei der Arzneistoff vorzugsweise ein IMiD ist, der vorzugsweise aus der aus Thalidomid, Lenalidomid, Pomalidomid, CC-122 (Avadomid), CC-220 (Iberdomid) und CC-885 bestehenden Gruppe ausgewählt ist.

14. Verfahren zur Kontrolle der zytotoxischen Aktivität von T-Zellen und/oder NK-Zellen und/oder zur Kontrolle der Zytokinsekretion durch T-Zellen und/oder NK-Zellen, wobei das Verfahren Folgendes umfasst:
a) Exprimieren eines chimären Polypeptids nach Anspruch 9 in der T-Zelle und/oder NK-Zelle;
b) Ex vivo-Inkontaktbringen der Zellen mit einem Inhibitor der reprimierbaren Protease, mit einer Protac, die an die auf Proteolyse abzielende Chimäre(Protac)-Bindungsdomäne bindet, oder mit einem Arzneistoff, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, wobei vorzugsweise der Arzneistoff ein IMiD ist, der vorzugsweise aus der aus Thalidomid, Lenalidomid, Pomalidomid, CC-122 (Avadomid), CC-220 (Iberdomid) und CC-885 bestehenden Gruppe ausgewählt ist;
c) optional Erhöhen oder Verringern der Konzentration des Inhibitors der reprimierbaren Protease, der Protac oder des Arzneistoffs, der es der CRBN-Polypeptid-Substrat-Domäne ermöglicht, an das CRBN-Protein zu binden, wodurch der durch den Ubiquitinweg vermittelte Abbau des chimären Polypeptids gefördert wird, wobei vorzugsweise der Arzneistoff ein IMiD ist, der vorzugsweise aus der aus Thalidomid, Lenalidomid, Pomalidomid, CC-122 (Avadomid), CC-220 (Iberdomid) und CC-885 bestehenden Gruppe ausgewählt ist.

## Revendications

1. Cellule comprenant un polypeptide chimérique, ou comprenant un acide nucléique comprenant un polynucléotide codant pour ledit polypeptide chimérique, dans laquelle le polypeptide chimérique comprend :
a) une première partie comprenant un domaine SH2 d'une protéine qui se lie à un motif d'activation à base de tyrosine d'immunorécepteur phosphorylé (ITAM) ;
b) une deuxième partie comprenant un domaine de déstabilisation régulé par petites molécules ; et
c) une troisième partie comprenant un motif de commutation à base de tyrosine d'immunorécepteur (ITSM), et/ou un motif inhibiteur à base de tyrosine d'immunorécepteur (ITIM), de préférence un ITSM et un ITIM.

2. Cellule selon la revendication 1, dans laquelle le domaine de déstabilisation régulé par petites molécules est choisi dans le groupe constitué par
i) un dégron à auto-excision (SED), où le SED comprend une protéase répressible, un site de clivage apparenté et une séquence de dégron ;
ii) un domaine de liaison à la chimère ciblant la protéolyse (Protac), où la Protac comprend un groupe de liaison à l'ubiquitine ligase E3 (E3LB), facultativement un lieur, et un groupe de liaison à une protéine qui se lie au domaine de liaison à la Protac dans le polypeptide chimérique ; et
iii) un domaine de substrat polypeptidique CRBN capable de se lier à la protéine CRBN en réponse à un médicament, favorisant ainsi la dégradation médiée par la voie de l'ubiquitine du polypeptide chimérique.

3. Cellule selon l'une quelconque des revendications précédentes, dans laquelle l'ITAM est un ITAM compris dans un complexe de récepteur de cellules T (TCR) et/ou un complexe de récepteur de cellules NK (NKR) et/ou un récepteur antigénique chimérique (CAR), de préférence un ITAM dérivé d'une chaîne zêta CD3, d'une chaîne epsilon CD3, d'une chaîne delta CD3, d'une chaîne gamma CD3, d'une chaîne gamma du récepteur d'immunoglobuline FcεRI et DAP12, et/ou dans laquelle la cellule comprend en outre un récepteur de cellules T et/ou un récepteur antigénique chimérique (CAR) et/ou un récepteur de cellules NK (NKR), de préférence dans laquelle la cellule est une cellule T et/ou une cellule CAR T et/ou une cellule NK et/ou une cellule CAR NK.

4. Cellule selon l'une quelconque des revendications précédentes, dans laquelle le domaine SH2 provient d'une protéine choisie dans le groupe constitué par Zap70, Syk et Lck, et/ou dans laquelle le polypeptide chimérique comprend plus d'un domaine SH2 d'une protéine qui se lie à un motif d'activation à base de tyrosine d'immunorécepteur phosphorylé.

5. Cellule selon l'une quelconque des revendications précédentes, dans laquelle l'ITIM et/ou l'ITSM provient/proviennent d'une protéine de récepteur inhibiteur, de préférence d'une protéine de récepteur immunitaire inhibiteur, de préférence d'une protéine choisie dans le groupe constitué par PD1, BTLA, SIRPalpha, SIGLEC5, SIGLEC9, SIGLEC11, PECAM1 ou LY9.

6. Cellule selon l'une quelconque des revendications précédentes, dans laquelle le domaine de substrat polypeptidique CRBN est une protéine à doigt de zinc C2H2 ou un fragment de celle-ci qui est capable de se lier de manière inductible par un médicament au polypeptide CRBN, et/ou dans laquelle le domaine de substrat polypeptidique CRBN, est choisi dans le groupe constitué par IKZF1, IKZF3, ZFN654, ZNF787, ZNF653, ZFP91, ZNF276, ZNF827, ou un fragment de ceux-ci qui est capable de se lier de manière inductible par un médicament au polypeptide CRBN, de préférence dans laquelle ledit fragment est choisi dans le groupe constitué par IKZF1 ZF2-3 (SEQ ID NO : 41), IKZF3 ZF2-3 (SEQ ID NO : 42), ZFP91 ZF4-5 (SEQ ID NO : 43), ZNF276 ZF4-5 (SEQ ID NO : 44), ZNF653 ZF4-5 (SEQ ID NO : 45) et ZNF692 ZF4-5 (SEQ ID NO : 46) et/ou dans laquelle le domaine de substrat polypeptidique CRBN comprend un polypeptide de fusion hybride, dans laquelle le polypeptide de fusion hybride comprend au moins un premier fragment d'une première protéine à doigt de zinc C2H2 et un second fragment d'une seconde protéine à doigt de zinc C2H2, dans laquelle la combinaison dudit premier fragment et dudit second fragment dans le polypeptide de fusion hybride est capable de se lier de manière inductible par un médicament au polypeptide CRBN, de préférence dans laquelle le polypeptide de fusion hybride compris dans le domaine de substrat polypeptidique CRBN comprend un premier fragment choisi parmi la spire bêta de ZFP91 ZF4 (LQCEICGFTCR ; SEQ ID NO : 52), ZFN653 ZF4 (LGCEICGYQCR ; SEQ ID NO 53), ZNF276 ZF4 (LQCEVCGFQCR : SEQ ID NO : 54), ZNF827 ZF1 (FQCPICGLVIK ; SEQ ID NO : 55) et un second fragment choisi parmi l'hélice alpha de IKZF1 ZF2 (QKGNLLRHIKLH ; SEQ ID NO : 56), de préférence, le polypeptide de fusion hybride comprend la spire bêta de ZFP91 ZF4 et l'hélice alpha de IKZF1 ZF2, de préférence dans laquelle le polypeptide de fusion hybride comprend l'une choisie parmi SEQ ID NO 47-51.

7. Cellule selon l'une quelconque des revendications précédentes, dans laquelle le domaine de liaison de substrat polypeptidique CRBN comprend ou comprend en outre IKZF1 ZF3 (FKCHLCNYACRRRDALTGHLRTH ; SEQ ID NO : 57), de préférence dans laquelle le domaine de liaison de substrat polypeptidique CRBN comprend la spire bêta de ZFP91 ZF4, l'hélice alpha de IKZF1 ZF2 et IKZF1 ZF3, et/ou dans laquelle le médicament qui permet au domaine de substrat polypeptidique CRBN de se lier à la protéine CRBN, favorisant ainsi la dégradation médiée par la voie de l'ubiquitine du polypeptide chimérique, est un IMiD, de préférence choisi dans le groupe constitué par la thalidomide, le lénalidomide, le pomalidomide, le CC-122 (avadomide), le CC-220 (iberdomide) et le CC-885.

8. Cellule selon l'une quelconque des revendications précédentes dans laquelle
a) le domaine SH2 comprend ou consiste en une séquence d'acides aminés ayant une identité d'au moins 80% avec une séquence d'acides aminés selon SEQ ID NO 7-11 ou comprend ou consiste en une séquence d'acides aminés ayant une identité d'au moins 80% avec une séquence d'acides aminés selon SEQ ID NO 12-14 ;
b) la première partie du polypeptide chimérique comprend ou consiste en une séquence d'acides aminés ayant une identité d'au moins 80% avec une séquence d'acides aminés selon SEQ ID NO 7-11 ou comprend ou consiste en une séquence d'acides aminés ayant une identité d'au moins 80% avec une séquence d'acides aminés selon SEQ ID NO 12-14 ;
c) l'ITAM est YxxL/Ix(6-8)YxxL/I ;
d) le domaine SH2 est un domaine SH2 qui se lie à un ITAM phosphorylé compris dans SEQ ID NO 1-6, ou qui se lie à une séquence d'acides aminés ayant une identité supérieure ou égale à 80% avec une séquence d'acides aminés selon SEQ ID NO 1-6 ;
e) l'ITIM est S/I/V/LxYxxI/V/L, et/ou l'ITSM est TxYxxV/I ;
f) l'ITSM et/ou l'ITIM est un ITSM et/ou un ITIM qui est compris dans SEQ ID No 15-22 ;
g) la troisième partie du polypeptide chimérique comprend ou consiste en une séquence d'acides aminés ayant une identité d'au moins 80% avec une séquence d'acides aminés selon SEQ ID NO 15-22 ;
h) la deuxième partie du polypeptide chimérique comprend ou consiste en une séquence d'acides aminés selon SEQ ID NO : 35 ou 40 ou SEQ ID NO : 41-57 ; et/ou
i) le polypeptide chimérique comprend une séquence d'acides aminés ayant une identité supérieure ou égale à 80% avec une séquence d'acides aminés selon SEQ ID NO 23-34, SEQ ID NO 36 ou SEQ ID NO 58-68.

9. Polypeptide chimérique tel que défini dans l'une quelconque des revendications 1 à 8, et/ou acide nucléique comprenant un polynucléotide codant pour un polypeptide chimérique tel que défini dans l'une quelconque des revendications 1 à 8, et/ou vecteur comprenant ledit acide nucléique et/ou cellule comprenant ledit vecteur.

10. Composition pharmaceutique comprenant la cellule de l'une quelconque des revendications 1 à 8, le polypeptide chimérique, l'acide nucléique et/ou le vecteur de la revendication 9.

11. Cellule selon les revendications 1 à 8 pour une utilisation en tant que médicament, de préférence pour une utilisation dans le traitement du cancer chez un sujet, de préférence dans laquelle le traitement comprend
a) l'administration au sujet d'une population de cellules selon l'une quelconque des revendications 1 à 8 ;
b) facultativement, l'administration au sujet d'un inhibiteur de la protéase répressible ou d'une Protac qui se lie au domaine de liaison à la chimère ciblant la protéolyse (Protac) ou d'un médicament qui permet au domaine de substrat polypeptidique CRBN de se lier à la protéine CRBN, favorisant ainsi la dégradation médiée par la voie de l'ubiquitine du polypeptide chimérique, de préférence où le médicament est un IMiD, de préférence choisi dans le groupe constitué par la thalidomide, le lénalidomide, le pomalidomide, le CC-122 (avadomide), le CC-220 (iberdomide) et le CC-885 ;
c) facultativement, si l'étape b) est réalisée, l'augmentation ou la réduction de la concentration de l'inhibiteur de la protéase répressible ou d'une Protac qui se lie au domaine de liaison à la chimère ciblant la protéolyse (Protac) ou du médicament qui permet au domaine de substrat polypeptidique CRBN de se lier à la protéine CRBN, favorisant ainsi la dégradation médiée par la voie de l'ubiquitine du polypeptide chimérique, de préférence où le médicament est un IMiD, de préférence choisi dans le groupe constitué par la thalidomide, le lénalidomide, le pomalidomide, le CC-122 (avadomide), le CC-220 (iberdomide) et le CC-885.

12. Procédé de fourniture d'une cellule selon l'une quelconque des revendications 1 à 8, dans lequel le procédé comprend la mise en contact de la cellule avec un acide nucléique ou le vecteur de la revendication 9, dans lequel l'acide nucléique ou le vecteur est mis en contact avec la cellule ex vivo.

13. Procédé de régulation de l'expression d'un polypeptide chimérique dans une cellule, dans lequel le procédé comprend la mise en contact de la cellule exprimant le polypeptide chimérique selon la revendication 9 avec un inhibiteur de la protéase répressible, avec une Protac qui se lie au domaine de liaison à la chimère ciblant la protéolyse (Protac), ou avec le médicament qui permet au domaine de substrat polypeptidique CRBN de se lier à la protéine CRBN, favorisant ainsi la dégradation médiée par la voie de l'ubiquitine du polypeptide chimérique, de préférence où le médicament est un IMiD, de préférence choisi dans le groupe constitué par la thalidomide, le lénalidomide, le pomalidomide, le CC-122 (avadomide), le CC-220 (iberdomide) et le CC-885, et dans lequel la cellule exprimant le polypeptide chimérique est mise en contact avec l'inhibiteur ex vivo.

14. Procédé de régulation de l'activité cytotoxique des cellules T et/ou des cellules NK et/ou de régulation de la sécrétion de cytokines par les cellules T et/ou les cellules NK, dans lequel le procédé comprend
a) l'expression d'un polypeptide chimérique selon la revendication 9 dans la cellule T et/ou la cellule NK ;
b) la mise en contact ex vivo des cellules avec un inhibiteur de la protéase répressible, avec une Protac qui se lie au domaine de liaison à la chimère ciblant la protéolyse (Protac), ou avec un médicament qui permet au domaine de substrat polypeptidique CRBN de se lier à la protéine CRBN, favorisant ainsi la dégradation médiée par la voie de l'ubiquitine du polypeptide chimérique, de préférence où le médicament est un IMiD, de préférence choisi dans le groupe constitué par la thalidomide, le lénalidomide, le pomalidomide, le CC-122 (avadomide), le CC-220 (iberdomide) et le CC-885 ;
c) facultativement, l'augmentation ou la réduction de la concentration de l'inhibiteur de la protéase répressible, de la Protac, ou du médicament qui permet au domaine de substrat polypeptidique CRBN de se lier à la protéine CRBN, favorisant ainsi la dégradation médiée par la voie de l'ubiquitine du polypeptide chimérique, de préférence où le médicament est un IMiD, de préférence choisi dans le groupe constitué par la thalidomide, le lénalidomide, le pomalidomide, le CC-122 (avadomide), le CC-220 (iberdomide) et le CC-885.
